# EUROPEAN PATENT APPLICATION

(11) **EP 4 744 584 A1**
(43) Date of publication of application: **20.05.2026**
(21) Application number: 24906343.9
(22) Date of filing: 17.12.2024
(51) Int. Cl.: A61B 5/024, A61B 5/026, A61B 5/029, A61B 5/11, A61B 5/00, A61B 5/053, A61B 5/0531, A61B 5/332, A61B 5/33

(54) **MONITORING METHOD AND SYSTEM AND RELATED APPARATUS**

(30) Priority: 19.12.2023 CN 202311766418
(71) Applicant: Huawei Technologies Co., Ltd., Shenzhen, Guangdong 518129 (CN)
(72) Inventor: ZHAO, Shuai, Shenzhen, Guangdong 518129 (CN); XU, Teng, Shenzhen, Guangdong 518129 (CN); REN, Huichao, Shenzhen, Guangdong 518129 (CN); QI, Biao, Shenzhen, Guangdong 518129 (CN)
(74) Representative: Pfenning, Meinig & Partner mbB
(86) International application number: PCT/CN2024/139925
(87) International publication number: WO 2025/130863

(57) **Abstract**

This application discloses a monitoring method, a system, and a related apparatus, applied to a first electronic device. The first electronic device is provided with a first circuit, and the first circuit includes a first electrode, a second electrode, a third electrode, a fourth electrode, an excitation current generation unit, and a voltage measurement unit. A first instruction is received, and in response to the first instruction, it is determined, through the first circuit, that the first electrode and the second electrode are in good contact with skin. In addition, first information is determined through the first circuit, the first electrode is in contact with a first position on the skin of a user, the second electrode is in contact with a second position on the skin of the user, and the first position and the second position are respectively at two ends of thoracic tissue. The first information is output, and the first information includes one or more of the following: a cardiac output, a stroke volume, a heart rate, an ejection fraction, and a cardiac function determining result. In this way, a cardiac function condition of the user can be monitored in real time, so that the user conveniently performs monitoring anytime and anywhere.

## Description

This application claims priority to Chinese Patent Application No. 202311766418.9, filed with the China National Intellectual Property Administration on December 19, 2023 and entitled "MONITORING METHOD, SYSTEM, AND RELATED APPARATUS", which is incorporated herein by reference in its entirety.

### TECHNICAL FIELD

This application relates to the field of electronic technologies, and in particular, to a monitoring method, a system, and a related apparatus.

### BACKGROUND

With continuous development of electronic technologies, electronic devices are more closely related to daily life, and more electronic devices have a health monitoring function. Cardiac function monitoring is an important branch of health monitoring.

When cardiac function monitoring needs to be performed, a hospital may monitor a cardiac function of a user by using an echocardiogram or a medical device like a cardiac impedance hemodynamic monitor, and determine a cardiac function condition of the user based on a monitoring result.

However, it is inconvenient for the user to carry the medical device around, and a cardiac function monitoring result cannot be obtained in real time in the foregoing manner.

### SUMMARY

This application provides a monitoring method, a system, and a related apparatus, to obtain a cardiac function monitoring result of a user in real time, so that the user can learn of a health condition of the user in a timely manner.

According to a first aspect, this application provides a monitoring method, applied to a first electronic device. The first electronic device is provided with a first circuit, and the first circuit includes a first electrode, a second electrode, a third electrode, a fourth electrode, an excitation current generation unit, and a voltage measurement unit. The method includes: receiving a first instruction, where the first instruction instructs the first electronic device to start cardiac function monitoring; in response to the first instruction, determining, through a first loop, that the first electrode is in good contact with skin, where the first loop includes the first electrode, the excitation current generation unit, and the third electrode; determining, through a second loop, that the second electrode is in good contact with the skin, where the second loop includes the second electrode, the excitation current generation unit, and the fourth electrode; determining first information through a third loop, where the third loop includes the first electrode, the excitation current generation unit, the voltage measurement unit, and the second electrode, the first electrode is in contact with a first position on the skin of a user, the second electrode is in contact with a second position on the skin of the user, and the first position and the second position are respectively at two ends of thoracic tissue; and outputting the first information, where the first information includes one or more of the following: a cardiac output, a stroke volume, a heart rate, an ejection fraction, a comprehensive result, a first result, a second result, a third result, and a fourth result, the comprehensive result indicates whether a cardiac function of the user is normal, the first result indicates whether the cardiac output is normal, the second result indicates whether the stroke volume is normal, the third result indicates whether the heart rate is normal, and the fourth result indicates whether the ejection fraction is normal.

The first circuit may be the monitoring circuit in any implementation of the following second aspect.

In this way, the first electronic device can obtain a cardiac function monitoring result of the user in real time, so that the user can learn of a health condition of the user in a timely manner. In addition, the first electronic device may further determine whether an electrode is in good contact with the skin of the user.

In a possible implementation, receiving the first instruction specifically includes: receiving the first instruction sent by a second electronic device; or receiving a first operation of the user on the first electronic device, and generating the first instruction.

In this way, the first electronic device may start cardiac function monitoring in response to an operation of the user or in response to an instruction sent by another electronic device.

In a possible implementation, the method further includes: outputting a first prompt in response to the first instruction, where the first prompt is used to inform the user that cardiac function monitoring is started.

In this way, the first electronic device may remind, by using the first prompt, the user whether cardiac function monitoring is started.

In a possible implementation, before determining the first information through the third loop, the method further includes: determining that a user status is a first state; and determining the first information through the third loop specifically includes: determining a cardiac function indicator through the third loop, where the cardiac function indicator includes any one or more of the following: the cardiac output, the stroke volume, the heart rate, and the ejection fraction; and determining the first information based on the first state and the cardiac function indicator.

In this way, the first electronic device can obtain the user status in real time, and determine, based on the user status, whether the cardiac function indicator of the user is normal.

In a possible implementation, the first state is an exercise state, a resting state, or a sleep state.

In another possible implementation, the first state may further include but is not limited to any one or more of the following: a scared state, an oxygen-deficient state, a diving state, a meditative state, and the like.

In a possible implementation, determining that the user status is the first state specifically includes: detecting that the user status is the first state; or receiving a status setting operation of the user, and in response to the operation, determining that the user status is the first state; or receiving second information sent by the second electronic device, and determining, based on the second information, that the user status is the first state.

In this way, the first electronic device may determine the user status based on the status setting operation of the user, or may determine the user status based on information sent by another electronic device. The first electronic device may alternatively detect the user status by using a component such as a sensor.

In a possible implementation, when the first state is the exercise state, the first information further includes a first graphic, and the first graphic indicates a relationship between the stroke volume and an exercise heart rate.

In this way, when the user is in the exercise state, the first electronic device may output the first graphic to inform the user of the relationship between the stroke volume and the exercise heart rate.

In a possible implementation, outputting the first information specifically includes: sending a second instruction to the second electronic device, where the second instruction indicates that the first electronic device outputs the first information.

In this way, the first electronic device may alternatively output the first information through the second electronic device.

In a possible implementation, the first electronic device is earphones, and the method further includes: playing, by the earphones, first audio before the first instruction is received; and pausing play of the first audio when the first instruction is received.

In a possible implementation, the first electronic device is the earphones; playing the first audio specifically includes: playing the first audio at first volume; and outputting the first information specifically includes: playing the first information at the first volume if the user wears a left earphone or a right earphone.

In a possible implementation, the first electronic device is the earphones, and the method further includes: playing the first information at second volume if the user wears neither the left earphone nor the right earphone, where the second volume is greater than the first volume.

In this way, when the first electronic device is the earphones, the first electronic device may determine output volume of the first information based on whether the user wears the earphones.

In a possible implementation, the first electronic device is the earphones, and outputting the first information specifically includes: sending a second instruction to the second electronic device if the user wears neither the left earphone nor the right earphone, where the second instruction indicates that the first electronic device outputs the first information.

In this way, when the first electronic device is the earphones, the first electronic device may determine, based on whether the user wears the earphones, whether to output the first information through the second electronic device.

In a possible implementation, the first electronic device is earphones, the earphones include a left earphone and a right earphone, there is a wired connection between the left earphone and the right earphone, the first electrode and the third electrode are disposed on the left earphone, and the second electrode and the fourth electrode are disposed on the right earphone.

In a possible implementation, the first electronic device is a wearable device, the wearable device includes a movement and a watchband, the first electrode and the third electrode are located at one end of the watchband, the second electrode and the fourth electrode are located at the other end of the watchband, and a length of the watchband is greater than a first length.

In a possible implementation, the first electronic device is a wearable device, the wearable device includes a movement and a watchband, the movement includes a first button and a second button, the first electrode and the third electrode are located on a rear surface of the movement, the second electrode is located on the first button, and the fourth electrode is located on the second button.

In a possible implementation, the first electronic device is a wearable device, the wearable device includes a movement and a watchband, the movement includes a first button and a second button, the first electrode and the third electrode are located on the watchband, the second electrode is located on the first button, and the fourth electrode is located on the second button.

In a possible implementation, the first electronic device is a mobile phone, the mobile phone includes one or more buttons, one or more of the first electrode, the second electrode, the third electrode, and the fourth electrode are disposed on the one or more buttons, and the first electrode, the second electrode, the third electrode, and the fourth electrode are not in contact with each other.

In a possible implementation, the one or more buttons of the mobile phone include a volume button and a fingerprint button, and that the one or more of the first electrode, the second electrode, the third electrode, and the fourth electrode are disposed on the one or more buttons specifically includes: The first electrode and the third electrode are disposed on the volume button, and the second electrode and the fourth electrode are disposed on the fingerprint button.

In a possible implementation, the one or more buttons of the mobile phone include a first volume button, a second volume button, and a fingerprint button, and that the one or more of the first electrode, the second electrode, the third electrode, and the fourth electrode are disposed on the one or more buttons specifically includes: The first electrode is disposed on the first volume button, the third electrode is disposed on the second volume button, and the second electrode and the fourth electrode are disposed on the fingerprint button.

In this way, when the first electronic device is in different device forms, each electrode may be disposed at different positions.

According to a second aspect, this application provides a monitoring circuit, including: a first electrode, a second electrode, a third electrode, a fourth electrode, a first analog switch, a second analog switch, an excitation current generation unit, and a voltage measurement unit. The first analog switch includes a first port and a second port, and the second analog switch includes a third port and a fourth port; the first electrode is connected to the second electrode through the first port of the first analog switch, the excitation current generation unit, and the third port of the second analog switch; the third electrode is connected to the fourth electrode through the fourth port of the second analog switch, the excitation current generation unit, and the second port of the first analog switch; the voltage measurement unit is connected to the third electrode and the fourth electrode, or the voltage measurement unit is connected to the first electrode and the second electrode; and when the first electrode is in contact with a first position on skin of a user, the second electrode is in contact with a second position on the skin of the user, and the first position and the second position are respectively at two ends of thoracic tissue, the excitation current generation unit is configured to generate a current flowing through a heart of the user, and the voltage measurement unit is configured to measure a voltage between two ends of the heart of the user.

In this way, the voltage between the two ends of the heart of the user and the current flowing through the heart of the user can be obtained in real time by using the monitoring circuit, to determine a cardiac impedance of the user, and obtain a cardiac function monitoring result of the user.

In a possible implementation, that the first electrode is connected to the second electrode through the first port of the first analog switch, the excitation current generation unit, and the third port of the second analog switch specifically includes: the first electrode, the first analog switch, the excitation current generation unit, the second analog switch, and the second electrode are sequentially connected, the first electrode is connected to the first port of the first analog switch, and the second electrode is connected to the third port of the second analog switch; and that the third electrode is connected to the second electrode through the fourth port of the second analog switch, the excitation current generation unit, and the second port of the first analog switch specifically includes: the third electrode, the second analog switch, the excitation current generation unit, the first analog switch, and the fourth electrode are sequentially connected, the third electrode is connected to the fourth port of the second analog switch, and the fourth electrode is connected to the second port of the first analog switch.

In a possible implementation, the excitation current generation unit is connected to the first analog switch and the second analog switch.

In a possible implementation, the first analog switch is configured to electrically connect to the first port or the second port, and the second analog switch is configured to electrically connect to the third port or the fourth port.

It should be noted that, the first analog switch and the second analog switch may alternatively be not electrically connected to any port.

In a possible implementation, that the excitation current generation unit is configured to generate the current flowing through the heart of the user, and the voltage measurement unit is configured to measure the voltage between the two ends of the heart of the user specifically includes: when the first analog switch is electrically connected to the first port, and the second analog switch is electrically connected to the third port, the excitation current generation unit is configured to generate the current flowing through the heart of the user, and the voltage measurement unit is configured to measure the voltage between the two ends of the heart of the user; or when the first analog switch is electrically connected to the second port, and the second analog switch is electrically connected to the fourth port, the excitation current generation unit is configured to generate the current flowing through the heart of the user, and the voltage measurement unit is configured to measure the voltage between the two ends of the heart of the user.

In a possible implementation, when the first analog switch is electrically connected to the first port, and the second analog switch is electrically connected to the fourth port, the monitoring circuit is configured to determine whether the first electrode and the third electrode are in good contact with the skin.

In a possible implementation, when the first analog switch is electrically connected to the second port, and the second analog switch is electrically connected to the third port, the monitoring circuit is configured to determine whether the second electrode and the fourth electrode are in good contact with the skin.

In a possible implementation, when the first analog switch is electrically connected to the first port, and the second analog switch is electrically connected to the fourth port, or when the first analog switch is electrically connected to the second port, and the second analog switch is electrically connected to the third port, a frequency of the current generated by the excitation current generation unit is less than a first frequency.

In a possible implementation, a frequency of the current generated by the excitation current generation unit is greater than a first frequency.

In a possible implementation, the first frequency may be 1 kilohertz (KHz).

According to a third aspect, this application provides an electronic device, which is a first electronic device. The first electronic device includes the circuit according to any implementation of the second aspect.

In a possible implementation, the first electronic device is earphones, the earphones include a left earphone and a right earphone, the left earphone and the right earphone are wiredly connected, the first electrode and the third electrode are disposed on the left earphone, and the second electrode and the fourth electrode are disposed on the right earphone.

In a possible implementation, the first electronic device is a wearable device, the wearable device includes a movement and a watchband, the first electrode and the third electrode are located at one end of the watchband, the second electrode and the fourth electrode are located at the other end of the watchband, and a length of the watchband is greater than a first length.

In a possible implementation, the first electronic device is a wearable device, the wearable device includes a movement and a watchband, the movement includes a first button and a second button, the first electrode and the third electrode are located on a rear surface of the movement, the second electrode is located on the first button, and the fourth electrode is located on the second button.

In a possible implementation, the first electronic device is a wearable device, the wearable device includes a movement and a watchband, the movement includes a first button and a second button, the first electrode and the third electrode are located on the watchband, the second electrode is located on the first button, and the fourth electrode is located on the second button.

In a possible implementation, the first electronic device is a mobile phone, the mobile phone includes one or more buttons, one or more of the first electrode, the second electrode, the third electrode, and the fourth electrode are disposed on the one or more buttons, and the first electrode, the second electrode, the third electrode, and the fourth electrode are not in contact with each other.

In a possible implementation, the one or more buttons of the mobile phone include a volume button and a fingerprint button, and that the one or more of the first electrode, the second electrode, the third electrode, and the fourth electrode are disposed on the one or more buttons specifically includes: The first electrode and the third electrode are disposed on the volume button, and the second electrode and the fourth electrode are disposed on the fingerprint button.

In a possible implementation, the one or more buttons of the mobile phone include a first volume button, a second volume button, and a fingerprint button, and that the one or more of the first electrode, the second electrode, the third electrode, and the fourth electrode are disposed on the one or more buttons specifically includes: The first electrode is disposed on the first volume button, the third electrode is disposed on the second volume button, and the second electrode and the fourth electrode are disposed on the fingerprint button.

According to a fourth aspect, this application provides a monitoring system, including a first electronic device and a second electronic device. There is a wired connection between the first electronic device and the second electronic device, and the monitoring system includes the circuit in any implementation of the second aspect.

In this way, a monitoring circuit may be formed by using a plurality of electronic devices, to cooperatively monitor a cardiac function of a user.

In a possible implementation, the first electrode and the third electrode are located on the first electronic device, and the second electrode and the fourth electrode are located on the second electronic device.

In a possible implementation, the first electronic device is earphones, and the second electronic device is a mobile phone; or the first electronic device is a mobile phone, and the second electronic device is earphones.

According to a fifth aspect, this application provides an electronic device, which is a first electronic device, including one or more processors, one or more memories, and a first circuit. The first circuit includes a first electrode, a second electrode, a third electrode, a fourth electrode, a first analog switch, a second analog switch, an excitation current generation unit, and a voltage measurement unit. The one or more memories are coupled to the one or more processors. The one or more memories are configured to store computer program code, and the computer program code includes computer instructions. When the one or more processors execute the computer instructions, a communication apparatus is enabled to perform the monitoring method according to any one of the possible implementations of any one of the foregoing aspects.

According to a sixth aspect, this application provides a chip system, used in a first electronic device. The chip system includes a processing circuit and an interface circuit. The interface circuit is configured to receive code instructions and transmit the code instructions to the processing circuit. The processing circuit is configured to run the code instructions, so that the chip system performs the monitoring method according to any one of the possible implementations of any one of the foregoing aspects.

According to a seventh aspect, an embodiment of this application provides a readable storage medium, including instructions. When the instructions are run on a first electronic device, the first electronic device is enabled to perform the monitoring method according to any one of the possible implementations of any one of the foregoing aspects.

According to an eighth aspect, an embodiment of this application provides a computer program product. When the computer program product runs on a computer, the computer is enabled to perform the monitoring method according to any one of the possible implementations of any one of the foregoing aspects.

For beneficial effects of the third aspect to the eighth aspect, refer to the beneficial effects of the first aspect and the second aspect.

### BRIEF DESCRIPTION OF DRAWINGS

FIG. 1 is a diagram of a system architecture of a health monitoring system 1000 according to an embodiment of this application;
FIG. 2A to FIG. 2C are diagrams of device forms of three types of earphones 10 according to an embodiment of this application;
FIG. 3A to FIG. 3E are diagrams of device forms of three types of watches 20 according to an embodiment of this application;
FIG. 4A and FIG. 4B are diagrams of device forms of two types of mobile phones 30 according to an embodiment of this application;
FIG. 5A is a diagram of a hardware structure of an electronic device 100 according to an embodiment of this application;
FIG. 5B is a diagram of a structure of a monitoring circuit according to an embodiment of this application;
FIG. 5C is a diagram of a current signal generated by an excitation current generation unit according to an embodiment of this application;
FIG. 5D is a diagram of a structure of another monitoring circuit according to an embodiment of this application;
FIG. 5E is a diagram of a circuit connection of a monitoring circuit according to an embodiment of this application;
FIG. 5F to FIG. 5H are diagrams of circuit connections when an analog switch is electrically connected to different ports according to an embodiment of this application;
FIG. 6A is a diagram of a position relationship between thoracic tissue and a human body according to an embodiment of this application;
FIG. 6B to FIG. 6F are diagrams of distribution of contact points between a plurality of electrodes and skin according to an embodiment of this application;
FIG. 7 is a schematic flowchart of a monitoring method according to an embodiment of this application;
FIG. 8A is a schematic flowchart of determining a cardiac function indicator based on a cardiac impedance curve according to an embodiment of this application;
FIG. 8B is a diagram of a cardiac impedance curve in a two-dimensional coordinate system according to an embodiment of this application;
FIG. 8C is a diagram of a first derivative curve of a cardiac impedance curve in a two-dimensional coordinate system according to an embodiment of this application;
FIG. 9A to FIG. 9C are diagrams of a group of interfaces of outputting cardiac function indicators according to an embodiment of this application;
FIG. 10A to FIG. 10C are diagrams of another group of interfaces of outputting cardiac function indicators according to an embodiment of this application;
FIG. 11 is a schematic flowchart of determining an output manner of a cardiac function indicator by earphones 10 according to an embodiment of this application;
FIG. 12A-1 and FIG. 12A-2 are a schematic flowchart of another monitoring method according to an embodiment of this application;

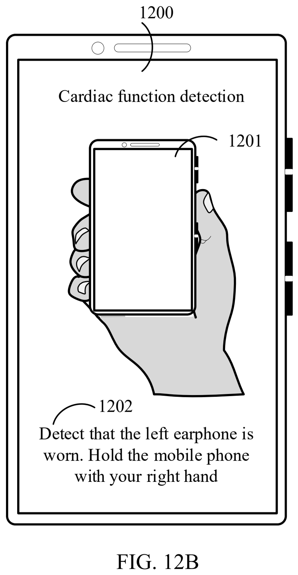

FIG. 12B to FIG. 12D show a group of interfaces in which a mobile phone 30 outputs prompts according to an embodiment of this application;

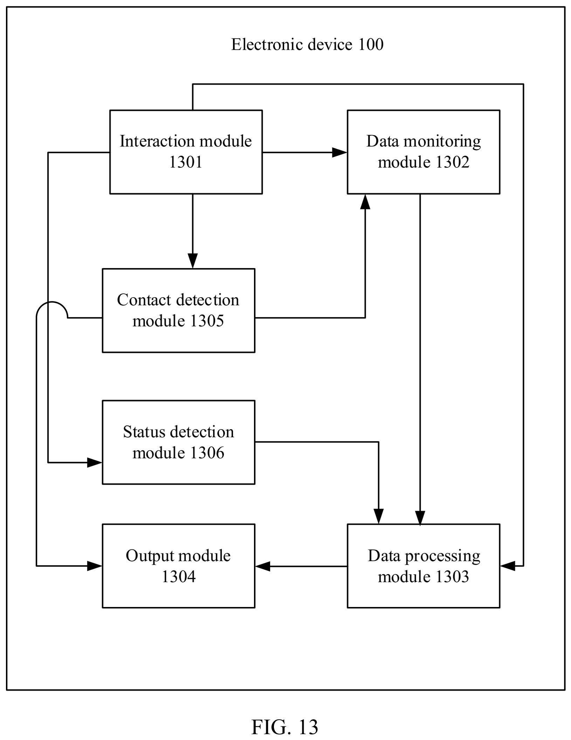

FIG. 13 is a diagram of functional modules of an electronic device 100 according to an embodiment of this application;

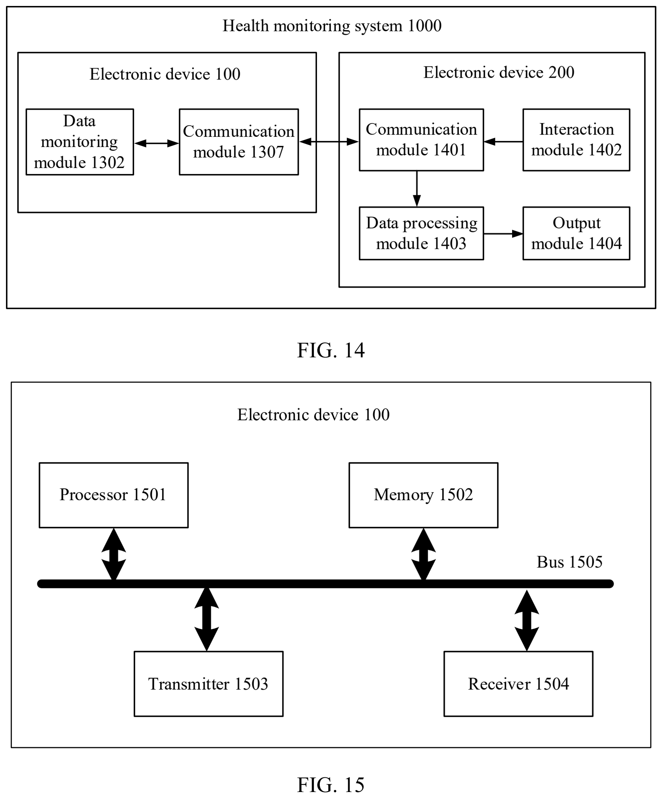

FIG. 14 is a diagram of functional modules of a health monitoring system 1000 according to an embodiment of this application;
FIG. 15 is a diagram of a physical entity apparatus of an electronic device 100 according to an embodiment of this application; and

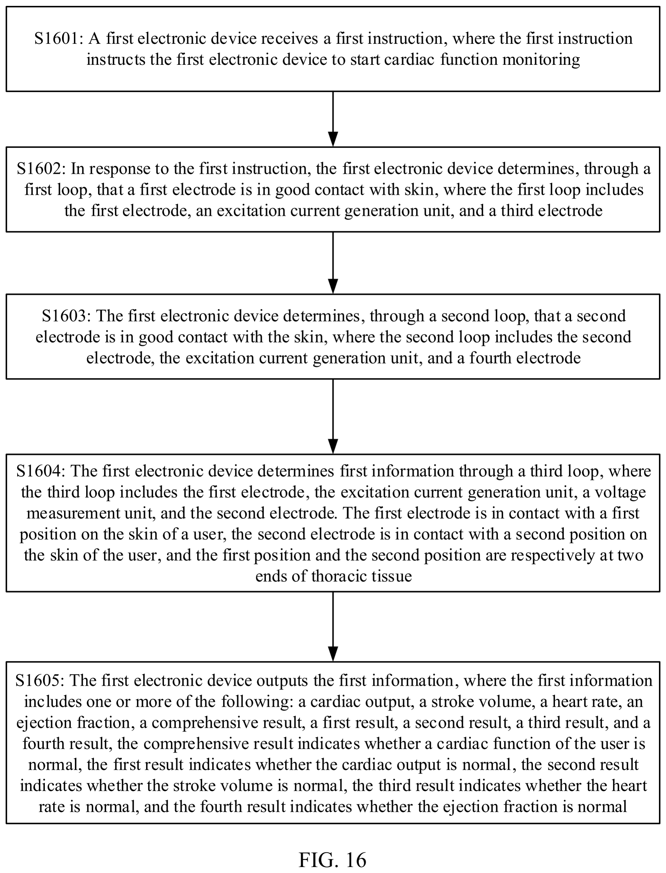

FIG. 16 is a schematic flowchart of a monitoring method according to an embodiment of this application.

### DESCRIPTION OF EMBODIMENTS

Technical solutions in embodiments of this application are clearly described below in detail with reference to the accompanying drawings. In the descriptions of embodiments of this application, unless otherwise specified, "/" indicates "or". For example, A/B may indicate A or B. The term "and/or" in this specification merely describes an association relationship for describing associated objects, and indicates that three relationships may exist. For example, A and/or B may indicate the following three cases: Only A exists, both A and B exist, and only B exists. In addition, in the descriptions of embodiments of this application, "a plurality of" means two or more.

The following terms "first" and "second" are merely used for description, but should not be understood as indicating or implying relative importance or implying a quantity of indicated technical features. Therefore, a feature limited by "first" or "second" may explicitly or implicitly include one or more such features. In the descriptions of embodiments of this application, unless otherwise specified, "a plurality of" means two or more.

The term "user interface (user interface, UI)" in the following embodiments of this application is a medium interface for interaction and information exchange between an application or an operating system and a user, and implement conversion between an internal form of information and an acceptable form of the user. A user interface is source code compiled in a specific computer language such as Java or the extensible markup language (extensible markup language, XML). The interface source code is parsed and rendered on an electronic device, and finally presented as content that can be recognized by the user. A common presentation form of the user interface is a graphic user interface (graphic user interface, GUI), which refers to a user interface that is displayed in a graphical manner and that is related to a computer operation. The GUI may be a visible interface element displayed on a display of an electronic device, for example, a text, an icon, a button, a menu, a tab, a text box, a dialog box, a status bar, a navigation bar, or a widget.

The following describes some technical terms in embodiments of this application.

Heart rate (heart rate, HR): The heart rate refers to a quantity of heartbeats per minute. Each heartbeat can complete one systole and one diastole.

Cardiac output (cardiac output, CO): The cardiac output refers to a total volume of blood pumped from one ventricle (a left ventricle or a right ventricle) per minute, and is also referred to as an output per minute. Outputs of the left and right ventricles are basically equal.

Stroke volume (stroke volume, SV): The stroke volume refers to a volume of blood ejected by a single pump from one ventricle (a left ventricle or a right ventricle) of a heart. A product of the stroke volume and a heart rate is a cardiac output.

Ejection fraction: The ejection fraction refers to a ratio of a stroke volume to a total volume of blood in a heart.

Human body safe current: The human body safe current is a minimum safe current that passes through a human body. When a current passing through the human body is less than or equal to the human body safe current, the current does not cause damage to the human body.

Thoracic tissue: The thoracic tissue is a cavity enclosed by sternums, thoracic vertebrae, and ribs. An upper part is connected to a neck, and a lower part is separated from an abdominal cavity by a diaphragm. Organs such as a heart and a lung are in the thoracic tissue.

Cardiac impedance curve: The cardiac impedance curve is a curve representing a relationship between cardiac impedance and time. The cardiac impedance is a ratio of a voltage between two ends of a heart to a current flowing through the heart. The heart is located in thoracic tissue, the thoracic tissue is an electric conductor, and impedance of the thoracic tissue may also be considered as cardiac impedance (also referred to as heart impedance). Therefore, electrodes are separately placed on skin at two ends (for example, a left end and a right end, or an upper end and a lower end) of the thoracic tissue, and a low-amplitude constant current is input to the thoracic tissue by using the electrodes, so that a cardiac impedance curve of a user can be determined based on a current flowing through the thoracic tissue and a voltage between the two ends of the thoracic tissue. The cardiac impedance curve can be used to determine cardiac function indicators such as a cardiac output and a stroke volume.

Dermal resistance (dermal resistance): Skin can conduct electricity. The dermal resistance is a resistance of a cuticle on a skin surface. In embodiments of this application, two electrodes may be in contact with the skin, and conduct a current by using the skin. If a distance between the two electrodes is less than a preset distance (for example, 5 centimeters or 3 centimeters), a resistance provided by skin between the two electrodes may be referred to as a dermal resistance.

Body resistance: In embodiments of this application, two electrodes may be in contact with skin. If a connection line between two contact points of the two electrodes and the skin can cross two ends (for example, upper and lower ends or left and right ends) of thoracic tissue, a resistance provided by a body between the two electrodes may be referred to as a body resistance. Because the body includes a heart, the body resistance may also be referred to as cardiac impedance in embodiments of this application.

Electrocardiogram: A heart pumps blood rhythmically, and systole and diastole rhythms of the heart are controlled by a cardiac electrical activity. In a normal case, a sinoatrial node regularly generates impulses, and all myocardia generate electric impulses through a special conduction system. An electric field generated by such electric impulses is throughout parts of a body. A generated micro current is conducted to a body surface through body tissue, so that different parts of the body surface generate different potentials.

Electrocardiogram measurement principle: Electrocardiogram measurement is to measure a potential difference between different parts of a body surface by using electrodes in contact with skin, and determine an electrocardiogram of a user based on a relationship between the potential difference between the different parts and time. It can be learned from the foregoing noun explanation of the electrocardiogram that the potential difference between different parts of the body surface of the user is caused by regular impulses generated by the sinoatrial node in the heart. Therefore, when the potential difference between the different parts is measured, a plurality of electrodes may be placed in contact with skin at the different parts of the body, and the potential difference between the different parts is measured by using a voltage measurement unit.

The following describes a system architecture of a health monitoring system 1000 according to an embodiment of this application.

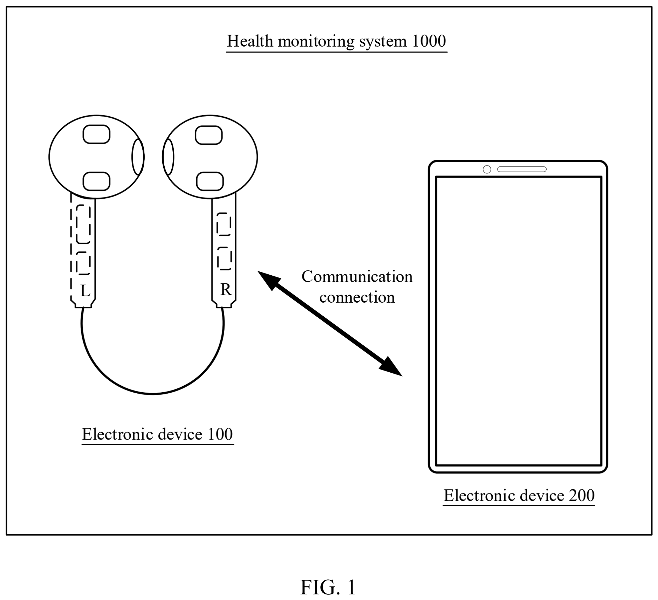

As shown in FIG. 1, the health monitoring system 1000 may include an electronic device 100 and an electronic device 200. A monitoring circuit may be disposed in the electronic device 100. The monitoring circuit may include a plurality of electrodes. When the plurality of electrodes are in contact with specified positions on skin of a user, the electronic device 100 may determine a cardiac impedance curve of the user by using the monitoring circuit. The cardiac impedance curve may be used to determine a cardiac function indicator of the user. The cardiac function indicator may include a cardiac output and a stroke volume. Optionally, the cardiac function indicator may further include a heart rate.

In some embodiments, after determining the cardiac impedance curve of the user, the electronic device 100 may determine and output the cardiac function indicator of the user based on the cardiac impedance curve of the user. After determining the cardiac function indicator, the electronic device 100 may output the cardiac function indicator. In some embodiments, the electronic device 100 may establish a communication connection to the electronic device 200. The communication connection may be a wired communication connection, or may be a wireless communication connection. The wireless communication connection may be a wireless communication connection established by the electronic device 100 and the electronic device 200 by using any one of wireless communication technologies such as a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, and NearLink (NearLink). In this case, that the electronic device 100 outputs the cardiac function indicator may be: sending an output instruction 1 to the electronic device 200, where the output instruction 1 may include the cardiac function indicator, and the output instruction 1 indicates the electronic device 200 to output the cardiac function indicator.

In some other embodiments, if the electronic device 100 establishes the communication connection to the electronic device 200, after determining the cardiac impedance curve of the user, the electronic device 100 may also send the cardiac impedance curve to the electronic device 200. The electronic device 200 may determine the cardiac function indicator of the user based on the cardiac impedance curve sent by the electronic device 100. Then, the electronic device 200 may output the cardiac function indicator, or may send an output instruction 2 to the electronic device 100. The output instruction 2 may include the cardiac function indicator, and the output instruction 2 may indicate the electronic device 100 to display the cardiac function indicator.

In the health monitoring system 1000, the electronic device 100 may be earphones shown in FIG. 1, or may be a wearable device such as a watch or a band, or may be an electronic device such as a mobile phone or a tablet computer. The electronic device 200 may be a mobile phone shown in FIG. 1, or may be a wearable device such as a watch or a band, or may be an electronic device such as a tablet computer. This is not limited in this application.

It may be understood that the health monitoring system 1000 shown in FIG. 1 is merely an example. In embodiments of this application, the health monitoring system 1000 may further include more or fewer electronic devices than or electronic devices different from those in the foregoing embodiment. This is not limited in this application.

The following describes diagrams of device forms of a plurality of types of electronic devices 100 according to an embodiment of this application.

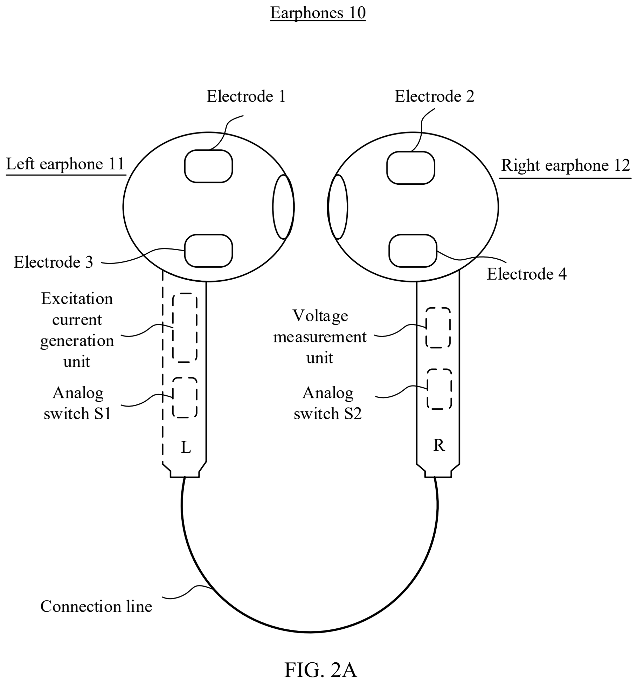

In some application scenarios, the electronic device 100 may be earphones 10. FIG. 2A to FIG. 2C are diagrams of device forms of three types of earphones 10 according to an embodiment of this application.

For example, the earphones 10 may be Bluetooth earphones shown in FIG. 2A.

As shown in FIG. 2A, the earphones 10 may include a left earphone 11 and a right earphone 12. There is a wired connection between the left earphone 11 and the right earphone 12. The wired connection may be implemented by using a connection line (for example, an electrical connection line) shown in FIG. 2A. It should be noted that the connection line between the left earphone 11 and the right earphone 12 may be configured to conduct a current.

One or more electrodes, for example, an electrode 1, may be disposed on a surface of the left earphone 11. When a left ear of a user wears the left earphone 11, the electrode 1 may be in contact with skin of the user. Similarly, one or more electrodes, for example, an electrode 2, may also be disposed on a surface of the right earphone 12. When a right ear of the user wears the right earphone 12, the electrode 2 may be in contact with the skin of the user. In some embodiments, an electrode 3 may be further disposed on the surface of the left earphone 11, and an electrode 4 may be further disposed on the surface of the right earphone 12. On the left earphone 11, the electrode 3 and the electrode 1 are not in contact with each other, and when the left ear of the user wears the left earphone 11, the electrode 3 may also be in contact with the skin of the user. On the right earphone 12, the electrode 4 and the electrode 2 are not in contact with each other, and when the right ear of the user wears the right earphone 12, the electrode 4 may also be in contact with the skin of the user.

The earphones 10 may further include an excitation current generation unit, a voltage measurement unit, one or more analog switches (for example, an analog switch S1 and an analog switch S2), and the like. The excitation current generation unit and the voltage measurement unit may be disposed inside the earphones 10, and the one or more analog switches may also be disposed inside the earphones 10, or may be disposed as visible mechanical switches outside the earphones 10. For example, in the embodiment shown in FIG. 2A, the excitation current generation unit and the analog switch S1 may be disposed inside the left earphone 11, and the voltage measurement unit and the analog switch S2 may be disposed inside the right earphone 12.

It may be understood that the embodiment shown in FIG. 2A is merely an example. In embodiments of this application, circuit components such as the excitation current generation unit, the voltage measurement unit, and the analog switch may be alternatively disposed at other positions inside the earphones 10, or the analog switch is disposed as a mechanical switch on a surface of the earphones 10. This is not limited in this application.

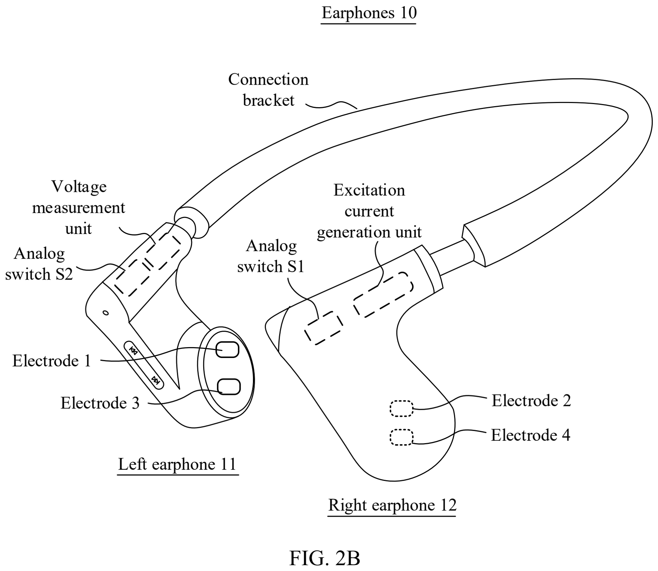

For another example, the earphones 10 may alternatively be Bluetooth earphones shown in FIG. 2B below.

As shown in FIG. 2B, the earphones 10 may include a left earphone 11 and a right earphone 12. There is a wired connection between the left earphone 11 and the right earphone 12. The wired connection may be implemented by using a connection bracket shown in FIG. 2B. It should be noted that the connection bracket between the left earphone 11 and the right earphone 12 may be configured to conduct a current. Compared with the connection line shown in FIG. 2A, the connection bracket has a specific support capability, and the support capability of the connection bracket can enable the connection bracket to maintain a specific shape (for example, an arc). In some embodiments, the connection bracket may further have a specific deformation capability. For example, the connection bracket is expanded into a straight line shape.

In the earphones 10 shown in FIG. 2B, one or more electrodes (for example, an electrode 1, an electrode 2, an electrode 3, and an electrode 4) may be disposed on surfaces of the left earphone 11 and the right earphone 12. For distribution positions of the electrodes, refer to related descriptions in the embodiment shown in FIG. 2A. In addition, the earphones 10 shown in FIG. 2B may also include circuit components such as an excitation current generation unit, a voltage measurement unit, and one or more analog switches (for example, an analog switch S1 and an analog switch S2). For positions of the plurality of circuit components, refer to related descriptions in the embodiment shown in FIG. 2A.

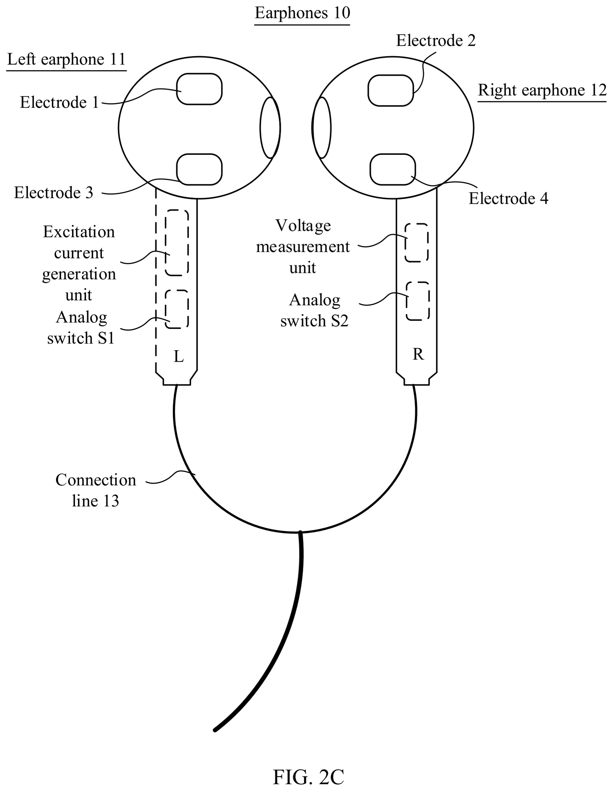

For another example, the earphones 10 may alternatively be wired earphones shown in FIG. 2C.

As shown in FIG. 2C, the earphones 10 may include a left earphone 11, a right earphone 12, and a connection line 13. The connection line 13 is a multi-end connection line, and the connection line 13 may include three ports. Two of the ports may be respectively connected to the left earphone 11 and the right earphone 12, and the remaining port may be vacant, or may be connected to another electronic device (for example, a mobile phone or a tablet computer). It should be noted that the connection line 13 may be configured to conduct a current.

In the earphones 10 shown in FIG. 2C, one or more electrodes (for example, an electrode 1, an electrode 2, an electrode 3, and an electrode 4) may be disposed on surfaces of the left earphone 11 and the right earphone 12. For distribution positions of the electrodes, refer to related descriptions in the embodiment shown in FIG. 2A. In addition, the earphones 10 shown in FIG. 2C may also include circuit components such as an excitation current generation unit, a voltage measurement unit, and one or more analog switches (for example, an analog switch S1 and an analog switch S2). For positions of the plurality of circuit components, refer to related descriptions in the embodiment shown in FIG. 2A.

It may be understood that the embodiments shown in FIG. 2A to FIG. 2C are merely some examples. In embodiments of this application, the earphones 10 may alternatively be in a device form different from that in the foregoing embodiments, for example, over-ear headphones. This is not limited in this application.

It should be noted that in the earphones 10 shown in FIG. 2A to FIG. 2C, the connection line or the connection bracket between the left earphone 11 and the right earphone 12 is deformable. Therefore, a distance between the left earphone 11 and the right earphone 12 is adjustable, and a distance between the electrode on the left earphone 11 and the electrode on the right earphone 12 is also adjustable. In this way, in a measurement process, the user may adjust the distance between the left earphone 11 and the right earphone 12 according to an actual measurement scenario, to adjust the distance between the electrode on the left earphone 11 and the electrode on the right earphone 12, so that a more accurate result is measured.

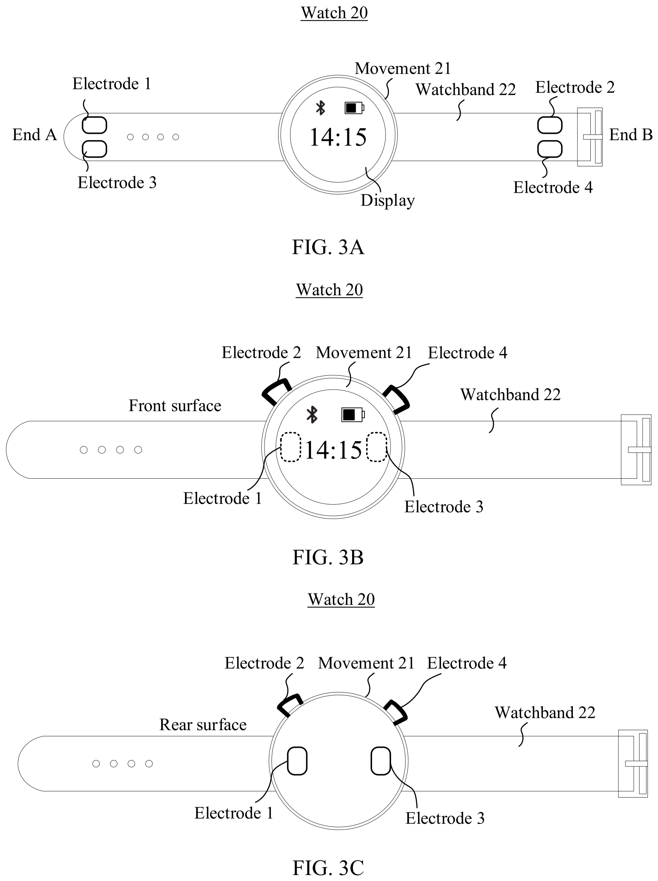

In some application scenarios, the electronic device 100 may be a watch 20. The watch 20 may include a movement and a watchband. A plurality of electrodes of the watch 20 may be disposed on the watchband, or may be disposed on the movement, or may be separately disposed on the movement and the watchband. FIG. 3A to FIG. 3E are diagrams of device forms of three types of watches 20 according to an embodiment of this application.

For example, as shown in FIG. 3A, the watch 20 may include a movement 21 and a watchband 22. The movement 21 may include a display. The display may be configured to display a monitoring result, and may be further configured to display information such as time. The watch 20 may include a plurality of electrodes, for example, an electrode 1 and an electrode 2, and optionally, may further include an electrode 3 and an electrode 4. The plurality of electrodes may be disposed on the watchband 22.

The watchband 22 may include a front surface and a rear surface. When the display of the watch 20 is placed on a desktop (or another horizontal plane) in an upward direction, an upward surface of the watchband 22 is the front surface of the watchband 22, and a surface that is of the watchband 22 and that is in contact with the desktop (or the another horizontal plane) is the rear surface of the watchband 22. A plurality of electrodes may be disposed on the watchband 22, and the plurality of electrodes may be disposed on a same surface of the watchband 22. For example, the plurality of electrodes may be all disposed on the front surface of the watchband 22, or may be all disposed on the rear surface of the watchband 22. In addition, the watchband 22 may include two ends, that is, an end A and an end B shown in FIG. 3A. The electrode 1 and the electrode 2 may be separately disposed at different ends of the watchband 22. For example, in the embodiment shown in FIG. 3A, the electrode 1 and the electrode 3 may be disposed at the end A of the watchband 22, the electrode 2 and the electrode 4 may be disposed at the end B of the watchband 22, and the plurality of electrodes are all disposed on the front surface of the watchband 22. In addition, the plurality of electrodes disposed on the watchband 22 are not in contact with each other.

It should be noted that, in the foregoing case, a distance between the electrode 1 and the electrode 2 needs to be greater than a minimum length, and the minimum length may be a preset average length of hearts of human bodies. In this way, during measurement, the electrode 1 and the electrode 2 can cross two ends of a heart to determine cardiac impedance. In some other embodiments, the distance between the electrode 1 and the electrode 2 further needs to be less than or equal to a maximum length. The maximum length may be a length of the watchband 22, or may be a preset length value. When it is ensured that the electrode 1 and the electrode 2 can cross the two ends of the heart, shorter distances between the heart and both the electrode 1 and the electrode 2 indicate a more accurate measurement result. In this way, accuracy of the measurement result can be improved.

It may be understood that the watch 20 shown in FIG. 3A is merely an example. In some embodiments, one or more buttons (for example, a watch crown) may be further disposed on the movement 21. This is not limited in this application. In addition, shapes of the movement 21 and the display, a shape and a length of the watchband 22, and the like are not limited in this application.

In some other embodiments, a plurality of electrodes in the watch 20 may be alternatively disposed on the movement 21, for example, separately disposed on a rear surface of the movement 21 and a button of the movement 21. The movement 21 may include a front surface and the rear surface. When the user wears the watch 20, a surface that is in contact with skin of the user is the rear surface of the movement 21, and a surface opposite to the rear surface is the front surface of the movement 21. For example, for electrode distribution on the button of the movement 21, refer to the following embodiment shown in FIG. 3B, and for electrode distribution on the rear surface of the movement 21, refer to the following embodiment shown in FIG. 3C.

As shown in FIG. 3B, in the watch 20, one or more buttons (including a watch crown and the like) may be disposed on the movement 21, and an electrode may be disposed on one or more of the one or more buttons. For example, an electrode 2 may be disposed on one of the buttons. Optionally, an electrode 4 may be further disposed on another button. The electrode 2 and the electrode 4 are not in contact with each other. In addition, an electrode 1 may be disposed on the rear surface of the movement 21. Optionally, an electrode 3 may be further disposed. The electrode 1 and the electrode 3 are not in contact with each other.

As shown in FIG. 3C, in the watch 20, an electrode 1 may be disposed on the rear surface of the movement 21. Optionally, an electrode 3 may be further disposed. When the electrode 1 and the electrode 3 are disposed on the rear surface of the movement 21, the electrode 1 and the electrode 3 are not in contact with each other. In addition, one or more buttons (including a watch crown and the like) may be disposed on the movement 21, and an electrode may be disposed on one or more of the one or more buttons. For example, an electrode 2 may be disposed on one of the buttons. Optionally, an electrode 4 may be further disposed on another button. The electrode 2 and the electrode 4 are not in contact with each other.

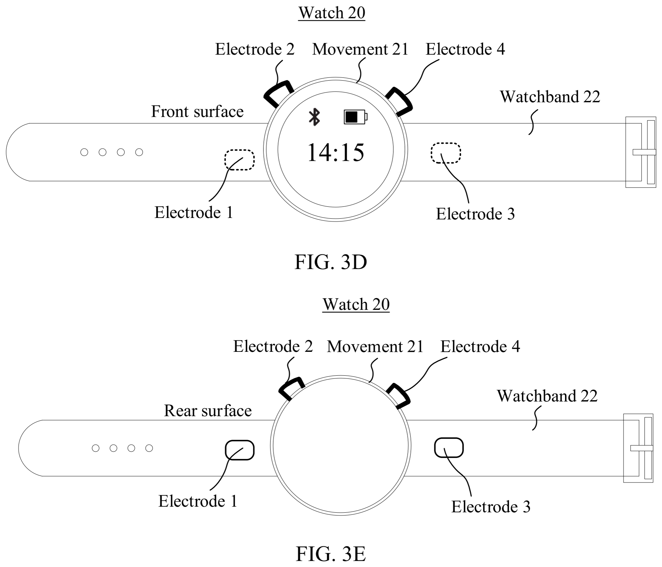

In some other embodiments, a plurality of electrodes in the watch 20 may be alternatively separately disposed on the movement 21 and the watchband 22. For example, for electrode distribution on the movement 21, refer to the following embodiment shown in FIG. 3D, and for electrode distribution on the watchband 22, refer to the following embodiment shown in FIG. 3E.

As shown in FIG. 3D, in the watch 20, one or more buttons (including a watch crown and the like) may be disposed on the movement 21, and an electrode may be disposed on one or more of the one or more buttons. For example, an electrode 2 may be disposed on one of the buttons. Optionally, an electrode 4 may be further disposed on another button. The electrode 2 and the electrode 4 are not in contact with each other. In addition, an electrode 1 may be disposed on the watchband 22. Optionally, an electrode 3 may be further disposed on the watchband 22. The electrode 1 and the electrode 3 are not in contact with each other. It should be noted that an electrode on the watchband 22 is disposed on the rear surface of the watchband 22, and the rear surface of the watchband 22 is a surface on which the watchband 22 is in contact with a wrist of the user when the user wears the watch 20. In addition, when the user wears the watch 20, the electrode 1 and the electrode 3 can be in contact with the wrist of the user.

As shown in FIG. 3E, in the watch 20, an electrode 1 may be disposed on the rear surface of the watchband 22. Optionally, an electrode 3 may be further disposed on the rear surface of the watchband 22. Position relationships of the electrode 1 and the electrode 3 relative to the movement 21 may be the same. For example, both electrodes are located on a left side (or a right side) of the movement 21. Position relationships of the electrode 1 and the electrode 3 relative to the movement 21 may alternatively be different. For example, the electrode 1 is located on a left side of the movement 21, and the electrode 3 is located on a right side of the movement 21. When the electrode 1 and the electrode 3 are disposed on the rear surface of the watchband 22, the electrode 1 and the electrode 3 are not in contact with each other. In addition, one or more buttons (including a watch crown and the like) may be disposed on the movement 21, and an electrode may be disposed on one or more of the one or more buttons. For example, an electrode 2 may be disposed on one of the buttons. Optionally, an electrode 4 may be further disposed on another button. The electrode 2 and the electrode 4 are not in contact with each other.

In some other embodiments, the electrode 1 and the electrode 3 may be alternatively disposed on the front surface of the watchband 22, and the front surface of the watchband 22 is a surface opposite to the rear surface of the watchband 22. In this case, the electrode 2 and the electrode 4 may be disposed on the rear surface of the movement 21, or disposed on a surface on which the button is in contact with the wrist of the user. This is not limited in this application.

It may be understood that FIG. 3A to FIG. 3E are merely three examples. In embodiments of this application, the electrode may be alternatively disposed at another position on the watch 20. In addition, the watch 20 in the foregoing embodiment may be replaced with a wearable device such as a band. This is not limited in this application.

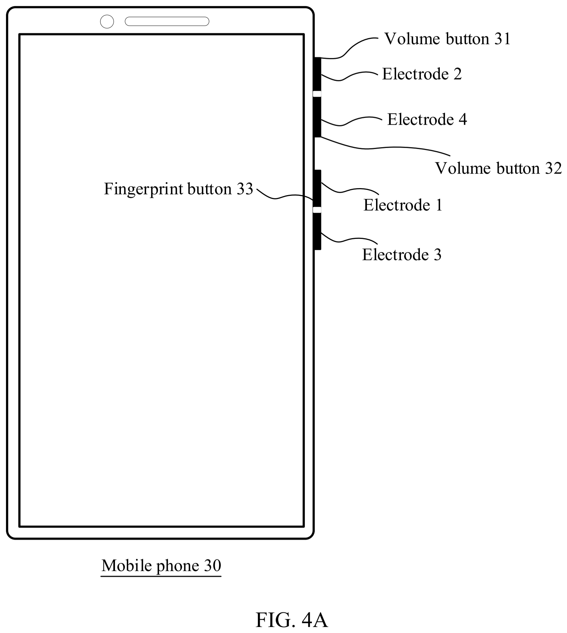

FIG. 4A is a diagram of a device form of a mobile phone 30 according to an embodiment of this application.

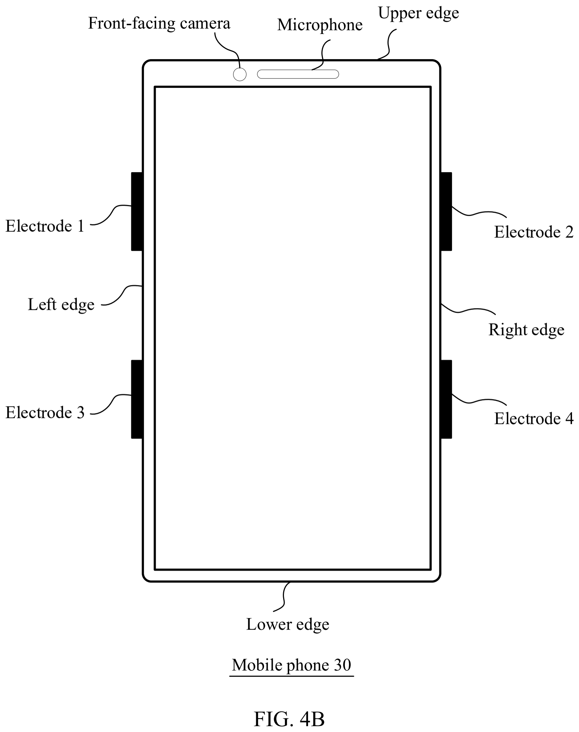

As shown in FIG. 4B, the mobile phone 30 may include one or more buttons and a plurality of electrodes. One or more of the plurality of electrodes may be disposed on the one or more buttons. For example, the one or more buttons may include a volume button 31, a volume button 32, a fingerprint button 33, and the like. The plurality of electrodes may include an electrode 1 and an electrode 2. Optionally, the plurality of electrodes may further include an electrode 3 and an electrode 4. The plurality of electrodes may be separately disposed on different buttons, or may be separately disposed in different regions of a same button. For example, the electrode 1 may be disposed on the volume button 31, and the electrode 3 may be disposed on the volume button 32. The electrode 2 may be disposed in an upper half region of the fingerprint button 33, and the electrode 4 may be disposed in a lower half region of the fingerprint button 33. It should be noted that the electrode 1 and the electrode 3 are not in contact with each other, and the electrode 2 and the electrode 4 are not in contact with each other.

FIG. 4B is a diagram of a device form of another mobile phone 30 according to an embodiment of this application.

As shown in FIG. 4B, the mobile phone 30 may include a plurality of electrodes, a front-facing camera, a microphone, and the like. The plurality of electrodes may include an electrode 1 and an electrode 2, and optionally, may further include an electrode 3 and an electrode 4. The mobile phone 30 may have four edges: an upper edge, a lower edge, a left edge, and a right edge. The upper edge is an edge closest to the front-facing camera and/or the microphone, the lower edge is an edge parallel to the upper edge, the left edge and the right edge are two parallel edges, and both the left edge and the right edge are perpendicular to the upper edge. The plurality of electrodes of the mobile phone 30 may be separately disposed on the left edge and the right edge of the mobile phone 30. For example, the electrode 1 and the electrode 3 are disposed on the left edge of the mobile phone 30, and the electrode 2 and the electrode 4 are disposed on the right side of the mobile phone 30. It should be noted that the electrode 1 and the electrode 3 are not in contact with each other, and the electrode 2 and the electrode 4 are not in contact with each other.

It may be understood that FIG. 4A and FIG. 4B are merely two examples. In some embodiments, the mobile phone 30 may alternatively be a mobile phone having a foldable screen, or may include more or fewer buttons than or buttons different from those in the embodiment shown in FIG. 4A, or may be another mobile phone in a form different from that shown in the foregoing embodiment. In addition, the electrodes in the mobile phone 30 may be alternatively disposed at positions different from those in the foregoing embodiment (for example, separately disposed on the upper edge and the lower edge of the mobile phone 30, or disposed on a rear surface of the mobile phone 30). This is not limited in this application. In some other embodiments, the mobile phone 30 may also be replaced with an electronic device such as a tablet computer. This is not limited in this application either.

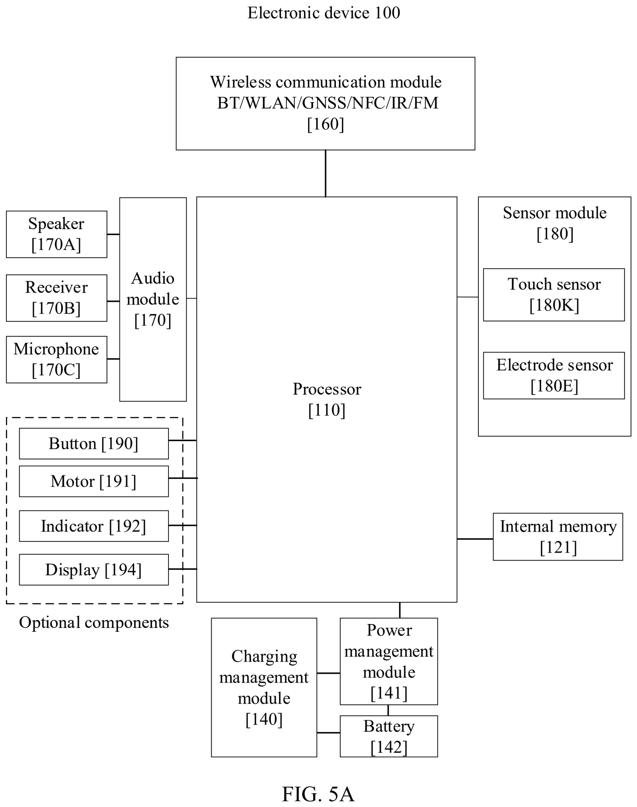

FIG. 5A is a diagram of a hardware structure of an electronic device 100 according to an embodiment of this application.

The electronic device 100 may be the earphones 10 in the embodiments shown in FIG. 2A to FIG. 2C, or may be the watch 20 (or another wearable device such as a band) in the embodiments shown in FIG. 3A to FIG. 3C, or may be the mobile phone shown in FIG. 4A and FIG. 4B, or the like. In some embodiments, the electronic device 100 may alternatively be a tablet computer, a handheld computer, a cellular phone, a personal digital assistant (personal digital assistant, PDA), an augmented reality (augmented reality, AR) device, a virtual reality (virtual reality, VR) device, an artificial intelligence (artificial intelligence, AI) device, an in-vehicle device, a smart home device, and/or a smart city device. A specific type of the electronic device is not particularly limited in embodiments of this application.

As shown in FIG. 5A, the electronic device 100 may include a processor 110, an internal memory 121, a charging management module 140, a power management module 141, a battery 142, a wireless communication module 160, an audio module 170, and a sensor module 180. In some embodiments, the electronic device 100 may further include any one or more of the following: a button 190, a motor 191, an indicator 192, a display 194, and the like. The sensor module 180 may include an electrode sensor 180E and a touch sensor 180K. In some embodiments, the sensor module 180 may further include any one or more of the following: a pressure sensor, a gyro sensor, a barometric pressure sensor, a magnetic sensor, an acceleration sensor, a distance sensor, an optical proximity sensor, a fingerprint sensor, a temperature sensor, an ambient light sensor, a bone conduction sensor, an airbag sensor, and the like.

It may be understood that the structure shown in this embodiment of the present invention does not constitute a specific limitation on the electronic device 100. In some other embodiments of this application, the electronic device 100 may include more or fewer components than those shown in the figure, or combine some components, or split some components, or have different component arrangements. The components shown in the figure may be implemented by hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, and/or a neural-network processing unit (neural-network processing unit, NPU). Different processing units may be independent components, or may be integrated into one or more processors.

The controller may generate an operation control signal based on instruction operation code and a time sequence signal, to complete control of instruction fetching and instruction execution.

A memory may be further disposed in the processor 110, to store instructions and data. In some embodiments, the memory in the processor 110 is a cache. The memory may store instructions or data just used or cyclically used by the processor 110. If the processor 110 needs to use the instructions or data again, the instructions or data may be directly invoked from the memory. This avoids repeated access, and reduces waiting time of the processor 110, so that system efficiency is improved.

In some embodiments, the processor 110 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like.

The charging management module 140 is configured to receive charging input from a charger. The charger may be a wireless charger, or may be a wired charger. In some wired charging embodiments, the charging management module 140 may receive charging input from a wired charger. In some wireless charging embodiments, the charging management module 140 may receive wireless charging input through a wireless charging coil of the electronic device 100. When charging the battery 142, the charging management module 140 may further supply power to the electronic device through the power management module 141.

The power management module 141 is configured to connect to the battery 142, the charging management module 140, and the processor 110. The power management module 141 receives input of the battery 142 and/or the charging management module 140, and supplies power to the processor 110, the internal memory 121, the display 194, the wireless communication module 160, and the like. The power management module 141 may be further configured to monitor parameters such as a battery capacity, a battery cycle count, and a battery health status (leakage or impedance). In some other embodiments, the power management module 141 may alternatively be disposed in the processor 110. In some other embodiments, the power management module 141 and the charging management module 140 may alternatively be disposed in a same component.

The wireless communication module 160 may provide a solution for wireless communication that is applied to the electronic device 100 and that includes a wireless local area network (wireless local area network, WLAN) (such as a wireless fidelity (wireless fidelity, Wi-Fi) network), Bluetooth (Bluetooth, BT), a global navigation satellite system (global navigation satellite system, GNSS), frequency modulation (frequency modulation, FM), near field communication (near field communication, NFC), an infrared (infrared, IR) technology, NearLink (NearLink), and the like. The wireless communication module 160 may be one or more components that integrate at least one communication processing module.

The electronic device 100 implements a display function by using the GPU, the display 194, the application processor, and the like. The GPU is a microprocessor for image processing, and is connected to the display 194 and the application processor. The GPU is configured to perform mathematical and geometric calculation and render graphics. The processor 110 may include one or more GPUs that execute program instructions to generate or change display information.

The display 194 is configured to display an image, a video, and the like. The display 194 includes a display panel. The display panel may be manufactured by using a liquid crystal display (liquid crystal display, LCD), an organic light-emitting diode (organic light-emitting diode, OLED), an active-matrix organic light-emitting diode (active-matrix organic light-emitting diode, AMOLED), a flexible light-emitting diode (flexible light-emitting diode, FLED), a mini-LED, a micro-LED, a micro-OLED, a quantum dot light-emitting diode (quantum dot light-emitting diode, QLED), or the like. In some embodiments, the electronic device 100 may include one or N displays 194, where N is a positive integer greater than 1.

The internal memory 121 may include one or more random access memories (random access memory, RAM) and one or more non-volatile memories (non-volatile memory, NVM). The random access memory may be directly read and written by the processor 110, may be configured to store an executable program (for example, a machine instruction) of an operating system or another running program, and may be further configured to store data of a user and an application. The non-volatile memory may also store an executable program, data of a user and an application, and the like, and may be loaded into the random access memory in advance, for direct reading and writing by the processor 110.

The electronic device 100 may implement an audio function, for example, music playing or recording, by using the audio module 170, the application processor, and the like. The audio module 170 may include any one or more of the following: a speaker 170A, a receiver 170B, and a microphone 170C.

The audio module 170 is configured to convert digital audio information into an analog audio signal for output, and is also configured to convert analog audio input into a digital audio signal. The audio module 170 may be further configured to encode and decode audio signals. In some embodiments, the audio module 170 may be disposed in the processor 110, or some functional modules of the audio module 170 may be disposed in the processor 110.

The speaker 170A, also referred to as a "loudspeaker", is configured to convert an audio electrical signal into a sound signal. The electronic device 100 may be used to listen to music or answer a call in a hands-free mode over the speaker 170A.

The receiver 170B, also referred to as an "earpiece", is configured to convert an audio electrical signal into a sound signal. When a call is answered or a voice message is listened to by using the electronic device 100, the receiver 170B may be put close to a human ear to listen to a voice.

The microphone 170C, also referred to as a "mike" or a "mic", is configured to convert a sound signal into an electrical signal. When making a call or sending a voice message, the user may make a sound near the microphone 170C through the mouth, to enter a sound signal to the microphone 170C. At least one microphone 170C may be disposed on the electronic device 100. In some other embodiments, two microphones 170C may be disposed on the electronic device 100. In addition to sound signal collection, a noise reduction function may be further implemented. In some other embodiments, three, four, or more microphones 170C may be alternatively disposed on the electronic device 100, to collect a sound signal, implement noise reduction, recognize a sound source, implement a directional recording function, and the like.

In some embodiments, the audio module 170 may further include a headset jack, and the headset jack is configured to connect to a wired headset. The headset jack may be the USB interface, or may be a 3.5 mm open mobile terminal platform (open mobile terminal platform, OMTP) standard interface or a cellular telecommunications industry association of the USA (cellular telecommunications industry association of the USA, CTIA) standard interface.

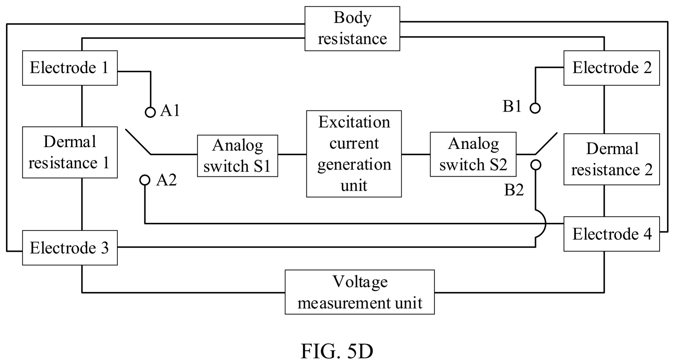

The electrode sensor 180E may be a plurality of electrodes in a monitoring circuit, for example, the electrode 1, the electrode 2, the electrode 3, and the electrode 4 in the embodiment shown in FIG. 2A. For specific content of the monitoring circuit, refer to related descriptions in the following embodiments shown in FIG. 5D to FIG. 5H. Details are not described herein.

The touch sensor 180K is also referred to as a "touch device". The touch sensor 180K may be disposed on the display 194. The touch sensor 180K and the display 194 form a touchscreen, which is also referred to as a "touch screen". The touch sensor 180K is configured to detect a touch operation performed on or near the touch sensor. The touch sensor may transfer the detected touch operation to the application processor to determine a type of a touch event. Visual output related to the touch operation may be provided by using the display 194. In some other embodiments, the touch sensor 180K may alternatively be disposed on a surface of the electronic device 100 at a position different from that of the display 194.

Optionally, the sensor module 180 may further include but is not limited to any one or more of the following: a pressure sensor, a gyro sensor, a barometric pressure sensor, a magnetic sensor, an acceleration sensor, a distance sensor, an optical proximity sensor, an ambient light sensor, a fingerprint sensor, a temperature sensor, a bone conduction sensor, a barometric pressure sensor, and the like.

The pressure sensor is configured to sense a pressure signal, and may convert the pressure signal into an electrical signal. In some embodiments, the pressure sensor may be disposed on the display 194. When a touch operation is performed on the display 194, the electronic device 100 detects strength of the touch operation through the pressure sensor. The electronic device 100 may also calculate a position of the touch based on a detection signal of the pressure sensor. In some embodiments, touch operations performed on a same touch position but has different touch operation strength may correspond to different operation instructions.

The gyro sensor may be configured to determine a motion gesture of the electronic device 100. In some embodiments, angular velocities of the electronic device 100 around the three axes (that is, the x-axis, the y-axis, and the z-axis) may be determined by using the gyro sensor.

The barometric pressure sensor is configured to measure barometric pressure. In some embodiments, the electronic device 100 calculates an altitude based on a barometric pressure value measured by the barometric pressure sensor, to assist in positioning and navigation.

The magnetic sensor includes a Hall sensor. In some embodiments, the electronic device 100 may implement a function such as a compass by using the magnetic sensor.

The acceleration sensor may detect values of acceleration of the electronic device 100 in all directions (usually on three axes). When the electronic device 100 is static, the acceleration sensor can detect magnitude and a direction of gravity. The acceleration sensor may be further configured to recognize a posture of the electronic device, and is applied to applications such as switching between landscape mode and vertical mode and a pedometer.

The distance sensor is configured to measure a distance. The electronic device 100 may measure a distance through infrared or laser.

The optical proximity sensor may include, for example, a light-emitting diode (LED) and a light detector such as a photodiode. The light-emitting diode may be an infrared light-emitting diode. The electronic device 100 emits infrared light by using the light-emitting diode. The electronic device 100 detects infrared reflected light from a nearby object by using the photodiode. When sufficient reflected light is detected, the electronic device 100 may determine that there is an object near the electronic device 100. When insufficient reflected light is detected, the electronic device 100 may determine that there is no object near the electronic device 100.

The ambient light sensor is configured to sense ambient light brightness.

The fingerprint sensor is configured to collect a fingerprint. The electronic device 100 may implement fingerprint-based unlocking, application lock access, fingerprint-based photographing, fingerprint-based call answering, or the like based on a feature of the collected fingerprint.

The temperature sensor is configured to detect a temperature. In some embodiments, the electronic device 100 executes a temperature processing policy based on the temperature detected by the temperature sensor. In some embodiments, the temperature sensor may be configured to detect a temperature of the electronic device 100. In some embodiments, the temperature sensor may also be configured to detect a body temperature of a user. In some other embodiments, the temperature sensor may be further configured to detect a temperature of an environment in which the electronic device 100 is located.

The bone conduction sensor may obtain a vibration signal. In some embodiments, the bone conduction sensor may also be in contact with a human pulse, and receive a blood pressure beating signal. In some embodiments, the application processor may parse heart rate information based on the blood pressure beating signal obtained by the bone conduction sensor 180M, to implement a heart rate detection function.

The airbag sensor may be configured to detect blood pressure of the user.

The button 190 includes a power button, a volume button, and the like. The button 190 may be a mechanical button, or may be a touch button. The electronic device 100 may receive button input, and generate button signal input related to user settings and function control of the electronic device 100.

The motor 191 may generate a vibration prompt. The motor 191 may be used for an incoming call vibration prompt, or may be used for touch vibration feedback. For example, touch operations performed on different applications (for example, photo taking and audio playing) may be corresponding to different vibration feedback effects. The motor 191 may also correspond to different vibration feedback effects for touch operations performed in different regions of the display 194. Different application scenarios (for example, a time reminder, information receiving, an alarm clock, and a game) may also be corresponding to different vibration feedback effects. A touch vibration feedback effect may be further customized.

The indicator 192 may be an indicator light, and may indicate a charging status or a power change, or may indicate a message, a missed call, a notification, or the like.

It should be noted that, in embodiments of this application, for a hardware structure of the electronic device 200, refer to the hardware structure of the electronic device 100 shown in FIG. 5A. Details are not described again in this application.

An embodiment of this application provides a monitoring method. The electronic device 100 may include a monitoring circuit. The monitoring circuit may include an electrode 1 and an electrode 2. The electrode 1 and the electrode 2 are respectively disposed at different positions on a surface of the electronic device 100. The electronic device 100 may receive and respond to an operation of starting cardiac function monitoring by a user, or receive and respond to a start instruction sent by the electronic device 200, and determine and output a cardiac function indicator of the user by using the electrode 1 and the electrode 2. The cardiac function indicator may include a cardiac output and a stroke volume. When the electronic device 100 performs cardiac function monitoring, the electrode 1 is in contact with a contact point 1 on skin, the electrode 2 is in contact with a contact point 2 on the skin, and the contact point 1 and the contact point 2 are respectively located at two ends (for example, an upper end and a lower end, or a left end and a right end) of thoracic tissue. The electronic device 100 may be a portable device such as earphones, a watch, a band, or a mobile phone.

In this way, the user may perform cardiac function monitoring by using the electronic device 100. In addition, because the electronic device 100 is a device frequently used in daily life and is easy to carry, the user can obtain a cardiac function monitoring result in real time.

The following describes the monitoring circuit in the electronic device 100 according to an embodiment of this application.

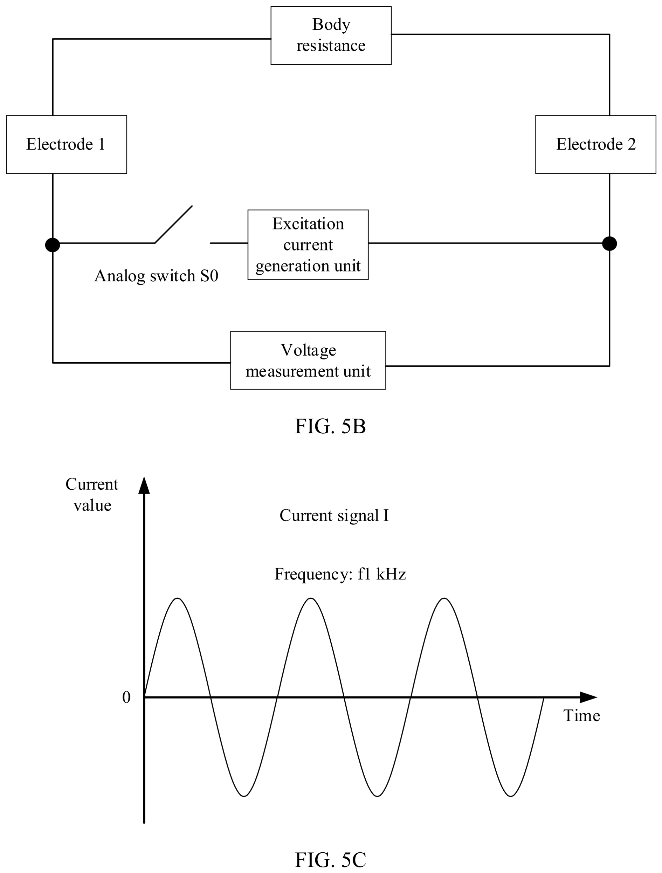

FIG. 5B is a diagram of a structure of a monitoring circuit according to an embodiment of this application.

As shown in FIG. 5B, the monitoring circuit may include an excitation current generation unit, a voltage measurement unit, an electrode 1, an electrode 2, and an analog switch S0.

Two ends of the analog switch S0 may be respectively connected to the excitation current generation unit and the electrode 1.

The excitation current generation unit may include two ends: a positive electrode and a negative electrode, and the positive electrode and the negative electrode may be respectively connected to the analog switch S0 and the electrode 2. The excitation current generation unit may generate a constant current. For a current signal generated by the excitation current generation unit, refer to related descriptions in the following embodiment shown in FIG. 5D. Details are not described herein.

The voltage measurement unit may be connected to the excitation current generation unit in a parallel manner. For example, the voltage measurement unit may include two ends: a positive electrode and a negative electrode. One end may be connected to the electrode 2 and the negative electrode of the excitation current generation unit, and the other end may be connected to the positive electrode of the excitation current generation unit. The voltage measurement unit may measure a voltage between two ends of the electrode 1 and the electrode 2.

When both the electrode 1 and the electrode 2 are in good contact with skin of a user, and contact positions of the electrode 1 and the electrode 2 and the skin are respectively at two ends of thoracic tissue, because a resistance exists in a human body, the electrode 1 may be considered as being connected to the electrode 2 by using the body resistance. In this case, if the analog switch S0 is turned on, a loop may be formed between the electrode 1 and the electrode 2. Cardiac impedance of the user can be determined by measuring a current flowing through the body resistance and a voltage between two ends of the body resistance in the loop. The cardiac impedance of the user is a ratio of the voltage between the two ends of the body resistance to the current flowing through the body resistance.

It should be noted that, cardiac function measurement needs to determine the cardiac impedance of the user. Therefore, a current flowing through a heart of the user and a voltage between two ends of the heart of the user need to be determined. Compared with the electrocardiogram measurement principle, the cardiac function measurement requires the excitation current generation unit to generate the current flowing through the heart of the user. Therefore, the monitoring circuit for measuring the cardiac impedance of the user needs to include the excitation current generation unit, while a circuit for measuring an electrocardiogram does not need an excitation current generation unit.

It may be understood that FIG. 5B merely describes an example of a monitoring circuit that can measure cardiac impedance of a user. In embodiments of this application, the monitoring circuit may further include more circuit components (for example, an electrode) than those in the embodiment shown in FIG. 5B, or include circuit components different from those in the foregoing embodiment. This is not limited in this application.

FIG. 5C is a diagram of a current signal generated by an excitation current generation unit according to an embodiment of this application.

As shown in FIG. 5C, in a two-dimensional coordinate system, a horizontal axis may represent time, and a vertical axis may represent a current value. The current signal generated by the excitation current generation unit may be a current signal I shown in FIG. 5C. The current signal I may be a sinusoidal signal with a constant frequency, and the frequency of the current signal I may be f1 kilohertz (kHz). In some embodiments, f1 may be greater than or equal to 1.

It may be understood that the current signal shown in FIG. 5C is merely an example. In some other embodiments, the current signal may alternatively be a cosine signal, a square wave signal, or the like. This is not limited in this application.

In some embodiments, the monitoring circuit in the electronic device 100 not only can measure the cardiac impedance of the user, but also can determine whether an electrode is in good contact with the skin of the user.

For example, FIG. 5D is a diagram of a structure of another monitoring circuit according to an embodiment of this application.

As shown in FIG. 5D, the monitoring circuit may include an excitation current generation unit, a voltage measurement unit, one or more analog switches, and a plurality of electrodes. The one or more analog switches may include an analog switch S1 and an analog switch S2, and the plurality of electrodes may include an electrode 1, an electrode 2, an electrode 3, and an electrode 4.

In the monitoring circuit, the excitation current generation unit may include two ends: a positive electrode and a negative electrode, and the positive electrode and the negative electrode may be respectively connected to the analog switch S2 and the analog switch S1. For example, the positive electrode may be connected to the analog switch S2, and the negative electrode may be connected to the analog switch S1. The excitation current generation unit may generate a current. It should be noted that, for a diagram of a waveform of the current signal generated by the excitation current generation unit, refer to related descriptions in the embodiment shown in FIG. 5C. In some embodiments, in different scenarios, frequencies of current signals generated by the excitation current generation unit may be different. For example, when the monitoring circuit measures cardiac impedance of a user, a frequency of a current signal generated by the excitation current generation unit may be greater than or equal to 1 kHz. When the monitoring circuit determines whether an electrode is in good contact with skin of a user, a frequency of a current signal may be less than 1 kHz. In some other embodiments, the excitation current generation unit may generate a same current signal in different scenarios, for example, generate a current signal whose frequency is greater than or equal to 1 kHz. This is not limited in this application.

One end of the voltage measurement unit may be connected to the electrode 4, and the other end may be connected to the electrode 3. The voltage measurement unit may measure a voltage between two ends of the electrode 3 and the electrode 4. In some other embodiments, the two ends of the voltage measurement unit may be alternatively respectively connected to the electrode 1 and the electrode 2. In this case, the voltage measurement unit may measure a voltage between two ends of the electrode 1 and the electrode 2.

The analog switch S1 may include two ports: a port A1 and a port A2. The port A1 may be connected to the electrode 1, and the port A2 may be connected to the electrode 4. The analog switch S1 may choose to be electrically connected to the port A1 or the port A2, or may choose not to be electrically connected to any port.

The analog switch S2 may include two ports: a port B1 and a port B2. The port B1 may be connected to the electrode 2, and the port B2 may be connected to the electrode 3. The analog switch S2 may choose to be electrically connected to the port B1 or the port B2, or may choose not to be electrically connected to any port.

When none of the electrodes in the monitoring circuit is in contact with the skin of the user (or contact is poor), because any two electrodes are in contact with each other, any two electrodes in the monitoring circuit are not connected to each other. When the plurality of electrodes in the monitoring circuit are in good contact with the skin of the user, because there is a resistance (for example, a dermal resistance or a body resistance) in a human body, the electrode in the monitoring circuit may be connected to another electrode by using the skin, the body, or the like.

In this embodiment of this application, a contact point between the electrode 1 and the skin of the user may be referred to as a contact point 1, a contact point between the electrode 2 and the skin of the user may be referred to as a contact point 2, a contact point between the electrode 3 and the skin of the user may be referred to as a contact point 3, and a contact point between the electrode 4 and the skin of the user may be referred to as a contact point 4. When all electrodes in the monitoring circuit are in contact with the skin of the user, because a distance between the electrode 1 and the electrode 3 is relatively short, and a distance between the electrode 2 and the electrode 4 is relatively short, the electrode 1 and the electrode 3 may be considered as being connected by using a dermal resistance 1. The dermal resistance 1 is a resistance of skin between the electrode 1 and the electrode 3. The electrode 2 and the electrode 4 may be considered as being connected by using a dermal resistance 2. The dermal resistance 2 is a resistance of skin between the electrode 2 and the electrode 4. Similarly, it may also be considered that the electrode 1 and the electrode 2 are connected to the electrode 3 and the electrode 4 by using a dermal resistance. If the contact point 1 and the contact point 2 are respectively located at two ends of thoracic tissue, a resistance between the contact point 1 and the contact point 2 may be equivalent to the body resistance (also referred to as a cardiac resistance) of the user. In this case, the electrode 1 and the electrode 2 may be considered as being connected by using the body resistance, and the electrode 3 and the electrode 4 may also be considered as being connected by using the body resistance. In this case, the monitoring circuit can determine the cardiac impedance of the user, to determine a cardiac output and a stroke volume of the user.

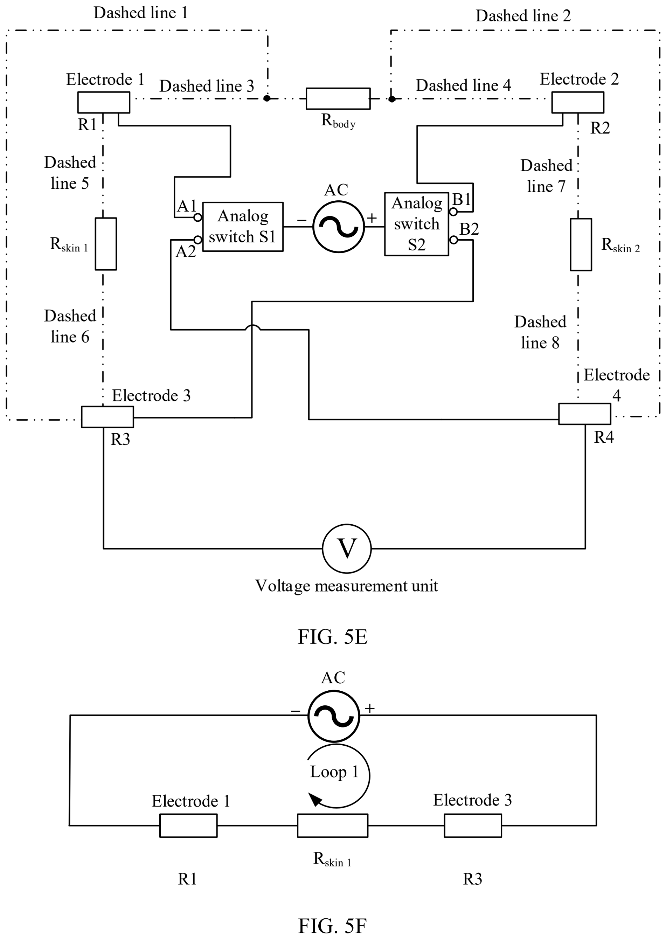

FIG. 5E is a diagram of a circuit connection of a monitoring circuit according to an embodiment of this application.

As shown in FIG. 5E, the monitoring circuit may include the following circuit components: an electrode 1, an electrode 2, an electrode 3, an electrode 4, an analog switch S1, an analog switch S2, an excitation current generation unit AC, a voltage measurement unit V, and the like.

A positive electrode of the voltage measurement unit V may be connected to the electrode 4, and a negative electrode of the voltage measurement unit V may be connected to the electrode 3. The voltage measurement unit V may be configured to measure a voltage between two ends of the electrode 3 and the electrode 4. In some other embodiments, the positive electrode of the voltage measurement unit may be alternatively connected to the electrode 3, and the negative electrode of the voltage measurement unit may be connected to the electrode 4.

A positive electrode of the excitation current generation unit AC may be connected to the analog switch S2, and a negative electrode of the excitation current generation unit AC may be connected to the analog switch S1. The analog switch S1 may include two ports: a port A1 and a port A2. The port A1 may be connected to the electrode 1, and the port A2 may be connected to the electrode 4. The analog switch S1 may receive an instruction sent by the electronic device 100 (or another electronic device), and in response to the instruction, choose to be electrically connected to the port A1 or the port A2, or not to be electrically connected to any port. Similarly, the analog switch S2 may also include two ports: a port B1 and a port B2. The port B1 may be connected to the electrode 2, and the port B2 may be connected to the electrode 3. The analog switch S1 may receive an instruction sent by the electronic device 100 (or another electronic device), and in response to the instruction, choose to be electrically connected to the port B1 or the port B2, or not to be electrically connected to any port.

The excitation current generation unit AC may be configured to generate a constant current. The current is less than or equal to a human body safe current, and is harmless to a human body. When the excitation current generation unit AC is in a loop, the excitation current generation unit AC may output a current by using the positive electrode, and adjust a magnitude of the output current based on a current flowing into the negative electrode, so as to keep the magnitude of the current in the loop stable. Therefore, the excitation current generation unit AC may further determine, based on whether a current flows into the negative electrode, whether the excitation current generation unit AC is in a loop, so as to determine whether an electrode is in good contact with the skin.

When all electrodes in the monitoring circuit are in contact with the skin of the user, that is, when the electrode 1 is in contact with the contact point 1, the electrode 2 is in contact with the contact point 2, the electrode 3 is in contact with the contact point 3, and the electrode 4 is in contact with the contact point 4, the monitoring circuit may further include a contact resistance generated by each electrode. The contact resistances may include a contact resistance R1 corresponding to the electrode 1, a contact resistance R2 corresponding to the electrode 2, a contact resistance R3 corresponding to the electrode 3, and a contact resistance 4 corresponding to the electrode 4. In addition, the electrode 1 and the electrode 3, the electrode 2 and the electrode 4, the electrode 1 and the electrode 2, and the electrode 3 and the electrode 4 may be connected through the skin. A dermal resistance between the electrode 1 and the electrode 3 may be referred to as *R*_{skin 1}, and a dermal resistance between the electrode 2 and the electrode 4 may be referred to as *R*_{skin 2}. If the contact point 1 and the contact point 2 are respectively located at two ends of the thoracic tissue, the electrode 1 and the electrode 2 may be connected through the body of the user. A resistance between the electrode 1 and the electrode 2 may be considered as a body resistance *R*_{body} of the user. In this case, a resistance between the electrode 3 and the electrode 4 may also be considered as a body resistance *R*_{body} of the user.

In addition, when the contact point 1 and the contact point 2 are respectively located at two ends of the thoracic tissue, and both the electrode 3 and the electrode 4 are in contact with the skin, the electrode 3 may be connected to the electrode 4 through the body of the user. In other words, a resistance between the electrode 3 and the electrode 4 may also be considered as a body resistance *R*_{body}. Therefore, as shown in FIG. 5E, the electrode 3 may be connected to the electrode 4 through a dashed line 1, the body resistance *R*_{body}, and a dashed line 2. It should be noted that the dashed lines (the dashed line 1 and the dashed line 2) herein do not mean physical circuit connection lines, but are intended to assist in describing that the electrode 3 can be connected to the electrode 4 through the body of the user. That is, the body of the user may be equivalent to the dashed line 1, the body resistance *R*_{body}, and the dashed line 2 shown in FIG. 5E. Similarly, when the electrode 1 is connected to the electrode 2 through the body of the user, the body of the user may also be equivalent to a dashed line 3, the body resistance *R*_{body}, and a dashed line 4 shown in FIG. 5E. Similarly, when the electrode 1 is connected to the electrode 3 through local skin of the user, the skin between the electrode 1 and the electrode 3 may also be equivalent to a dashed line 5, *R*_{skin 1}, and a dashed line 6 shown in FIG. 5E. When the electrode 2 is connected to the electrode 4 through local skin of the user, the skin between the electrode 2 and the electrode 4 may also be equivalent to a dashed line 7, *R*_{skin 2}, and a dashed line 8 shown in FIG. 5E. It may be understood that, when a loop can be formed between two electrodes by using the body of the user or the skin of the user, dashed lines at two ends of the dermal resistance or another resistance may be considered as conductive; otherwise, the dashed lines may be considered as disconnected (that is, non-conductive).

When all electrodes in the monitoring circuit are in contact with the skin of the user, the analog switch S1 and the analog switch S2 may choose to be electrically connected to different ports, to form a loop among different electrodes.

FIG. 5F to FIG. 5H are diagrams of circuit connections when an analog switch is electrically connected to different ports in a case that all electrodes in a monitoring circuit are in contact with skin of a user.

For example, if all the electrodes in the monitoring circuit are in contact with the skin of the user, when the analog switch S1 is electrically connected to the port A1 and the analog switch S2 is electrically connected to the port B2, a loop may be formed between the electrode 1 and the electrode 3. Therefore, the dashed line 5 and the dashed line 6 shown in FIG. 5E may be considered as conductive. In this case, the monitoring circuit may form a loop 1 shown in FIG. 5F between the electrode 1 and the electrode 3.

As shown in FIG. 5F, the loop 1 may include the excitation current generation unit AC, the electrode 1, and the electrode 3. Because the electrode 1 and the electrode 3 are in good contact with the skin of the user, the electrode 1 and the electrode 3 may be connected by using the dermal resistance *R*_{skin 1}. The loop 1 may further include the contact resistance R1 of the electrode 1 and the contact resistance R3 of the electrode 3. That is, the excitation current generation unit AC, the contact resistance R1, the contact resistance R3, and the dermal resistance *R*_{skin 1} may be connected in series to form the loop 1. In the loop 1, the excitation current generation unit AC may generate a current. The excitation current generation unit AC may further determine a magnitude of a current in the loop 1, and adjust, in real time, a magnitude of the current generated by the excitation current generation unit AC.

When the analog switch S1 is electrically connected to the port A1 and the analog switch S2 is electrically connected to the port B2, if the excitation current generation unit AC determines that a current exists between the electrode 1 and the electrode 3 (or determines that a current between the electrode 1 and the electrode 3 is greater than or equal to a preset current threshold), it indicates that the loop 1 can be formed in the monitoring circuit, that is, both the electrode 1 and the electrode 3 are in good contact with the skin of the user; or if the excitation current generation unit AC determines that no current exists between the electrode 1 and the electrode 3 (or determines that a current between the electrode 1 and the electrode 3 is less than a preset current threshold), it indicates that the loop 1 cannot be formed in the monitoring circuit, that is, at least one of the electrode 1 and the electrode 3 is in poor contact with the skin of the user.

Therefore, the monitoring circuit may determine, by controlling the analog switch S1 to be electrically connected to the port A1 and the analog switch S2 to be electrically connected to the port B2, whether an electrode among the electrode 1 and the electrode 3 is in poor contact with the skin.

For another example, if all the electrodes in the monitoring circuit are in contact with the skin of the user, when the analog switch S1 is electrically connected to the port A2 and the analog switch S2 is electrically connected to the port B1, a loop may be formed between the electrode 2 and the electrode 4. Therefore, the dashed line 7 and the dashed line 8 shown in FIG. 5E may be considered as conductive. In this case, the monitoring circuit may form a loop 2 shown in FIG. 5F between the electrode 2 and the electrode 4.

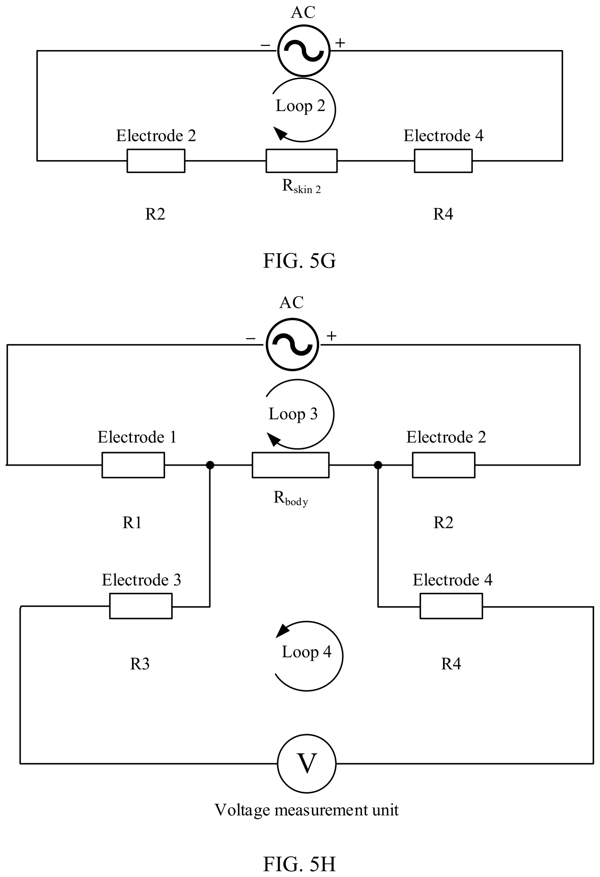

As shown in FIG. 5G, the loop 2 may include the excitation current generation unit AC, the electrode 2, and the electrode 4. Because the electrode 2 and the electrode 4 are in good contact with the skin of the user, the electrode 2 and the electrode 4 may be connected by using the dermal resistance *R*_{skin 2}. The loop 2 may further include the contact resistance R2 of the electrode 2 and the contact resistance R4 of the electrode 4. That is, the excitation current generation unit AC, the contact resistance R2, the contact resistance R4, and the dermal resistance *R*_{skin 2} may be connected in series to form the loop 2. In the loop 2, the excitation current generation unit AC may generate a current. The excitation current generation unit AC may further determine a magnitude of a current in the loop 2, and adjust, in real time, a magnitude of the current generated by the excitation current generation unit AC.

When the analog switch S1 is electrically connected to the port A2 and the analog switch S2 is electrically connected to the port B1, if the excitation current generation unit AC determines that a current exists between the electrode 2 and the electrode 4 (or determines that a current between the electrode 2 and the electrode 4 is greater than or equal to a preset current threshold), it indicates that the loop 2 can be formed in the monitoring circuit, that is, both the electrode 2 and the electrode 4 are in good contact with the skin of the user; or if the excitation current generation unit AC determines that no current exists between the electrode 2 and the electrode 4 (or determines that a current between the electrode 2 and the electrode 4 is less than a preset current threshold), it indicates that the loop 2 cannot be formed in the monitoring circuit, that is, at least one of the electrode 2 and the electrode 4 is in poor contact with the skin of the user.

Therefore, the monitoring circuit may determine, by controlling the analog switch S1 to be electrically connected to the port A2 and the analog switch S2 to be electrically connected to the port B1, whether an electrode among the electrode 2 and the electrode 4 is in poor contact with the skin.

For another example, if all the electrodes in the monitoring circuit are in contact with the skin of the user, and the contact point 1 and the contact point 2 are respectively located at two ends of the thoracic tissue, when the analog switch S1 is electrically connected to the port A1 and the analog switch S2 is electrically connected to the port B1 (or when the analog switch S1 is electrically connected to the port A2 and the analog switch S2 is electrically connected to the port B1), a loop may be formed between the electrode 1 and the electrode 2, and between the electrode 3 and the electrode 4. Therefore, the dashed line 1, the dashed line 2, the dashed line 3, and the dashed line shown in FIG. 5E may all be considered to be conductive. In this case, as shown in FIG. 5H, the monitoring circuit may form a loop 3 between the electrode 1 and the electrode 2, and form a loop 4 between the electrode 2 and the electrode 4.

As shown in FIG. 5H, the loop 3 between the electrode 1 and the electrode 2 may include the excitation current generation unit AC, the electrode 1, and the electrode 2, and the loop 4 between the electrode 3 and the electrode 4 may include the electrode 3, the electrode 4, and the voltage measurement unit V.

In the loop 3, because the electrode 1 and the electrode 2 are in good contact with the skin of the user, and a connection line between the contact point 1 and the contact point 2 can cross two ends of a heart of the user, the electrode 1 and the electrode 2 may be connected through the body resistance *R*_{body}. The loop 3 may further include the contact resistance R1 of the electrode 1 and the contact resistance R2 of the electrode 2. That is, the excitation current generation unit AC, the contact resistance R1, the contact resistance R2, and the body resistance *R*_{body} may be connected in series to form the loop 3. In the loop 3, the excitation current generation unit AC may generate a current. The excitation current generation unit AC may further determine a magnitude of a current in the loop 3, and adjust, in real time, a magnitude of the current generated by the excitation current generation unit AC, so that a magnitude of a current flowing through the body resistance *R*_{body} remains stable.

In the loop 4, because the electrode 3 and the electrode 4 are in good contact with the skin of the user, a distance between the electrode 1 and the electrode 3 is relatively short, and a distance between the electrode 2 and the electrode 4 is relatively short, the electrode 3 and the electrode 4 may also be connected through the body resistance *R*_{body}. The loop 4 may further include the contact resistance R3 of the electrode 3 and the contact resistance R4 of the electrode 4. That is, the contact resistance R3, the voltage measurement unit V, the contact resistance R4, and the body resistance *R*_{body} may be connected in series to form the loop 4. Because the body resistance *R*_{body} is also a component of the loop 3, a current generated by the excitation current generation unit AC flows through the body resistance *R*_{body}, and a voltage is formed between two ends of the body resistance *R*_{body}. In the loop 4, the voltage measurement unit V may measure a voltage between the electrode 3 and the electrode 4, that is, the voltage between the two ends of the body resistance *R*_{body}.

Therefore, when the analog switch S1 is electrically connected to the port A1 and the analog switch S2 is electrically connected to the port B1, the monitoring circuit 3 may determine, by using the excitation current generation unit AC, a current flowing through the body resistance *R*_{body}, and may further determine, by using the voltage measurement unit V, the voltage between the two ends of the body resistance *R*_{body}, to determine cardiac impedance of the user. For example, the cardiac impedance may be a ratio of the voltage between the two ends of the body resistance *R*_{body} to the current flowing through the body resistance *R*_{body}.

It may be understood that FIG. 5D to FIG. 5H are merely examples. In embodiments of this application, the monitoring circuit may include more or fewer circuit components than or circuit components different from those in the foregoing embodiments, or use a circuit structure different from that in the foregoing embodiments. This is not limited in this application.

The following describes a position relationship between contact points of the electrode 1 and the electrode 2 and the skin according to an embodiment of this application.

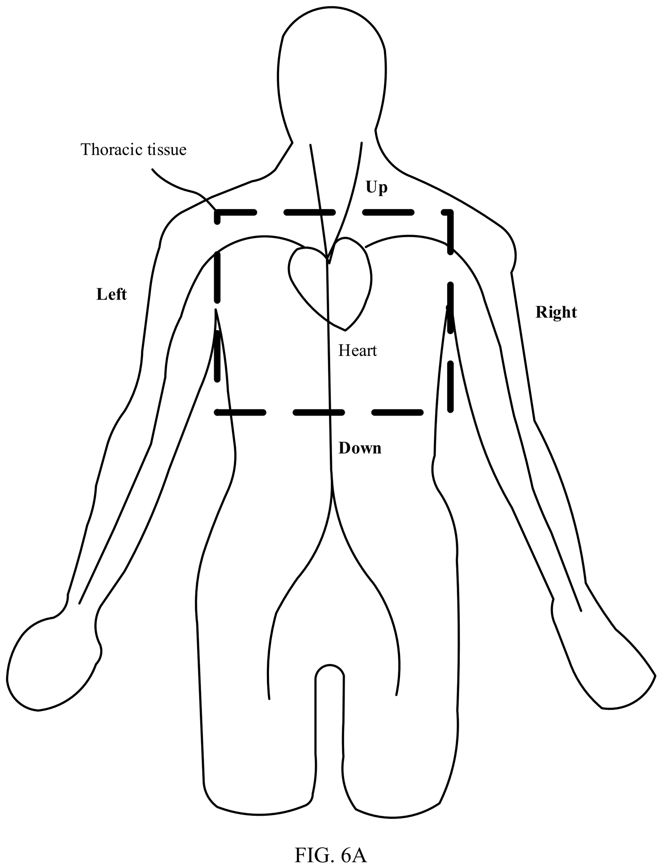

FIG. 6A is a diagram of a position relationship between a human body and thoracic tissue according to an embodiment of this application.

As shown in FIG. 6A, a heart may be located in the thoracic tissue, and cardiac impedance may be equivalent to impedance of the thoracic tissue, and may also be referred to as body impedance of a user. In the human body, an upper side of the thoracic tissue is a neck of the user, a lower side of the thoracic tissue is an abdomen of the user, a left side of the thoracic tissue is a left hand of the user, and a right side of the thoracic tissue is a right hand of the user. It should be noted that, in this embodiment of this application, the upper side, the lower side, the left side, and the right side of the thoracic tissue are determined based on a human blood flow direction, instead of an actual spatial position of a human body part. That is, the left hand of the user is always located on the left side of the thoracic tissue, the right hand of the user is always located on the right side of the thoracic tissue, a head of the user is always located above the thoracic tissue, and the abdomen of the user is always located below the thoracic tissue.

In this embodiment of this application, a contact point between an electrode 1 and skin may be referred to as a contact point 1, and a contact point between an electrode 2 and the skin may be referred to as a contact point 2. When the contact point 1 and the contact point 2 are respectively located at two ends (for example, upper and lower sides, or left and right sides) of the thoracic tissue, impedance between the electrode 1 and the electrode 2 may be considered as cardiac impedance. Therefore, in a process in which the electronic device 100 performs cardiac function monitoring, the electrode 1 and the electrode 2 need to meet the following monitoring condition: The electrode 1 and the electrode 2 are respectively located at two ends of the thoracic tissue.

The following describes position distribution of a plurality of contact points according to an embodiment of this application with reference to a specific scenario.

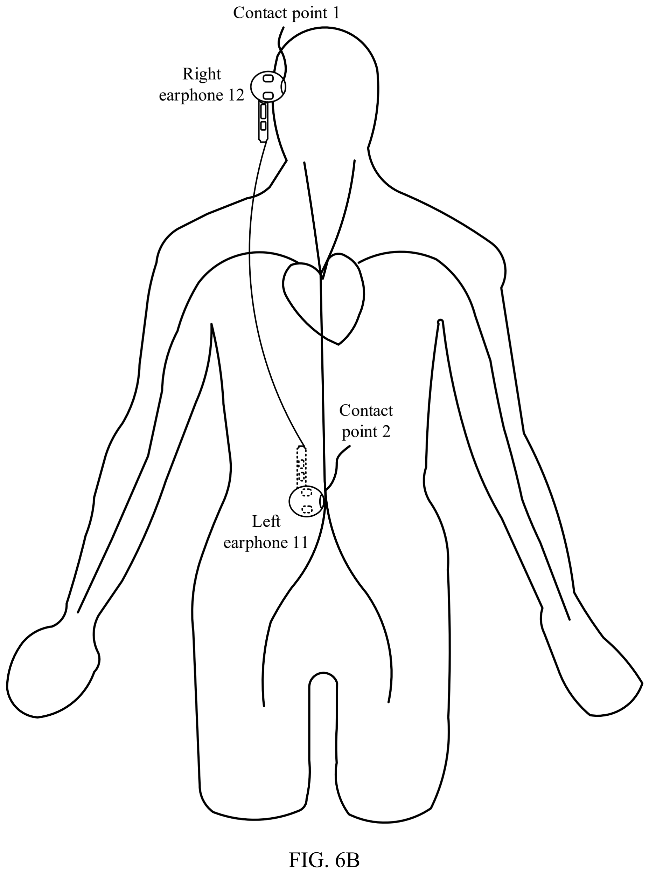

In some application scenarios, the electronic device 100 may be earphones 10. FIG. 6B is a diagram of distribution of contact points between electrodes on the earphones 10 and skin in a cardiac function monitoring process.

As shown in FIG. 6B, when the user uses the earphones 10 shown in FIG. 2A to perform cardiac function monitoring, the user may wear the right earphone 12 on the right ear, and stick the left earphone 11 to the abdomen, so that an electrode (for example, the electrode 1 and the electrode 3) on the left earphone 11 can be in contact with skin of the abdomen, and an electrode (for example, the electrode 2 and the electrode 4) on the right earphone 12 can be in contact with skin of the right ear. In this case, the contact point 1 between the electrode 1 on the left earphone 11 and the skin is located on the abdomen, and the contact point 2 between the electrode 2 on the right earphone 12 and the skin is located on the right ear. Because the abdomen is located below the thoracic tissue, and the right ear is located above the thoracic tissue, the contact point 1 and the contact point 2 are respectively located above and below the thoracic tissue, and meet the monitoring condition in the embodiment shown in FIG. 6A. In this case, impedance between the electrode 1 and the electrode 2 may be considered as the cardiac impedance, and the cardiac impedance of the user may be determined by measuring a voltage and a current between the electrode 1 and the electrode 2.

It may be understood that the embodiment shown in FIG. 6B is merely an example. In embodiments of this application, the user may alternatively measure a cardiac function by using the earphones 10 shown in FIG. 2B or FIG. 2C. In addition, the user may alternatively measure the cardiac function of the user in another measurement manner. For example, the left ear wears the left earphone 11, the electrode on the right earphone 12 is in contact with the skin of the abdomen, or the left hand is in contact with the electrode on the left earphone 11, and the right hand is in contact with the electrode on the right earphone 12. This is not limited in this application.

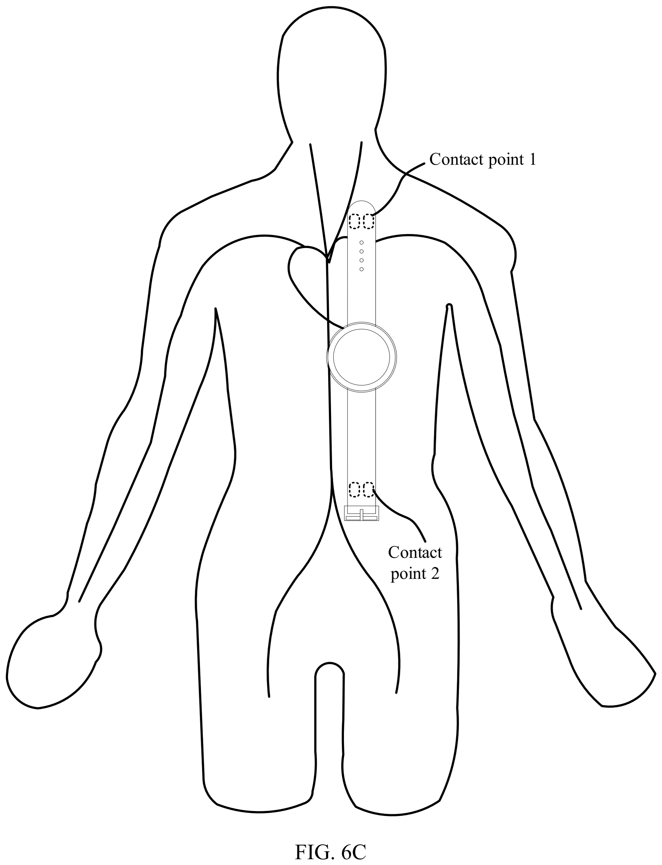

In some application scenarios, the electronic device 100 may be a watch 20. FIG. 6C is a diagram of distribution of contact points between electrodes on the watch 20 and skin in a cardiac function monitoring process.

As shown in FIG. 6C, when the user uses the watch 20 shown in FIG. 3A to measure the cardiac function, the user may separately make two ends of the watchband 22 be in contact with the chest of the user and the abdomen of the user, so that an electrode (for example, the electrode 1 and the electrode 3) at one end of the watchband 22 can be in contact with skin of the chest of the user, and an electrode (for example, the electrode 2 and the electrode 4) at the other end of the watchband 22 can be in contact with the skin of the abdomen of the user. In this case, the contact point 1 between the electrode 1 and the skin is located on the chest, and the contact point 2 between the electrode 2 and the skin is located on the abdomen. Because the abdomen is located below the thoracic tissue, and the chest belongs to an upper part the thoracic tissue, the contact point 1 and the contact point 2 are respectively located above and below the thoracic tissue, and meet the monitoring condition in the embodiment shown in FIG. 6A. In this case, impedance between the electrode 1 and the electrode 2 may be considered as the cardiac impedance, and the cardiac impedance of the user may be determined by measuring a voltage and a current between the electrode 1 and the electrode 2. It should be noted that, in the scenario shown in FIG. 6C, a length of the watchband 22 can span at least upper and lower ends (or left and right ends) of the thoracic tissue. In this way, it can be ensured that the electrode 1 and the electrode 2 can detect the cardiac impedance of the user. In addition, when it is ensured that a distance between the electrode 1 and the electrode 2 is greater than that between the two ends of the thoracic tissue, the distance between the electrode 1 and the electrode 2 should be as short as possible. In this way, it can be ensured that the contact point 1 and the contact point 2 are close enough to the heart, so as to obtain a more accurate measurement result. It may be understood that, compared with the watch 20 shown in FIG. 3A, a connection line between the left earphone 11 and the right earphone 12 in the foregoing embodiment is longer, and it is easier to ensure that the electrode 1 and the electrode 3 cross the two ends of the thoracic tissue.

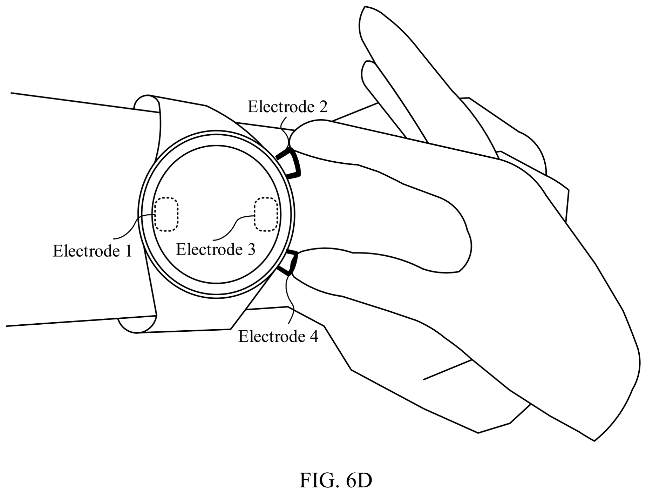

FIG. 6D is a diagram of distribution of contact points between electrodes on a watch 20 and skin in another cardiac function monitoring process.

As shown in FIG. 6D, when the user uses the watch 20 shown in FIG. 3B and FIG. 3C to measure the cardiac function, the user may wear the watch 20 on a wrist (for example, a left wrist), so that an electrode (for example, the electrode 1 and the electrode 3) on the rear surface of the movement 21 can be in contact with the wrist of the user. In addition, the user may touch an electrode (for example, the electrode 2 and the electrode 4) on the button with a finger of another hand (that is, the right hand). In this case, the contact point 1 between the electrode 1 and the skin is located on the left wrist of the user, and the contact point 2 between the electrode 2 and the user is located on the right-hand finger of the user. Because the left hand is located on the left side of the thoracic tissue, and the right hand is located on the right side of the thoracic tissue, the contact point 1 and the contact point 2 are respectively located on the left side and the right side of the thoracic tissue, and meet the monitoring condition in the embodiment shown in FIG. 6A. In this case, impedance between the electrode 1 and the electrode 2 may be considered as the cardiac impedance, and the cardiac impedance of the user may be determined by measuring a voltage and a current between the electrode 1 and the electrode 2.

It should be noted that the watch 20 in the embodiments shown in FIG. 3D and FIG. 3E may also measure the cardiac impedance of the user in a manner similar to that in the embodiment shown in FIG. 6D. In this case, the wrist of the user wearing the watch 20 may be in contact with one or more electrodes on the watchband 22, and the other hand of the user (that is, a hand that does not wear the watch 20) may be in contact with an electrode on the button. In this way, contact points between the electrode 1 and the electrode 2 and the skin of the user are respectively located on the left hand and the right hand of the user. Therefore, the contact point 1 and the contact point 2 are respectively located on the left side and the right side of the thoracic cavity tissue, and meet the monitoring condition in the embodiment shown in FIG. 6A. In this case, impedance between the electrode 1 and the electrode 2 may be considered as the cardiac impedance, and the cardiac impedance of the user may be determined by measuring a voltage and a current between the electrode 1 and the electrode 2.

It may be understood that the embodiments shown in FIG. 6C and FIG. 6D are merely two examples. In embodiments of this application, the user may alternatively measure the cardiac function by using a wearable device such as a band in the manner shown in the foregoing embodiment. A specific form of the wearable device is not limited herein in this application. In addition, the user may alternatively measure the cardiac function of the user in another measurement manner. For example, two hands are respectively in contact with electrodes at two ends of the watchband 22. This is not limited in this application.

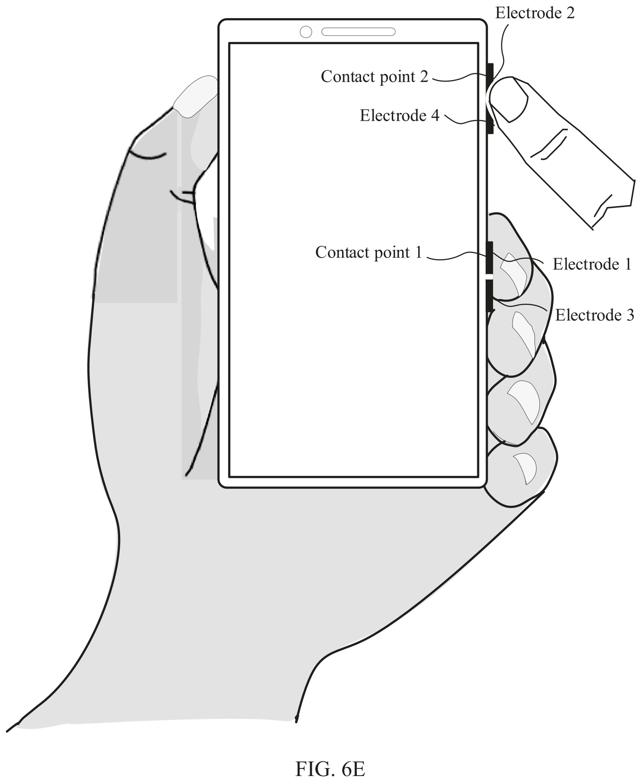

In some application scenarios, the electronic device 100 may be a mobile phone 30. FIG. 6E is a diagram of distribution of contact points between electrodes on the mobile phone 30 and skin in a cardiac function monitoring process.

For example, as shown in FIG. 6E, when the user uses the mobile phone 30 shown in FIG. 4A to measure the cardiac function, the user may hold the mobile phone 30 with the left hand, so that the left hand is in contact with one or more electrodes (for example, the electrode 1 and the electrode 3). In addition, the user may use a finger of the right hand to contact one or more other electrodes (for example, the electrode 2 and the electrode 4) of the mobile phone 30. In this case, the contact point 1 between the electrode 1 and the skin is located on the left hand, and the contact point between the electrode 2 and the skin is located on the right hand. Because the left hand is located on the left side of the thoracic tissue, and the right hand is located on the right side of the thoracic tissue, the contact point 1 and the contact point 2 are respectively located on the left side and the right side of the thoracic tissue, and meet the monitoring condition in the embodiment shown in FIG. 6A. In this case, impedance between the electrode 1 and the electrode 2 may be considered as the cardiac impedance, and the cardiac impedance of the user may be determined by measuring a voltage and a current between the electrode 1 and the electrode 2.

It may be understood that the embodiment shown in FIG. 6E is merely an example. In embodiments of this application, the user may alternatively measure the cardiac function by using an electronic device such as a tablet computer in the manner shown in the foregoing embodiment, or measure the cardiac function by using the mobile phone 30 shown in FIG. 4B. This is not limited in this application.

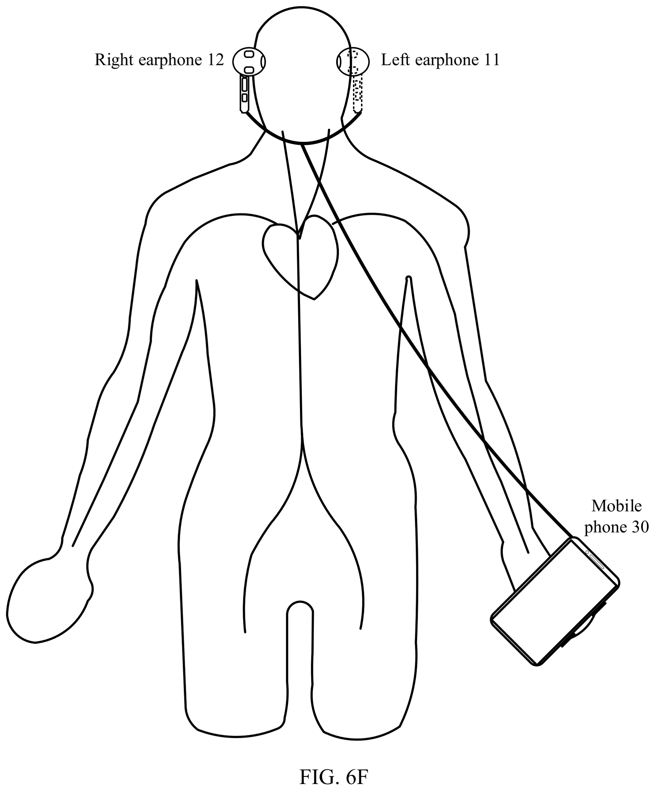

In some application scenarios, the electronic device 100 may be the earphones 10 shown in FIG. 2C, and the electronic device 200 may be a mobile phone (for example, the mobile phone 30 shown in FIG. 4A or FIG. 4B) on which an electrode is disposed. FIG. 6F is a diagram of distribution of contact points between electrodes in a health monitoring system 1000 and skin in a monitoring process.

As shown in FIG. 6F, the user may wear the earphones 10 shown in FIG. 2C, and the user may hold the mobile phone 30 with a single hand. The earphones 10 are wired earphones, and another port of a connection line of the earphones 10 is connected to the mobile phone 30. When the user wears the left earphone 11 and the right earphone 12, the user may hold the mobile phone 30 by using the left hand (or the right hand). In this case, the electrode on the right earphone 12 of the user may be in contact with the right ear, and the electrode on the mobile phone 30 may be in contact with the left hand of the user. Because the right ear is located on the right side of the thoracic tissue, and the left hand is located on the left side of the thoracic tissue, in this case, the electrode on the right earphone 12 and the electrode on the mobile phone 30 are respectively located on the left side and the right side of the thoracic tissue, and meet the monitoring condition in the embodiment shown in FIG. 6A. In this case, impedance between the electrode on the right earphone 12 and the electrode on the mobile phone 30 may be considered as the cardiac impedance, and the cardiac impedance of the user may be determined by measuring a voltage and a current between the two electrodes.

It should be noted that, in the application scenario shown in FIG. 6F, the electrode on the earphones 10, the electrode on the mobile phone 30, and a connection line between the earphones 10 and the mobile phone 30 may form the monitoring circuit shown in FIG. 5B or FIG. 5D. Another circuit component in the monitoring circuit may be located in the earphones 10 or the mobile phone 30. This is not limited in this application.

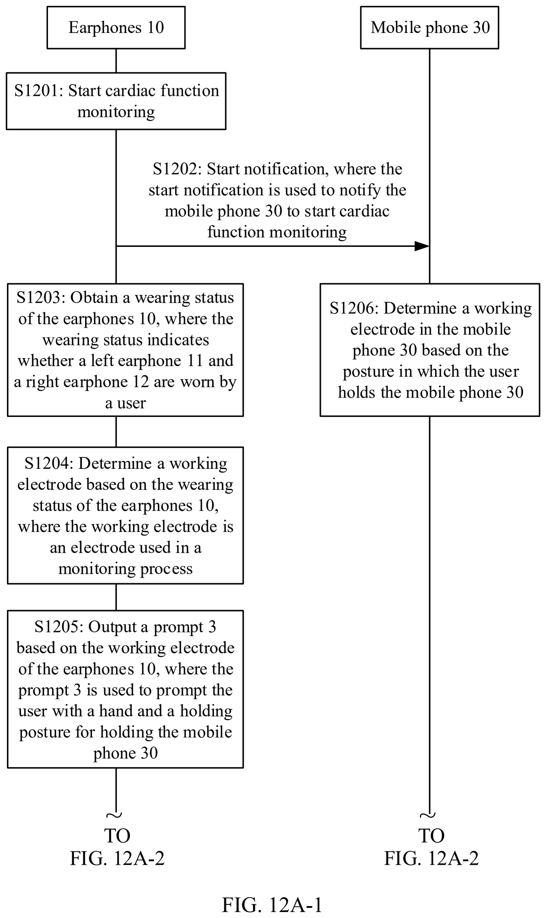

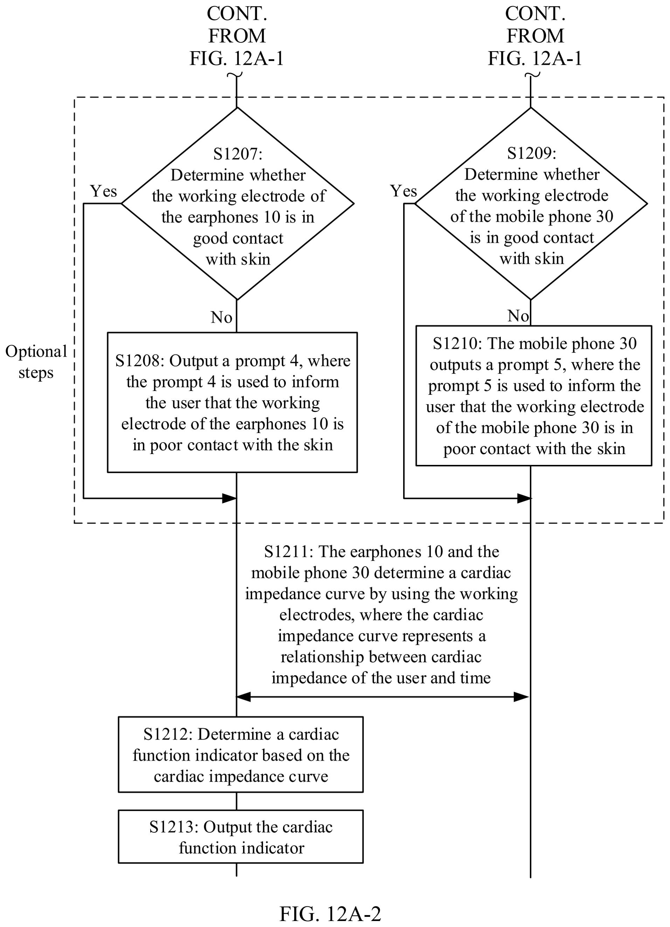

It may be understood that, the embodiment shown in FIG. 6F is merely an example to describe that the cardiac impedance of the user may be determined by using a plurality of devices. In embodiments of this application, the user may alternatively use a wearing manner and a holding manner that are different from those in the foregoing embodiment. For example, the user may wear the left earphone 11 and hold the mobile phone 30 with the right hand, and determine the cardiac impedance of the user based on the electrode on the left earphone 11 and the electrode on the mobile phone 30. This is not limited in this application. For a specific procedure in which the earphones 10 and the mobile phone 30 perform a monitoring method provided in an embodiment of this application, refer to related descriptions in the following embodiment shown inFIG. 12A-1 and FIG. 12A-2 . Details are not described herein.

The following describes a procedure of a monitoring method according to an embodiment of this application.

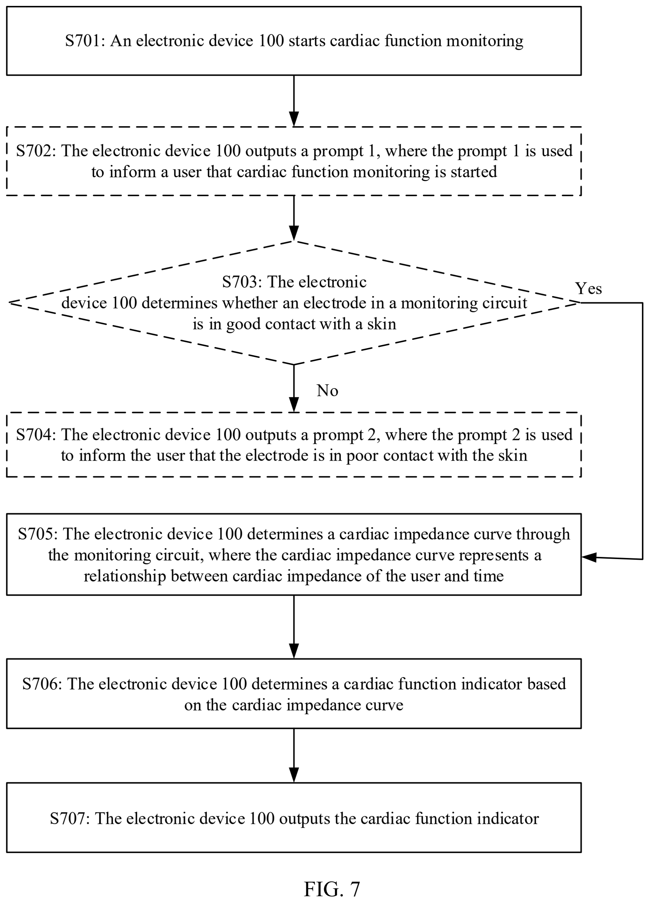

FIG. 7 is a schematic flowchart of performing a monitoring method according to an embodiment of this application.

As shown in FIG. 7, a specific procedure of performing the monitoring method by the electronic device 100 may include the following steps.

S701: The electronic device 100 starts cardiac function monitoring.

In some embodiments, the electronic device 100 may receive an operation 1 of a user on the electronic device 100, and start cardiac function monitoring in response to the operation. The operation 1 may be a preset operation. The operation 1 may vary with a device form of the electronic device 100. For example, if the electronic device 100 is earphones (for example, earphones 10), the operation 1 may be a double tap operation performed by the user on the earphones 10 (a left earphone 11 or a right earphone 12), or may be a press operation performed on a specified button on the earphones 10, or the like. If the electronic device 100 is a wearable device such as a watch 20, the operation 1 may be an operation performed by the user on a button on the watch 20, or may be an operation performed on a specified control (for example, a cardiac function measurement control in a health application) displayed on a display of the watch 20, or the like. If the electronic device 100 is a mobile phone 30, the operation 1 may alternatively be an operation performed on a specified control (for example, a cardiac function measurement control in a health application) displayed on a display 31 of the mobile phone 30, or the like.

In some other embodiments, the electronic device 100 may alternatively receive a start instruction sent by an electronic device 200, and start cardiac function monitoring in response to the start instruction. It should be noted that, in this case, the electronic device 100 may establish a communication connection to the electronic device 200. The communication connection may be a wired communication connection, or may be a wireless communication connection. For example, if the electronic device 100 is the earphones 10 in the embodiment shown in FIG. 2A or FIG. 2B, the communication connection between the electronic device 100 and the electronic device 200 may be a wireless communication connection such as a Bluetooth connection or a NearLink connection. If the electronic device 100 is the earphones 10 shown in FIG. 2C, the communication connection between the electronic device 100 and the electronic device 200 may be a wired connection. If the electronic device 100 is the mobile phone 30 in the embodiment shown in FIG. 4B, the communication connection between the electronic device 100 and the electronic device 200 may be a Bluetooth connection, a local area network connection, or the like. It may be understood that embodiments herein are merely some examples. In embodiments of this application, the communication connection between the electronic device 100 and the electronic device 200 may alternatively be a communication connection different from that in the foregoing embodiments. This is not limited in this application.

In some embodiments, after receiving the operation 1 of the user or receiving the start instruction sent by the electronic device 200, the electronic device 100 may output an operation prompt, where the operation prompt is used to inform the user of a position relationship between an electrode on the electronic device 100 and the user in a process of cardiac function monitoring. The electronic device 100 may inform the user of the position relationship between the electrode and the user in any one or more manners such as a text, a picture, an animation, a voice, indicator blinking, and vibration, so that the electronic device 100 can determine cardiac impedance of the user.

For example, if the electronic device 100 is the earphones 10 shown in FIG. 2A, the operation prompt may be a voice broadcast: "Wear one earphone, and stick the other earphone to the abdomen skin". If the electronic device 100 is the watch 20 shown in FIG. 3B and FIG. 3C, the operation prompt may be a text displayed on a display: "Wear the watch, and place the finger of the other hand on the electrode", or the like. It may be understood that the plurality of embodiments herein merely describe examples in which there may be a plurality of output manners of the operation prompt. In embodiments of this application, the output manner of the operation prompt may be an output manner different from that in the foregoing embodiment, and specific content of the operation prompt may be different from that in the foregoing embodiment. This is not limited in this application.

In some other embodiments, the electronic device 100 may alternatively start cardiac function monitoring when detecting that a monitoring condition is met. Optionally, when it is detected that the monitoring condition is met, an operation prompt may be further output, where the operation prompt is used to inform the user of a position relationship between an electrode on the electronic device 100 and the user in a process of cardiac function monitoring. The monitoring condition may include but is not limited to any one or more of the following: detecting that a physiological status of the user is abnormal (for example, a heart rate does not belong to a preset heart rate range), detecting that a psychological status of the user is abnormal (for example, frightened), detecting that the user is in an exercise state, detecting that the user has insomnia, detecting that a physical posture of the user is abnormal (for example, falling down), detecting that exercise equipment of the user is abnormal, detecting that a position of the user is in a preset region (for example, the user is in a high altitude region), or the like.

The following describes a specific manner in which the electronic device 100 determines whether the monitoring condition is met.

In some embodiments, the electronic device 100 may obtain user information, and determine, based on the user information, whether the electronic device 100 meets the monitoring condition. The user information may include but is not limited to any one or more of the following: physiological information, psychological information, exercise information, exercise equipment information, posture information, position information, interaction information, and the like. The physiological information may represent the physiological status of the user, and the physiological information may include but is not limited to any one or more of the following: a blood oxygen concentration, a heart rate, blood glucose, a body temperature, blood pressure, disease information, and the like. The psychological information may represent the psychological status of the user, and the psychological information may include but is not limited to any one or more of the following: a pressure value, being depressed, being calm, being excited, being scared, and the like. The exercise information may represent an exercise status of the user, and the exercise information may include but is not limited to any one or more of the following: swimming, diving, cycling, running, mountain climbing, rope skipping, yoga, and the like. The exercise equipment information may represent a status of exercise equipment, and the exercise equipment information may include but is not limited to any one or more of the following: an amount of remaining oxygen in an oxygen bottle, a weight of a smart backpack, a moving resistance of a bicycle, and the like. The posture information may represent a physical posture of the user, and the posture information may include but is not limited to any one or more of the following: falling down, missing a step, staying still, and the like. The position information may represent the position of the user, and the position information may include but is not limited to any one or more of the following: a geographical position of the user, longitude and latitude information of the position of the user, altitude information of the position of the user, depth information of the position of the user, and the like. The interaction information may include an interaction operation between the user and the electronic device 100 (or the electronic device 200), for example, receiving an operation of starting monitoring by the user. The interaction information may further include communication interaction between the electronic device 100 and the electronic device 200, for example, the start instruction sent by the electronic device 200 to the electronic device 100.

It should be noted that, in this embodiment of this application, a manner in which the electronic device 100 obtains the user information may include but is not limited to the following manners: The electronic device 100 detects the user information, the electronic device 100 receives the user information sent by the electronic device 200, or the electronic device 100 receives an operation of entering the user information (for example, disease information) by the user, and obtains the user information in response to the operation.

The following describes some manners of detecting the user information by the electronic device 100 according to an embodiment of this application.

For example, the electronic device 100 may detect the exercise information and the posture information of the user by using a component such as a gyro sensor (also referred to as a gyroscope) or an acceleration sensor. The electronic device 100 may detect the physiological information such as a heart rate, a blood oxygen concentration, and blood pressure of the user by using a component such as a photoplethysmography (photoplethysmography, PPG) module. The electronic device 100 may further collect a facial expression of the user by using a camera, and determine an emotion status of the user by using algorithm models such as image analysis and facial expression analysis. The electronic device 100 may also determine the psychological information such as a pressure value of the user based on the physiological information. The electronic device 100 may further detect the interaction information of the user by using a touch sensor. The electronic device 100 may detect the position information of the user by using a position sensor (for example, a global positioning chip). The electronic device 100 may further detect, based on a barometric pressure sensor, barometric pressure of an environment in which the user is located, and determine, based on the barometric pressure value, position information such as an altitude of the position of the user.

It may be understood that embodiments herein are merely some examples. In embodiments of this application, the electronic device 100 may include more or fewer embodiments than or components different from those in the foregoing embodiments. In addition, the electronic device 100 may alternatively collect the user information by using a sensor or another component different from that in the foregoing embodiments. This is not limited in this application.

The following describes some manners of determining, by the electronic device 100 based on the user information, whether the monitoring condition is met according to an embodiment of this application.

For another example, the electronic device 100 may determine whether the physiological information meets any one of the following: A heart rate does not belong to a preset heart rate range, blood pressure does not belong to a preset blood pressure range, a blood oxygen concentration does not belong to a preset blood oxygen concentration range, a body temperature does not belong to a preset body temperature range, and the like. If the physiological information meets any one of the foregoing, the electronic device 100 may determine that the physiological status of the user is abnormal, that is, determine that the monitoring condition is met.

For another example, the electronic device 100 may determine, based on the exercise information, the posture information, and a preset sleep period (for example, 23:00 to 06:00), whether the user has insomnia. Alternatively, the electronic device 100 may determine, based on interaction information of the user in the sleep period, whether the user has insomnia. If the user has insomnia, the electronic device 100 may determine that the monitoring condition is met.

For another example, the electronic device 100 may determine, based on the exercise information, whether the exercise status of the user meets any one of the following: a diving state, a mountain climbing state, a cycling state, a yoga state, a swimming state, a running state, and the like. If the exercise status meets any one of the foregoing, the electronic device 100 may determine that the monitoring condition is met.

For another example, the electronic device 100 may determine whether the psychological information meets any one of the following: The user is scared, the user is depressed, the user is excited, and the like. If the psychological information meets any one of the foregoing, the electronic device 100 may determine that the psychological status of the user is abnormal, that is, determine that the monitoring condition is met.

For another example, the electronic device 100 may determine, based on the position information, whether any one of the following is met: The user is in a high altitude region, the user is in a deep water region, and the like. If the position information meets any one of the foregoing, the electronic device 100 may determine that the position of the user is in the preset region, that is, determine that the monitoring condition is met.

For another example, the electronic device 100 may determine whether the posture information meets any one of the following: The user falls down, the user misses a step, and the like. If the posture information meets any one of the foregoing, the electronic device 100 may determine that the physical posture of the user is abnormal, that is, the monitoring condition is met.

For another example, the electronic device 100 may determine whether the exercise equipment information meets any one of the following: An amount of remaining oxygen in an oxygen bottle is less than a preset oxygen amount, a bicycle moving resistance is greater than a preset resistance, a weight of a smart backpack is greater than a preset weight, and the like. If the exercise equipment information meets any one of the foregoing, the electronic device 100 may determine that the exercise equipment of the user is abnormal, that is, the monitoring condition is met.

It may be understood that the foregoing embodiment merely describes examples of a plurality of manners of determining, based on the user information, whether the monitoring condition is met. In this embodiment of this application, the electronic device 100 may alternatively determine, based on a plurality of types of user information, whether the monitoring condition is met, or the electronic device 100 may determine, based on other information in the user information, whether the monitoring condition is met, and the monitoring condition may alternatively include more or fewer conditions than or conditions different from those in the foregoing embodiment. This is not limited in this application.

In some application scenarios, if the electronic device 100 is the earphones 10, and the earphones 10 plays audio before cardiac function monitoring is started, the earphones 10 may pause play of the audio after cardiac function monitoring is started.

S702: The electronic device 100 outputs a prompt 1, where the prompt 1 is used to inform the user that cardiac function monitoring is started.

Step S702 is an optional step.

The electronic device 100 may output the prompt 1 in any one or more manners such as displaying on the display, a voice, vibration, indicator blinking, and the like.

For example, if the electronic device 100 is the earphones 10, the earphones 10 may output the prompt 1 in a voice manner, for example, play a voice "Cardiac function monitoring has started, make ensure that the electrode is in good contact with the skin!". For another example, if the electronic device 100 is the watch 20, the watch 20 may output the prompt 1 in a display manner on the display. For example, a text "Cardiac function monitoring has started!" is displayed on the display. For another example, if the electronic device 100 is the mobile phone 30, the mobile phone 30 may output the prompt 1 in two manners: displaying on the display and vibration. For example, a text "Cardiac function monitoring has started!" is displayed, and a motor vibrates, to remind the user to view the prompt 1. It may be understood that the plurality of embodiments herein merely describe examples in which there may be a plurality of output manners of the prompt 1. In embodiments of this application, the output manner of the prompt 1 may be an output manner different from that in the foregoing embodiment, and specific content of the prompt 1 may be different from that in the foregoing embodiment. This is not limited in this application.

S703: The electronic device 100 determines whether an electrode in a monitoring circuit is in good contact with skin.

Step S703 and step S704 are optional steps.

In some embodiments, the monitoring circuit in the electronic device 100 may be the monitoring circuit shown in FIG. 5B. In this case, after cardiac function monitoring is started, the electronic device 100 may control the analog switch S0 in the monitoring circuit to be turned on, and determine whether a current exists in the current monitoring circuit. If it is determined that a current exists in the monitoring circuit, it is determined that the electrode is in good contact with the skin. In this case, the electronic device 100 may perform the following step S705. If it is determined that no current exists in the monitoring circuit, it is determined that the electrode is in poor contact with the skin. In this case, the electronic device 100 may perform the following step S704.

In some embodiments, the electronic device 100 may alternatively preset a current threshold, and determine, based on whether a magnitude of a current in the monitoring circuit reaches the current threshold, whether the electrode is in good contact with the skin. This is not limited in this application.

In some other embodiments, the monitoring circuit in the electronic device 100 may alternatively be the monitoring circuit shown in FIG. 5D and FIG. 5E. In this case, a specific procedure in which the electronic device 100 determines whether the electrode is in good contact with the skin may include the following steps.

1. The electronic device 100 controls the analog switch 1 to be electrically connected to the port A1.

2. The electronic device 100 controls the analog switch 2 to be electrically connected to the port B2.

After starting cardiac function monitoring, the electronic device 100 may deliver an instruction 1 to the analog switch 1, where the instruction 1 indicates the analog switch 1 to be electrically connected to the port A1. In addition, the electronic device 100 may also deliver an instruction 2 to the analog switch 2, where the instruction 2 indicates the analog switch 2 to be electrically connected to the port B2.

3. The electronic device 100 determines whether there is a current between the electrode 1 and the electrode 3.

When the analog switch 1 is electrically connected to the port A1 and the analog switch 2 is electrically connected to the port B2, if the electrode 1 and the electrode 3 are in good contact with the skin of the user, the monitoring circuit may form the loop shown in FIG. 5F between the electrode 1 and the electrode 3. When the electrode 1 and the electrode 3 form the loop, the electronic device 100 may determine that there is a current between the electrode 1 and the electrode 3. When no loop is formed between the electrode 1 and the electrode 3, there is no current between the electrode 1 and the electrode 3.

Therefore, the electronic device 100 may determine, based on whether there is a current between the electrode 1 and the electrode 3 (or based on whether a current between the electrode 1 and the electrode 3 is greater than a preset threshold), whether the electrode 1 and the electrode 3 are in good contact with the skin.

If the electronic device 100 determines that there is a current between the electrode 1 and the electrode 3, the electronic device 100 may perform the following step 5, to determine that both the electrode 1 and the electrode 3 are in good contact with the skin.

If the electronic device 100 determines that there is no current between the electrode 1 and the electrode 3, the electronic device 100 may perform the following step 4, to determine that at least one of the electrode 1 and the electrode 3 is in poor contact with the skin of the user.

4. At least one of the electrode 1 and the electrode 3 is in poor contact with the skin.

5. Both the electrode 1 and the electrode 3 are in good contact with the skin.

When it is determined that both the electrode 1 and the electrode 3 are in good contact with the skin, the electronic device 100 may perform the following step 6.

6. The electronic device 100 controls the analog switch 1 to be electrically connected to the port A2.

7. The electronic device 100 controls the analog switch 2 to be electrically connected to the port B1.

After determining that the electrode 1 and the electrode 3 are in good contact with the skin, the electronic device 100 may deliver an instruction 3 to the analog switch 1, where the instruction 3 indicates the analog switch 1 to be electrically connected to the port A2. In addition, the electronic device 100 may also deliver an instruction 4 to the analog switch 2, where the instruction 4 indicates the analog switch 2 to be electrically connected to the port B1.

8. The electronic device 100 determines whether there is a current between the electrode 2 and the electrode 4.

When the analog switch 1 is electrically connected to the port A2 and the analog switch 2 is electrically connected to the port B1, if the electrode 2 and the electrode 4 are in good contact with the skin of the user, the monitoring circuit may form the loop shown in FIG. 5G between the electrode 2 and the electrode 4. When the electrode 2 and the electrode 4 form the loop, the electronic device 100 may determine that there is a current between the electrode 2 and the electrode 4. When no loop is formed between the electrode 2 and the electrode 4, there is no current between the electrode 2 and the electrode 4.

Therefore, the electronic device 100 may determine, based on whether there is a current between the electrode 2 and the electrode 4 (or based on whether a current between the electrode 2 and the electrode 4 is greater than a preset threshold), whether the electrode 2 and the electrode 4 are in good contact with the skin.

If the electronic device 100 determines that there is a current between the electrode 2 and the electrode 4, the electronic device 100 may perform the following step 10, to determine that both the electrode 2 and the electrode 4 are in good contact with the skin.

If the electronic device 100 determines that there is no current between the electrode 2 and the electrode 4, the electronic device 100 may perform the following step 9, to determine that at least one of the electrode 2 and the electrode 4 is in poor contact with the skin of the user.

9. At least one of the electrode 2 and the electrode 4 is in poor contact with the skin.

10. Both the electrode 2 and the electrode 4 are in good contact with the skin.

It may be understood that the foregoing two embodiments merely describe examples of two manners of determining whether the electrode is in good contact with the skin. In this embodiment of this application, the monitoring circuit may alternatively use a circuit structure or a circuit component different from that in the foregoing embodiments, and a manner of determining whether the electrode is in good contact with the skin may be correspondingly changed based on a change of the monitoring circuit. This is not limited in this application.

When it is determined that the electrode is in poor contact, the electronic device 100 may perform the following step S704.

When it is determined that all electrodes are in good contact, the electronic device 100 may perform the following step S705.

S704: The electronic device 100 outputs a prompt 2, where the prompt 2 is used to inform the user that the electrode is in poor contact with the skin.

If the electronic device 100 determines that at least one electrode is in poor contact with the skin, the electronic device 100 may output the prompt 2, where the prompt 2 may be used to inform the user that the electrode in the monitoring circuit is in poor contact with the skin.

In some embodiments, the prompt 2 may further inform the user of a position relationship between the electronic device 100 and the electrode that is in poor contact with the skin. For example, if the electronic device 100 determines that an electrode among the electrode 1 and the electrode 3 is in poor contact, and the electronic device 100 is the earphones 10 shown in FIG. 2A, the prompt 2 may be "The left earphone is in poor contact with the skin, adjust the position of the left earphone". For another example, if the electronic device 100 determines that an electrode among the electrode 2 and the electrode 4 is in poor contact, and the electronic device 100 is the watch 20 shown in FIG. 3B and FIG. 3C, the prompt 2 may be "The finger is in poor contact with the electrode, adjust the position of the finger".

The electronic device 100 may alternatively output the prompt 2 in any one or more manners such as displaying on the display, a voice, vibration, indicator blinking, and the like.

S705: The electronic device 100 determines a cardiac impedance curve through the monitoring circuit, where the cardiac impedance curve represents a relationship between cardiac impedance of the user and time.

In some embodiments, after starting cardiac function monitoring, the electronic device 100 may determine the cardiac impedance curve of the user through the monitoring circuit.

In some other embodiments, the electronic device 100 may alternatively determine the cardiac impedance curve of the user through the monitoring circuit after performing step S703 and determining that all the electrodes in the monitoring circuit are in good contact with the skin.

For example, the monitoring circuit is the monitoring circuit in the embodiment shown in FIG. 5B. When all the electrodes in the monitoring circuit are in good contact with the skin, the electronic device 100 may control the analog switch S0 to be turned on; obtain a current curve by using the excitation current generation unit, where the current curve represents a relationship curve between a current flowing through a body of the user and time; obtain a voltage curve by using the voltage measurement unit, where the voltage curve represents a relationship curve between a voltage between two ends of the body of the user and time; and determine the cardiac impedance curve of the user based on the current curve and the voltage curve. The cardiac impedance of the user is equal to a ratio of the voltage between the two ends of the body of the user to the current flowing through the body of the user.

For example, the monitoring circuit is the monitoring circuit in the embodiments shown in FIG. 5D and FIG. 5E. When all the electrodes in the monitoring circuit are in good contact with the skin, the electronic device 100 may control the analog switch S1 to be electrically connected to the port A1, and control the analog switch S2 to be electrically connected to the port B1. In this case, the monitoring circuit may form a loop between the electrode 1 and the electrode 2, and measure a current flowing through the body of the user, to obtain a current curve. In addition, the monitoring circuit may also form a loop between the electrode 3 and the electrode 4, and measure a voltage between the two ends of the body of the user, to obtain a voltage curve. Then, the electronic device 100 may determine the current curve and the voltage curve based on the monitoring circuit, and determine the cardiac impedance curve of the user based on the current curve and the voltage curve.

It may be understood that the foregoing embodiments are merely some examples. In this embodiment of this application, the monitoring circuit may alternatively be a monitoring circuit different from that in the embodiments shown in FIG. 5B, FIG. 5D, and FIG. 5E. This is not limited in this application.

S706: The electronic device 100 determines a cardiac function indicator based on the cardiac impedance curve.

The cardiac impedance curve may be used to determine the cardiac function indicator, and the cardiac function indicator may include but is not limited to any one or more of the following: a cardiac output, a stroke volume, a heart rate, and an ejection fraction.

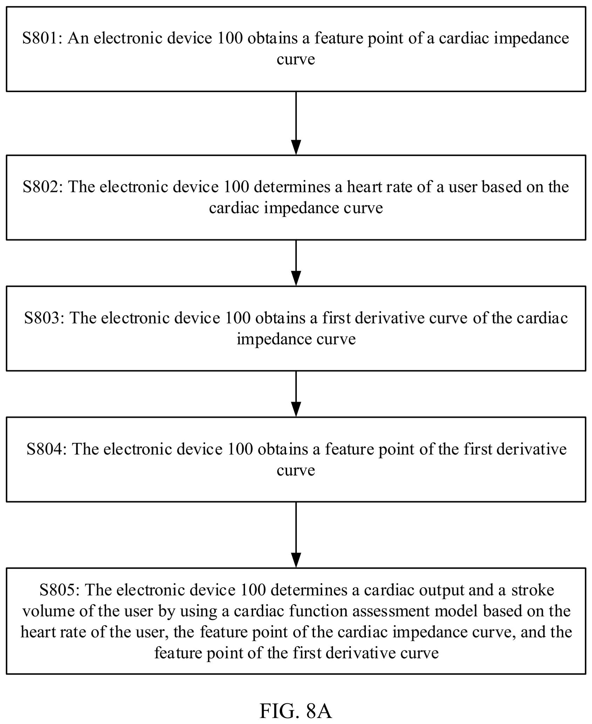

For a specific procedure in which the electronic device 100 determines the cardiac output and the stroke volume based on the cardiac impedance curve, refer to related descriptions in the following embodiment shown in FIG. 8A. Details are not described herein. In addition, because the ejection fraction is a ratio of the stroke volume to a total volume of cardiac blood, the ejection fraction may be obtained based on the stroke volume and the total volume of cardiac blood. In some embodiments, the total volume of cardiac blood may be obtained by using a built-in estimation algorithm of the electronic device 100. In some other embodiments, the total volume of cardiac blood may alternatively be a preset value, and the value may be obtained through estimation based on data of total volumes of cardiac blood of a plurality of testers by using an algorithm such as deep learning.

S707: The electronic device 100 outputs the cardiac function indicator.

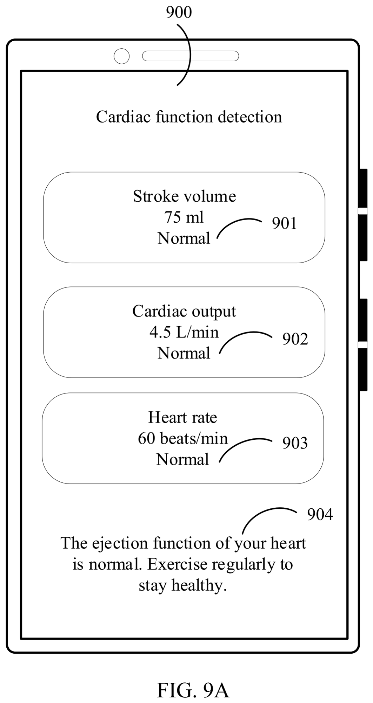

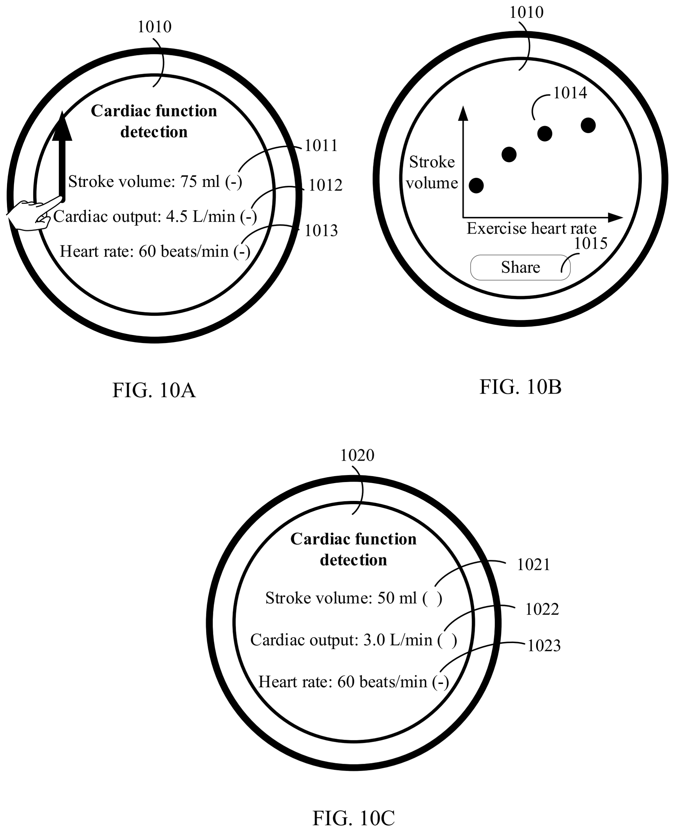

In some embodiments, the electronic device 100 may output the cardiac function indicator in one or more manners such as displaying on the display, a voice, vibration, and indicator blinking. For example, when the electronic device 100 outputs the cardiac function indicator in a display manner on the display, for an interface displayed by the electronic device 100, refer to related descriptions in the following embodiments shown in FIG. 9A to FIG. 9C and FIG. 10A to FIG. 10C.

In some other embodiments, that the electronic device 100 outputs the cardiac function indicator may also mean that the electronic device 100 sends an output instruction 1 to the electronic device 200, where the output instruction 1 may include the cardiac function indicator, and the output instruction 1 may indicate the electronic device 200 to output the cardiac function indicator.

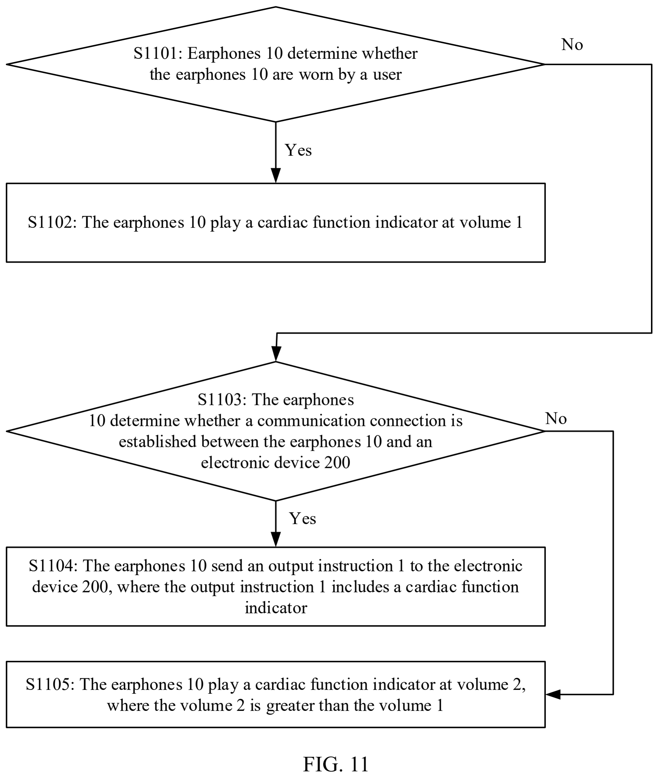

In some embodiments, if the electronic device 100 is the earphones 10, before outputting the cardiac function indicator, the electronic device 100 may determine an output manner of the cardiac function indicator based on factors such as whether the user wears the earphones and whether the electronic device 100 is connected to the electronic device 200. For a specific determining procedure, refer to related descriptions in the following embodiment shown in FIG. 11. Details are not described herein.

In some embodiments, the electronic device 100 may store value ranges of different cardiac function indicators. For example, Table 1 shows value ranges of cardiac function indicators stored in the electronic device 100 according to an embodiment of this application.

**Table 1**

| Cardiac function indicator | Value range |
|---|---|
| Cardiac output | 4.5-6 liters (L) |
| Stroke volume | 60-80 milliliters (mL) |
| Heart rate | 60-100 beats/minute (min) |

As shown in Table 1, the electronic device 100 may store value ranges of a plurality of cardiac function indicators. For example, a value range of the cardiac output may be 4.5-6 liters, a value range of the stroke volume may be 60-80 milliliters, and a value range of the heart rate may be 60-100 beats/minute.

It may be understood that the embodiment shown in Table 1 is merely an example. In this embodiment of this application, the cardiac function indicators may alternatively include more or fewer cardiac function indicators than or cardiac function indicators different from those in the foregoing embodiment, and value ranges of the cardiac function indicators may also be different from those in the foregoing embodiment. This is not limited in this application.

In some embodiments, after determining the cardiac function indicator, the electronic device 100 may determine a determining result of each cardiac function indicator based on a relationship between an actual value of the cardiac function indicator and a preset value range, for example, a determining result of the cardiac output and a determining result of the stroke volume. The determining result may include two types: normal and abnormal. Optionally, when the determining result is abnormal, the determining result may be further divided into a plurality of cases, for example, high, low, excessively high, and excessively low.

For example, if an actual value of the cardiac output is 7 liters, and the value range of the cardiac output stored in the electronic device 100 is 4.5-6 liters, the electronic device 100 may determine that the cardiac output of the user is high. If an actual value of the stroke volume is 80 milliliters, and the value range of the stroke volume stored in the electronic device 100 is 60-80 milliliters, the electronic device 100 may determine that the stroke volume of the user is normal. It may be understood that embodiments herein are merely two examples. In this embodiment of this application, an actual value of the cardiac function indicator and a preset value range may be different from those in the foregoing embodiment. This is not limited in this application.

After determining the determining result of each cardiac function indicator, the electronic device 100 may output the determining result of each cardiac function indicator while outputting the cardiac function indicator.

In some embodiments, the electronic device 100 may further determine a comprehensive determining result of the cardiac function of the user based on the relationship between the actual value of the cardiac function indicator and the preset value range. The comprehensive determining result indicates a cardiac function condition of the user, for example, whether the cardiac function of the user is normal. In some other embodiments, the electronic device 100 may further determine a comprehensive determining result of the cardiac function of the user based on the determining result of each cardiac function indicator. The comprehensive determining result may include normal and abnormal. Optionally, abnormal may also include but is not limited to any one or more of the following: subhealth, a disease risk, a high risk, and the like. For example, if the determining result of each cardiac function indicator of the user is normal, the comprehensive determining result may be normal; or if a determining result of at least one cardiac function indicator of the user is abnormal, the comprehensive determining result may be abnormal.

After determining the comprehensive determining result, the electronic device 100 may output the comprehensive determining result while outputting the cardiac function indicator.

In some embodiments, the electronic device 100 may store value ranges of the cardiac function indicator in different states. The status of the user may include a resting state and an exercise state, and optionally, may further include but is not limited to any one or more of the following: a sleep state, a high altitude state, a scared state, an oxygen-deficient state, a diving state, a meditative state, and the like. The electronic device 100 may determine the status of the user based on the user information. For specific content and an obtaining manner of the user information, refer to related descriptions in step S701. Details are not described herein again.

For example, Table 2 shows value ranges of cardiac function indicators in different states that are stored in the electronic device 100 according to an embodiment of this application.

**Table 2**

| Cardiac function indicator | Value range in a resting state | Value range in an exercise state |
|---|---|---|
| Cardiac output | 4.5-6 liters (L) | 9-18 liters |
| Stroke volume | 60-80 milliliters (mL) | 90-120 milliliters |
| Heart rate | 60-100 beats/minute (min) | 100-150 beats/minute |

As shown in Table 2, the electronic device 100 may store value ranges of a plurality of cardiac function indicators in different states. For example, in the resting state, a value range of the cardiac output may be 4.5-6 liters, a value range of the stroke volume may be 60-80 milliliters, and a value range of the heart rate may be 60-100 beats/minute. In the exercise state, a value range of the cardiac output may be 9-18 liters, a value range of the stroke volume may be 90-120 milliliters, and a value range of the heart rate may be 100-150 beats/minute.

It may be understood that the embodiment shown in Table 2 is merely an example. In this embodiment of this application, the electronic device 100 may alternatively store more or fewer user statuses and cardiac function indicators than or user statuses and cardiac function indicators different from those in the foregoing embodiment, and value ranges of the cardiac function indicators may also be different from those in the foregoing embodiment. This is not limited in this application.

In this case, after starting cardiac function monitoring (that is, after step S701), the electronic device 100 may determine the status of the user. For example, whether the user is in the exercise state is determined based on a component such as an accelerometer or a gyroscope, or whether the user is in the sleep state is determined based on a sleep monitoring module, or the current status of the user is determined based on a status setting operation of the user. A specific manner of determining the status of the user by the electronic device 100 is not limited in this application.

After determining the current user status and determining the cardiac function indicator of the user, the electronic device 100 may determine a determining result of each cardiac function indicator and/or a comprehensive determining result based on a relationship between an actual value of the cardiac function indicator and a value range of the cardiac function indicator in the current user status. Then, the electronic device 100 may output the determining result of each cardiac function indicator and/or the comprehensive determining result while outputting the cardiac function indicator.

In some other embodiments, the electronic device 100 may further output a status prompt while outputting the cardiac function indicator. The status prompt is used to inform the user of the current status and a health suggestion in the current status. The user status may be determined based on the user information. For example, if the electronic device 100 determines, based on the position information of the user, that the user is in a high altitude state, the status prompt may be "You are in a high altitude region currently, and it is recommended that you reduce exercise". For another example, if the electronic device 100 determines, based on the physiological information of the user, that the user is in a subhealth state, the status prompt may be "You are in a poor physical condition currently, and it is recommended that you avoid high intensity exercise". It may be understood that embodiments herein are merely some examples. In this embodiment of this application, the user status may further include more states than or states different from those in the foregoing embodiment, or the status prompt may include more or fewer status prompts than or status prompts different from those in the foregoing embodiment. This is not limited in this application.

According to the monitoring method provided in this embodiment of this application, the cardiac function of the user can be monitored at any time in daily life of the user, and cardiac function indicators such as the cardiac output and the stroke volume of the user can be obtained in real time.

In some embodiments, the electronic device 100 may alternatively send the cardiac impedance curve to the electronic device 200, and the electronic device 200 determines the cardiac function indicator based on the cardiac impedance curve. After determining the cardiac function indicator, the electronic device 200 may output the cardiac function indicator. In some other embodiments, after determining the cardiac function indicator, the electronic device 200 may alternatively send an output instruction 2 to the electronic device 100. The output instruction 2 may include the cardiac function indicator, and the output instruction 2 may indicate the electronic device 100 to output the cardiac function indicator. Optionally, the electronic device 200 may further determine a determining result based on the cardiac impedance curve. For specific content of the determining result, refer to related descriptions in step S707 shown in FIG. 7. In this case, the electronic device 200 may also output the determining result, or the output instruction 2 may also include the determining result.

In this way, when a computing capability of the electronic device 100 is limited, the electronic device 100 may determine the cardiac impedance curve, and calculate the cardiac function indicator by using the electronic device 200.

It may be understood that the embodiment herein is merely an example for description. Some steps in the method procedure shown in FIG. 7 may be performed by the electronic device 200. In this embodiment of this application, the electronic device 200 may alternatively perform more or fewer steps than or steps different from those in the foregoing embodiment. This is not limited in this application.

In some embodiments, the electronic device 100 may alternatively determine a current curve and a voltage curve, and send the current curve and the voltage curve to the electronic device 200. The electronic device 200 determines a cardiac impedance curve based on the current curve and the voltage curve, and determines a cardiac function indicator based on the cardiac impedance curve. The current curve represents a relationship between a current flowing through the heart of the user and time, and the voltage curve represents a relationship between a voltage between two ends of the heart of the user and time.

The following describes a specific procedure of determining a cardiac function indicator based on a cardiac impedance curve according to an embodiment of this application.

FIG. 8A is a schematic flowchart of determining a cardiac function indicator based on a cardiac impedance curve according to an embodiment of this application.

As shown in FIG. 8A, a specific procedure in which an electronic device 100 determines a cardiac function indicator based on a cardiac impedance curve may include the following steps.

S801: The electronic device 100 obtains a feature point of the cardiac impedance curve.

The feature point of the cardiac impedance curve may include a peak point and a trough point. The peak point is a local highest point of the curve, and the trough point is a local lowest point of the curve. The peak point and the trough point of the cardiac impedance curve can represent a systolic period, a diastolic period, and a pumping moment of a heart. When the heart undergoes periodic systolic and diastolic activities, blood flowing in thoracic tissue changes periodically with systole and diastole. This leads to a periodic change of impedance of the thoracic tissue. During systole, blood is ejected into an aorta, so that an aortic lumen expands, a cross-sectional area of the aortic lumen increases, and a blood volume of the aortic lumen increases. Because the blood is a good conductor, a resistance of the thoracic tissue decreases, and cardiac impedance decreases. During diastole, the blood returns to the heart, the aortic lumen retracts, the cross-sectional area of the aortic lumen decreases, the blood volume decreases, the resistance of the thoracic tissue increases, and the cardiac impedance increases. Therefore, the cardiac impedance decreases during systole, and the cardiac impedance increases during diastole.

It can be learned from the foregoing analysis that, from a peak point of the cardiac impedance curve to a next trough point, the cardiac impedance of the user gradually decreases, and the heart completes one systole; and from a trough point of the cardiac impedance curve to a next peak point, the cardiac impedance of the user gradually increases, and the heart completes one diastole. Therefore, the electronic device 100 may determine the systolic period, the diastolic period, and the cardiac pumping moment of the heart of the user based on a moment corresponding to the peak point and a moment corresponding to the trough point in the cardiac impedance curve. The cardiac pumping moment is a moment at which the diastolic period ends and systole starts. In some embodiments, parameters such as the systolic period, the diastolic period, and the cardiac pumping moment of the heart may be used to calculate a heart rate of the user, or may be used to calculate a cardiac output and a stroke volume.

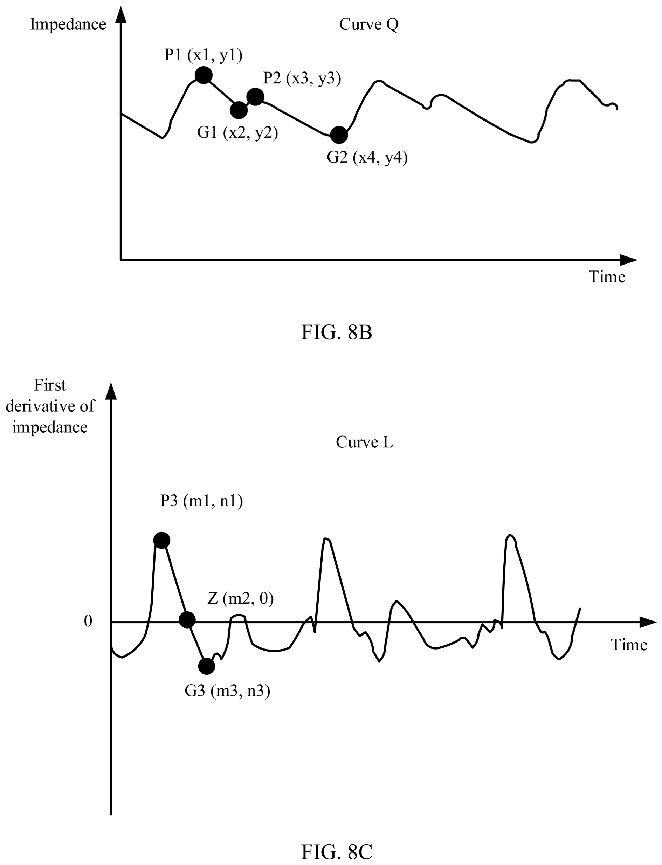

For example, FIG. 8B is a diagram of a cardiac impedance curve in a two-dimensional coordinate system according to an embodiment of this application.

As shown in FIG. 8B, the two-dimensional coordinate system may include a horizontal coordinate axis and a vertical coordinate axis, where the horizontal coordinate axis may represent time, and the vertical coordinate axis may represent a value of the cardiac impedance. The cardiac impedance curve may be a curve Q in the two-dimensional coordinate system. The curve Q may include a plurality of peak points, for example, a peak point P1 and a peak point P2, and the curve Q may further include a plurality of trough points, for example, a trough point G1 and a trough point G2. Two adjacent peak points of the trough point G1 are the peak point P1 and the peak point P2, and two adjacent trough points of the peak point P2 are the trough point G1 and the trough point G2. In addition, coordinates of the plurality of feature points in the two-dimensional coordinate system are sequentially the peak point P1 (x1, y1), the trough point G1 (x2, y2), the peak point P2 (x3, y3), and the trough point G2 (x4, y4), and x1<x2<x3<x4.

When the cardiac impedance curve is the curve Q shown in FIG. 8B, the systolic period of the heart of the user may be a time difference (x2-x1) between the peak point P1 and the trough point G1, or may be a time difference (x4-x3) between the peak point P2 and the trough point G2, or may be an average value of the two time differences, that is, (x2-x1+x4-x3)/2. The diastolic period of the heart of the user may be a time difference between the trough point G1 and the peak point P2, that is, (x3-x2). Moments corresponding to the peak point P1 and the peak point P2 may be considered as pumping moments of the heart.

It may be understood that the embodiment shown in FIG. 8B merely describes an example of how to determine the systolic period, the diastolic period, and the cardiac pumping moment of cardiac pumping based on the feature point of the cardiac impedance curve. In this embodiment of this application, the cardiac impedance curve may alternatively be a cardiac impedance curve different from that in the foregoing embodiment, and the electronic device 100 may determine the systolic period, the diastolic period, and the cardiac pumping moment of the heart based on more or fewer feature points than or feature points different from those in the foregoing embodiment. This is not limited in this application.

S802: The electronic device 100 determines a heart rate of the user based on the cardiac impedance curve.

Each heartbeat can complete one systole and one diastole. In some embodiments, the electronic device 100 may determine the heart rate of the user based on a sampling time length of the cardiac impedance curve and a quantity of peak points and a quantity of trough points in a sampling interval.

For example, the cardiac impedance curve is the curve Q shown in FIG. 8B. If the sampling time interval is [x1, x3], in the sampling interval, the curve Q includes two peak points P1 and P2, and one trough point G1. In this case, time required for one heartbeat of the user may be (x3-x1). Then, the electronic device 100 may calculate a quantity of heartbeats per minute based on the time required for the single heartbeat.

In some embodiments, the electronic device 100 may alternatively determine, based on the diastolic period and the systolic period of the heart, time required for a single heartbeat, and calculate a quantity of heartbeats per minute, to obtain the heart rate of the user.

In some other embodiments, the electronic device 100 may alternatively determine the heart rate of the user based on a time length of the sampling interval and a quantity of cardiac pumping times in the sampling interval.

S803: The electronic device 100 obtains a first derivative curve of the cardiac impedance curve.

The electronic device 100 may obtain the first derivative curve of the cardiac impedance curve (referred to as the first derivative curve below) by calculating a derivative of the cardiac impedance curve.

S804: The electronic device 100 obtains a feature point of the first derivative curve.

The feature point of the first derivative curve may include a peak point and a trough point, and may further include a point whose value is zero in a first derivative (also referred to as a zero point). The feature point of the first derivative curve can represent a change rate and a change trend of the cardiac impedance.

For example, FIG. 8C is a diagram of a first derivative curve of a cardiac impedance curve in a two-dimensional coordinate system according to an embodiment of this application.

As shown in FIG. 8C, the two-dimensional coordinate system may include a horizontal coordinate axis and a vertical coordinate axis, where the horizontal coordinate axis may represent time, and the vertical coordinate axis may represent a first derivative of the cardiac impedance. The first derivative curve may be a curve L in the two-dimensional coordinate system. The curve L may include a plurality of feature points, for example, a peak point P3, a zero point Z, and a trough point G3. Coordinates of the feature points are respectively the peak point P3 (m1, n1), the zero point Z (m2, 0), and the trough point G3 (m3, n3), m1<m2<m3, and n1>0>n2.

It can be learned from a characteristic of the first derivative that, in a time period [m1, m2] corresponding to the peak point P3 to the zero point Z, the cardiac impedance gradually increases and an increasing speed gradually decreases; and in a time period [m2, m3] corresponding to the zero point Z to the trough point G3, the cardiac impedance gradually decreases and a decreasing speed gradually increases.

S805: The electronic device 100 determines a cardiac output and a stroke volume of the user by using a cardiac function assessment model based on the heart rate of the user, the feature point of the cardiac impedance curve, and the feature point of the first derivative curve.

The electronic device 100 may store one or more cardiac function assessment models, and the cardiac function assessment models may be obtained through training by using an algorithm model such as XGBoost or a CNN convolutional neural network. The electronic device 100 may input the heart rate, the feature point of the cardiac impedance curve, and the feature point of the first derivative curve to the cardiac function assessment model. The cardiac function assessment model may determine the cardiac output and the stroke volume based on the foregoing input. In some other embodiments, the electronic device 100 may alternatively use parameters such as the diastolic period, the systolic period, and the pumping moment of the heart as an input of the cardiac function assessment model, so that the cardiac function assessment model calculates the cardiac output and the stroke volume.

For example, the cardiac function assessment model may obtain the cardiac output and the stroke volume through calculation based on the heart rate, the feature point of the cardiac impedance curve, the feature point of the first derivative curve, and the like by using a Kubicek formula.

It may be understood that the embodiment shown in FIG. 8A is merely an example. In this embodiment of this application, the electronic device 100 may alternatively determine the cardiac output and the stroke volume based on the cardiac impedance curve in a manner different from that in the foregoing embodiment. This is not limited in this application.

In some embodiments, the electronic device 100 may alternatively send the cardiac impedance curve to an electronic device 200, and the electronic device 200 determines a cardiac function indicator of the user by using a procedure that is the same as or similar to that shown in FIG. 8A. After determining the cardiac function indicator, the electronic device 200 may output the cardiac function indicator, or may send the cardiac function indicator to the electronic device 100.

The following describes diagrams of two groups of interfaces of outputting cardiac function indicators according to an embodiment of this application.

In some application scenarios, the electronic device 100 (or the electronic device 200) configured to output the cardiac function indicator may be an electronic device such as a mobile phone, a tablet computer, or a computer.

For example, if the electronic device 100 is the mobile phone 30 in the embodiment shown in FIG. 4A, after determining the cardiac function indicator, the electronic device 100 may display an output interface 900 shown in FIG. 9A.

As shown in FIG. 9A, the output interface 900 may include a plurality of cardiac function indicators. For example, a stroke volume is 75 ml, a cardiac output is 4.5 L/min, and a heart rate is 60 beats/min. Optionally, the electronic device 100 may further display a determining result of the cardiac function indicator near the cardiac function indicator, for example, a determining result 901, a determining result 902, and a determining result 903. The determining result 901 is displayed near the stroke volume, and indicates whether the stroke volume is normal. The determining result 902 is displayed near the cardiac output, and indicates whether the cardiac output is normal. The determining result 903 is displayed near the heart rate, and indicates whether the heart rate is normal. In the embodiment shown in FIG. 9A, determining results of the cardiac output, the stroke volume, and the heart rate of the user are all normal. Optionally, the output interface 900 may further display an assessment result 904, and the assessment result 904 may be a text "The ejection function of your heart is normal. Exercise regularly to stay healthy".

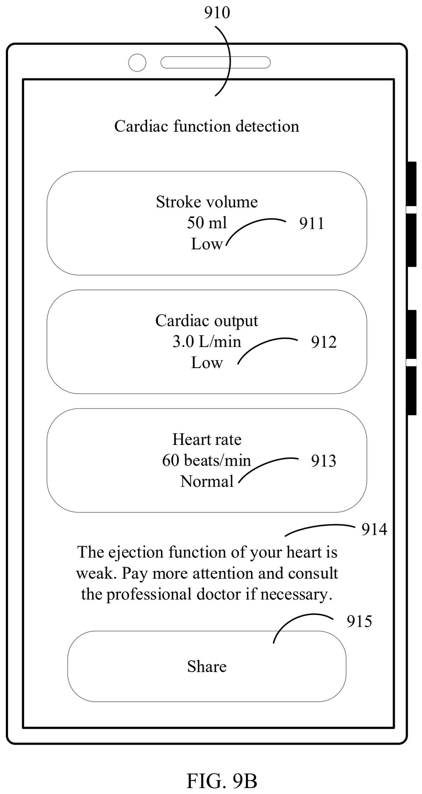

For another example, if the electronic device 100 is the mobile phone 30, after determining the cardiac function indicator, the electronic device 100 may display an output interface 910 shown in FIG. 9B.

As shown in FIG. 9B, the output interface 900 may include a plurality of cardiac function indicators. For example, a stroke volume is 50 ml, a cardiac output is 3.0 L/min, and a heart rate is 60 beats/min. Optionally, the electronic device 100 may further display a determining result of the cardiac function indicator near the cardiac function indicator, for example, a determining result 911, a determining result 912, and a determining result 913. Each determining result may indicate whether a corresponding cardiac function indicator is normal. In the embodiment shown in FIG. 9B, both the cardiac output and the stroke volume of the user are low, and are abnormal, and the determining result of the heart rate is normal. Optionally, the output interface 900 may further display an assessment result 914, and the assessment result 914 may be a text "The ejection function of your heart is weak. Pay more attention and consult the professional doctor if necessary". Further, optionally, the output interface 910 may further display a data sharing control 915, and the data sharing control 915 may be configured to trigger the electronic device 100 to send the cardiac function indicator, the determining result of the cardiac function indicator, and the like to another electronic device (for example, an electronic device selected by the user from an address book of the electronic device 100, or an electronic device preset by the user).

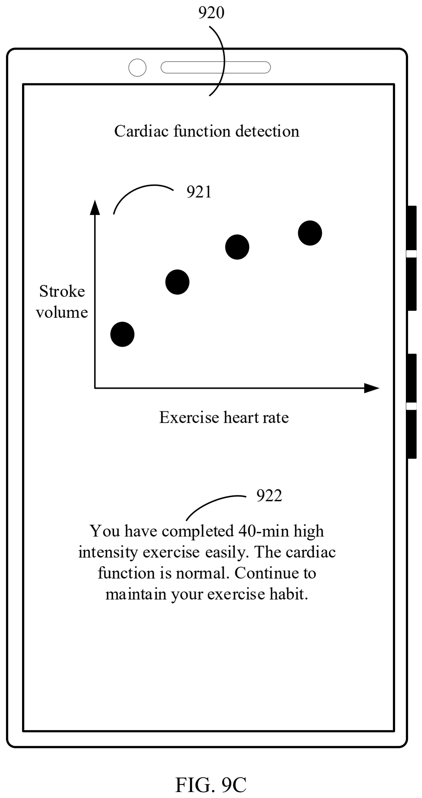

For another example, if the electronic device 100 is the mobile phone 30, and the user is in an exercise state in a process of performing cardiac function monitoring by the electronic device 100, after determining the cardiac function indicator, the electronic device 100 may display an output interface 920 shown in FIG. 9C.

As shown in FIG. 9C, the output interface 920 may include a diagram 921 of a cardiac function indicator and an assessment result 922. The diagram 921 of the cardiac function indicator may represent a relationship between a stroke volume (or a cardiac output) of the user and an exercise heart rate. The assessment result 922 may indicate whether a cardiac function of the user is normal. Optionally, the assessment result 922 may further indicate an exercise intensity, exercise time, and the like that are supported by the heart of the user. For example, the assessment result 922 may be a text "You have completed 40-min high intensity exercise easily. The cardiac function is normal. Continue to maintain your exercise habit".

In some other embodiments, if the electronic device 100 is the mobile phone 30, and the user is in an exercise state in a process of performing cardiac function monitoring by the electronic device 100, the electronic device 100 may first display the output interface 900 shown in FIG. 9A. Then, the electronic device 100 may receive a slide-up operation performed by the user on the output interface 900, and display, in the output interface 900 in response to the operation, content in the output interface 920 shown in FIG. 9C.

It may be understood that the embodiments shown in FIG. 9A to FIG. 9C are merely three examples. In embodiments of this application, an output interface for outputting the cardiac function indicator by the electronic device 100 may further include more or less content than or content different from that in the foregoing embodiments. This is not limited in this application.

In some application scenarios, the electronic device 100 (or the electronic device 200) configured to output the cardiac function indicator may be a wearable device such as a watch or a band.

For example, if the electronic device 100 is the watch 20, after determining the cardiac function indicator, the electronic device 100 may display an output interface 1010 shown in FIG. 10A.

As shown in FIG. 10A, the output interface 1010 may include a plurality of cardiac function indicators. For example, a stroke volume is 75 ml, a cardiac output is 4.5 L/min, and a heart rate is 60 beats/min. Optionally, the electronic device 100 may further display a determining result of the cardiac function indicator near the cardiac function indicator, for example, a determining result 1011, a determining result 1012, and a determining result 1013. Each determining result may indicate whether a corresponding cardiac function indicator is normal. The determining result may be represented by using a transverse line, an upward arrow, and a downward arrow. When the determining result is a transverse line, it indicates that the cardiac function indicator is normal; when the determining result is an upward arrow, it indicates that the cardiac function indicator is high; or when the determining result is a downward arrow, it indicates that the cardiac function indicator is low. In the embodiment shown in FIG. 10A, determining results of a cardiac output, a stroke volume, and a heart rate of the user are all transverse lines, indicating that the determining results of the three cardiac function indicators are all normal.

In some embodiments, if the user is in an exercise state in a process of performing cardiac function monitoring by the electronic device 100, the electronic device 100 may receive a slide-up operation performed by the user on the output interface 1010 shown in FIG. 10A, and display, in the output interface 1010 in response to the operation, a diagram 1014 of a cardiac function indicator shown in FIG. 10B. In some other embodiments, if the user is in an exercise state in a process of performing cardiac function monitoring by the electronic device 100, the electronic device 100 may directly display the output interface 1010 shown in FIG. 10B after determining the cardiac function indicator.

As shown in FIG. 10B, the diagram 1014 of the cardiac function indicator may represent a relationship between a stroke volume (or a cardiac output) of the user and an exercise heart rate. Optionally, in this case, the output interface 1010 may further display a data sharing control 1015, and the data sharing control 1015 may be configured to trigger the electronic device 100 to send the cardiac function indicator, the determining result of the cardiac function indicator, and the like to another electronic device (for example, an electronic device selected by the user from an address book of the electronic device 100, or an electronic device preset by the user).

For another example, FIG. 10C shows an output interface 1020 when a determining result is abnormal according to an embodiment of this application.

As shown in FIG. 10C, the output interface 1020 may include a plurality of cardiac function indicators. For example, a stroke volume is 50 ml, a cardiac output is 3.0 L/min, and a heart rate is 60 beats/min. The electronic device 100 may further display a determining result of the cardiac function indicator near the cardiac function indicator, for example, a determining result 1021, a determining result 1022, and a determining result 1023. For function descriptions and form descriptions of the determining result, refer to related descriptions in the embodiment shown in FIG. 10A. In the embodiment shown in FIG. 10B, both the determining result 1021 of the cardiac output of the user and the determining result 1022 of the stroke volume are downward arrows, indicating that the cardiac output and the stroke volume of the user are low. In addition, the determining result of the heart rate is a transverse line, indicating that the heart rate of the user is normal. It may be understood that, in some other embodiments, the electronic device 100 may alternatively output the determining result of each cardiac function indicator by using a symbol different from the foregoing arrow, or by using an identifier with a different color. For example, a red identifier for the user indicates that the cardiac function indicator of the user is high, a yellow identifier for the user indicates that the cardiac function indicator of the user is low, and a green identifier indicates that the cardiac function indicator of the user is normal. This application imposes no limitation on an output form of the determining result.

It may be understood that the embodiments shown in FIG. 10A to FIG. 10C are merely some examples. In embodiments of this application, an output interface for outputting the cardiac function indicator by the electronic device 100 may further include more or less content than or content different from that in the foregoing embodiments. This is not limited in this application.

In some application scenarios, if the electronic device 100 configured to output the cardiac function indicator is the earphones 10, before outputting the cardiac function indicator, the electronic device 100 may determine an output manner of the cardiac function indicator based on factors such as whether the user wears the earphones and whether the electronic device 100 is connected to the electronic device 200.

The following describes a procedure of determining an output manner of a cardiac function indicator when the electronic device 100 is earphones 10 according to an embodiment of this application.

As shown in FIG. 11, when the electronic device 100 is earphones 10, a specific procedure in which the earphones 10 determine an output manner of a cardiac function indicator may include the following steps.

S1101: The earphones 10 determine whether the earphones 10 are worn by a user.

The earphones 10 may separately determine whether a left earphone 11 and a right earphone 12 are worn by the user.

In some embodiments, earphone wearing detection may be implemented by connecting a pull-up resistor to a pin of the earphones 10. For example, after the left earphone 11 is inserted, a front end of the left earphone 11 (that is, a contact of a left channel) connects a detection pin to the left channel. In this case, because the left channel is connected to the ground and a resistance is small, it is equivalent to that the detection pin is grounded, and therefore the detection pin changes from a high level to a low level. After detecting that the high level changes to the low level, the detection pin may send an interrupt signal to a processor (for example, a CPU) of the earphones 10. The processor may determine, based on whether the interrupt signal is received, whether the left earphone 11 is worn. It may be understood that whether the right earphone 12 is worn by the user may also be determined in a similar manner.

In some other embodiments, earphone wearing monitoring may be alternatively implemented by using a component such as a gyro sensor or an acceleration sensor in the earphones 10.

It may be understood that the wearing detection manners herein are merely two examples. In this embodiment of this application, the earphones 10 may alternatively determine, in another manner, whether the earphones are worn by the user. This is not limited in this application.

If it is detected that the left earphone 11 and/or the right earphone 12 are/is worn by the user, the earphones 10 may perform the following step S1102.

If it is detected that neither the left earphone 11 nor the right earphone 12 is worn by the user, the earphones 10 may perform the following step S1103, or may perform the following step S1105.

S1102: The earphones 10 play a cardiac function indicator at volume 1.

In some embodiments, if the earphones 10 has played audio before performing cardiac function monitoring, the volume 1 may be volume of the audio previously played by the earphones 10.

The cardiac function indicator may include a cardiac output and a stroke volume, and optionally, may further include an indicator such as a heart rate.

In some embodiments, when playing the cardiac function indicator, the earphones 10 may also play a determining result. For specific content of the determining result, refer to related descriptions in step S707 shown in FIG. 7. Details are not described herein again.

S1103: The earphones 10 determine whether a communication connection is established between the earphones 10 and an electronic device 200.

When the user does not wear the earphones 10, the earphones 10 may further determine whether a communication connection is established to another electronic device (referred to as the electronic device 200 herein). The communication connection may be a wired communication connection, or may be a wireless communication connection.

If a communication connection is established between the earphones 10 and the electronic device 200, the earphones 10 may perform the following step S1104.

If no communication connection is established between the earphones 10 and any electronic device, the earphones 10 may perform the following step S1105.

S1104: The earphones 10 send an output instruction 1 to the electronic device 200, where the output instruction 1 includes a cardiac function indicator.

S1105: The earphones 10 play a cardiac function indicator at volume 2, where the volume 2 is greater than the volume 1.

When the user does not wear the earphones 10, the earphones 10 may play the cardiac function indicator at the volume 2. The volume 2 is greater than the volume 1.

In this way, when the user does not wear the earphones 10, the earphones 10 may increase the volume of playing the cardiac function indicator, so that the user hears played content.

It may be understood that the embodiment shown in FIG. 11 is merely an example for description. Factors such as whether the earphones 10 are worn and whether the earphones 10 establish a communication connection to another electronic device affect a manner of outputting the cardiac function indicator by the earphones 10. In this embodiment of this application, the earphones 10 may alternatively determine, in a manner different from that in the embodiment shown in FIG. 11, a manner of outputting the cardiac function indicator by the earphones 10, for example, receiving an operation of the user on a specified button on the earphones 10, and playing the cardiac function indicator in response to the operation. This is not limited in this application.

In some embodiments, for example, in the application scenario shown in FIG. 6F, the earphones 10 and the mobile phone 30 may cooperatively monitor the cardiac function of the user. The following describes a multi-device cooperative monitoring method according to an embodiment of this application.

FIG. 12A-1 and FIG. 12A-2 are a schematic flowchart of another monitoring method according to an embodiment of this application.

For example, as shown in FIG. 12A-1 and FIG. 12A-2 , a specific procedure of the monitoring method may include the following steps.

S1201: Earphones 10 start cardiac function monitoring.

For specific content of step S1201, refer to related descriptions in step S701 shown in FIG. 7. Details are not described herein again.

S1202: The earphones 10 sends a start notification to a mobile phone 30, where the start notification is used to notify the mobile phone 30 to start cardiac function monitoring.

The earphones 10 may send the start notification to the mobile phone 30 through a communication connection to the mobile phone 30. In some embodiments, the communication connection between the earphones 10 and the mobile phone 30 may be a wired communication connection. In some other embodiments, the communication connection between the earphones 10 and the mobile phone 30 may alternatively be a wireless communication connection such as a Bluetooth connection.

S1203: The earphones 10 obtain a wearing status of the earphones 10, where the wearing status indicates whether a left earphone 11 and a right earphone 12 are worn by a user.

The earphones 10 may perform wearing detection, determine whether the left earphone 11 is worn by the user, determine whether the right earphone 12 is worn by the user, and determine the wearing status of the earphones 10 based on a wearing detection result.

S1204: The earphones 10 determine a working electrode based on the wearing status of the earphones 10, where the working electrode is an electrode used in a monitoring process.

When it is determined that the user wears the left earphone 11 and does not wear the right earphone 12, the earphones 10 may determine an electrode (for example, the electrode 1 and the electrode 3) on the left earphone 11 as a working electrode.

When it is determined that the user does not wear the left earphone 11 and wears the right earphone 12, the earphones 10 may determine an electrode (for example, the electrode 2 and the electrode 4) on the right earphone 12 as a working electrode.

When it is determined that the user wears the left earphone 11 and the right earphone 12, the earphones 10 may determine an electrode on the left earphone 11 as a working electrode, or determine an electrode on the right earphone 12 as a working electrode.

S1205: The earphones 10 output a prompt 3 based on the working electrode of the earphones 10, where the prompt 3 is used to inform the user of a hand and a holding posture for holding the mobile phone 30.

When it is determined that the electrode on the left earphone 11 is the working electrode, the prompt 3 may prompt the user to hold the mobile phone 30 with the right hand, and make the right hand be in contact with at least one electrode on the mobile phone 30 when the mobile phone 30 is held.

When it is determined that the electrode on the right earphone 12 is the working electrode, the prompt 3 may prompt the user to hold the mobile phone 30 with the left hand, and make the left hand be in contact with at least one electrode on the mobile phone 30 when the mobile phone 30 is held.

In this way, it can be ensured that a contact point between the working electrode of the earphones 10 and skin of the user and a contact point between the working electrode of the mobile phone 30 and the skin of the user are respectively located at two ends of thoracic tissue, and it is determined that a resistance between the working electrodes of the two devices is a body resistance of the user.

S1206: The mobile phone 30 determines a working electrode in the mobile phone 30 based on the posture in which the user holds the mobile phone 30.

After receiving the start notification sent by the earphones 10, the mobile phone 30 may determine the posture in which the user holds the mobile phone 30, and determine the working electrode of the mobile phone 30.

In some embodiments, because the mobile phone 30 may determine a position of each electrode on the mobile phone 30 relative to the mobile phone 30, the mobile phone 30 may determine, based on the posture in which the user holds the mobile phone 30, an electrode that can be in contact with the skin of the user in the holding posture. It should be noted that in some embodiments, the mobile phone 30 may obtain, by using a sensor such as a gyroscope, the posture in which the user holds the mobile phone 30. In some other embodiments, the mobile phone 30 may alternatively determine, in another manner, the posture in which the user holds the mobile phone 30. This is not limited in this application.

For example, if the mobile phone 30 is the mobile phone 30 shown in FIG. 4B, and the holding posture of the user is holding the left and right sides of the mobile phone 30 with one hand, the mobile phone 30 may determine that the user can be in contact with all electrodes on the mobile phone 30. Therefore, the mobile phone 30 may select any one (or two) electrodes as working electrodes of the mobile phone 30.

S1207: The earphones 10 determine whether the working electrode of the earphones 10 is in good contact with the skin.

Step S1207 to step S1210 are all optional steps. In some embodiments, if the earphones 10 have more than one working electrode, the earphones 10 may perform step S1207 and step S1208; or if the mobile phone 30 has more than one working electrode, the mobile phone 30 may perform the following step S1209 and step S1210. In addition, step S1207 and step S1209 may be performed simultaneously, or only one of the steps may be performed, or the steps may be performed in sequence. In addition, an execution sequence of step S1207 and step S1209 is not limited in this embodiment of this application.

If the earphones 10 include at least two working electrodes, and the mobile phone 30 also includes at least two working electrodes, two working electrodes of the earphones 10 and two working electrodes of the mobile phone 30 may form a new monitoring circuit, and a circuit component in the monitoring circuit may be disposed in the earphones 10 and/or the mobile phone 30. In some embodiments, for a connection manner of circuit components in the monitoring circuit, refer to related descriptions in the embodiment shown in FIG. 5E. The two working electrodes of the earphones 10 may correspond to the electrode 1 and the electrode 3 shown in FIG. 5E, and the two working electrodes of the mobile phone 30 may correspond to the electrode 2 and the electrode 4 shown in FIG. 5E. In some other embodiments, the monitoring circuit including the working electrodes in the earphones 10 and the mobile phone 30 may alternatively be a monitoring circuit different from that in the foregoing embodiment. This is not limited in this application.

For a manner in which the earphones 10 determine whether the working electrode of the earphones 10 is in good contact with the skin, refer to related descriptions in step S703 shown in FIG. 7. Details are not described herein again.

When the earphones 10 determine that the working electrode of the earphones 10 is in good contact with the skin, the earphones 10 may perform the following step S1211. Optionally, the earphones 10 may also send a contact notification to the mobile phone 30, to notify the mobile phone 30 that the working electrode of the earphones 10 is in good contact.

When the earphones 10 determine that the working electrode of the earphones 10 is in poor contact with the skin, the earphones 10 may perform the following step S1208.

S1208: The earphones 10 output a prompt 4, where the prompt 4 is used to inform the user that the working electrode of the earphones 10 is in poor contact with the skin.

In some embodiments, the earphones 10 may output the prompt 4 in one or more manners such as voice playing, indicator blinking, and vibration. In some other embodiments, if the earphones 10 include a display, the earphones 10 may alternatively output the prompt 4 in a display manner on the display. In some other embodiments, the earphones 10 may alternatively send a reminder instruction 11 to the mobile phone 30, where the reminder instruction 1 indicates the mobile phone 30 to output the prompt 4, to inform the user that the working electrode of the earphones 10 is in poor contact with the skin of the user.

S1209: The mobile phone 30 determines whether the working electrode of the mobile phone 30 is in good contact with the skin.

For a manner in which the mobile phone 30 determines whether the working electrode of the mobile phone 30 is in good contact with the skin, refer to related descriptions in step S703 shown in FIG. 7. Details are not described herein again.

When the mobile phone 30 determines that the working electrode of the mobile phone 30 is in good contact with the skin, the mobile phone 30 may perform the following step S1211. Optionally, the mobile phone 30 may also send a contact notification to the earphones 10, to notify the earphones 10 that the working electrode of the mobile phone 30 is in good contact.

When the mobile phone 30 determines that the working electrode of the mobile phone 30 is in poor contact with the skin, the mobile phone 30 may perform the following step S1209.

S1210: The mobile phone 30 outputs a prompt 5, where the prompt 5 is used to inform the user that the working electrode of the mobile phone 30 is in poor contact with the skin.

In some embodiments, the mobile phone 30 may output the prompt 5 in one or more manners such as displaying on a display, voice playing, indicator blinking, and vibration. In some other embodiments, the mobile phone 30 may alternatively send a reminder instruction 2 to the earphones 10, where the reminder instruction 2 indicates the earphones 10 to output the prompt 5, to inform the user that the working electrode of the mobile phone 30 is in poor contact with the skin of the user.

S1211: The earphones 10 and the mobile phone 30 determine a cardiac impedance curve by using the working electrodes, where the cardiac impedance curve represents a relationship between cardiac impedance of the user and time.

In some embodiments, if the earphones 10 and the mobile phone 30 perform the foregoing step S1207 to step S1210, the earphones 10 and the mobile phone 30 perform step S1211 after determining that all working electrodes are in good contact.

S1212: The earphones 10 determine a cardiac function indicator based on the cardiac impedance curve.

S1213: The earphones 10 output the cardiac function indicator.

For specific content of step S1211 to step S1213, refer to related descriptions in step S705 to step S707 shown in FIG. 7. Details are not described herein again.

According to the monitoring method provided in this embodiment of this application, the cardiac function of the user may be monitored by using the earphones 10 and the mobile phone 30 (or a plurality of other electronic devices).

It should be noted that the embodiment shown inFIG. 12A-1 and FIG. 12A-2 merely describes an example in which the cardiac function of the user may be monitored in a multi-device cooperative manner. In this embodiment of this application, electronic devices that perform the monitoring method shown in FIG. 12A-1 and FIG. 12A-2 may alternatively be other electronic devices different from the earphones 10 and the mobile phone 30, for example, the earphones 10 and a tablet computer, or the earphones 10 and a watch. This is not limited in this application.

In some other embodiments, step S1201 and any one or more of step S1212 and step S1213 shown in FIG. 12A-1 and FIG. 12A-2 may be alternatively performed by the mobile phone 30. An execution body of step S1201 and step S1212 and step S1213 is not limited in this embodiment of this application.

In some application scenarios, when the mobile phone 30 and the earphones 10 perform cardiac function monitoring in cooperation, the mobile phone 30 may output a prompt to inform the user how to hold the mobile phone, or prompt the user whether the electrode is in good contact with the skin.

For example, when the left earphone is worn, the mobile phone 30 may display, based on a wearing detection result sent by the earphones 10, a guidance interface 1200 shown in FIG. 12B.

As shown in FIG. 12B, the guidance interface 1200 includes an operation guidance. The operation guidance may include an animation 1201 and a text 1202. The operation guidance may guide the user with a posture for holding the mobile phone and/or a manner for wearing the earphones. For example, when the left earphone is worn, the animation 1201 may be a dynamic image of holding the mobile phone with the right hand, and the text 1202 may include "Detect that the left earphone is worn. Hold the mobile phone with your right hand".

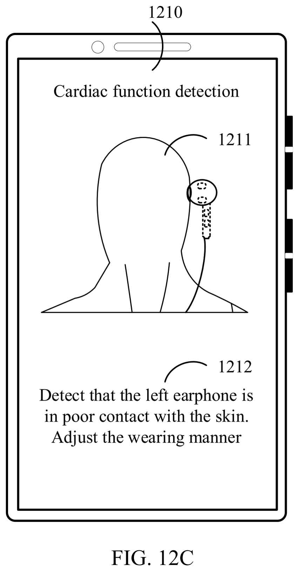

For another example, when it is detected that the left earphone 11 is in poor contact with the skin of the user, the mobile phone 30 may display a prompt interface 1210 shown in FIG. 12C.

As shown in FIG. 12C, the prompt interface 1210 may include a poor contact prompt, and the poor contact prompt may include an animation 1211 and a text 1212. The poor contact prompt may be used to inform the user that an electrode is in poor contact with the skin of the user currently. For example, when the left earphone is in poor contact with the skin, the animation 1211 may be a dynamic image in which the user wears the left earphone, and the text 1212 may include "Detect that the left earphone is in poor contact with the skin. Adjust the wearing manner".

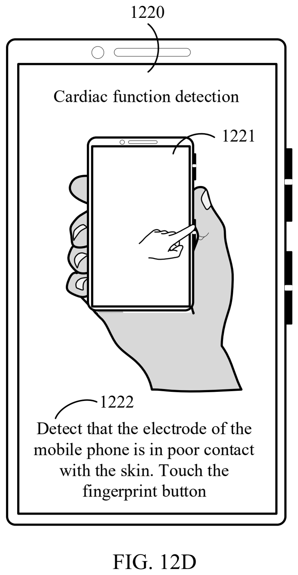

For another example, when it is detected that the electrode on the mobile phone 30 is in poor contact with the skin, the mobile phone 30 may display a prompt interface 1220 shown in FIG. 12D.

As shown in FIG. 12D, the prompt interface 1220 may include a poor contact prompt, and the poor contact prompt may include an animation 1221 and a text 1222. The poor contact prompt may be used to inform the user that an electrode is in poor contact with the skin of the user currently. For example, when the electrode of the mobile phone is in poor contact with the skin, the animation 1221 may be a dynamic image in which a finger of the user touches the electrode, and the text 1222 may include "Detect that the electrode of the mobile phone is in poor contact with the skin. Touch the fingerprint button".

It may be understood that FIG. 12B to FIG. 12D are merely some examples. In embodiments of this application, the mobile phone 30 may further output more or fewer prompts than or prompts different from those in the foregoing embodiments. This is not limited in this application.

The following describes functional modules of an electronic device 100 according to an embodiment of this application.

FIG. 13 is a diagram of functional modules of an electronic device 100 according to an embodiment of this application.

As shown in FIG. 13, the electronic device 100 may include an interaction module 1301, a data monitoring module 1302, a data processing module 1303, and an output module 1304. Optionally, the electronic device 100 may further include any one or more of the following: a contact detection module 1305 and a status detection module 1306.

The interaction module 1301 may receive an operation of a user, for example, an operation of starting cardiac function monitoring by the user, or an operation of viewing a cardiac function indicator by the user. In some embodiments, the interaction module 1301 may receive the operation of starting cardiac function monitoring by the user, and send a start instruction to the data monitoring module 1302 in response to the operation. The start instruction indicates the data monitoring module 1302 to start to monitor a current flowing through a body of the user and a voltage between two ends of thoracic tissue of the user. In some other embodiments, the interaction module 1301 may receive the operation of starting cardiac function monitoring by the user, and send a contact detection instruction to the contact detection module 1305 in response to the operation. The contact detection instruction indicates the contact detection module 1305 to determine a status of contact between an electrode in a monitoring circuit and skin. In some embodiments, the interaction module 1301 may further receive the operation of starting cardiac function monitoring by the user, and send a detection instruction to the status detection module 1306 in response to the operation. The detection instruction indicates the status detection module 1306 to determine a current status of the user. In some other embodiments, the interaction module 1301 may also receive an operation of setting the current status (for example, a resting state, a sleep state, or an exercise state) by the user, determine the user status in response to the operation, and send the user status to the data processing module 1303.

The data monitoring module 1302 may receive the start instruction, and in response to the start instruction, start to monitor the current flowing through the body of the user and the voltage between the two ends of the thoracic tissue of the user. It should be noted that the start instruction may be sent by the interaction module 1301, or may be sent by the electronic device 200. In some embodiments, the data monitoring module 1302 may further determine a cardiac impedance curve of the user based on the current flowing through the body of the user and the voltage between the two ends of the thoracic tissue of the user. After determining the cardiac impedance curve, the data monitoring module 1302 may send the cardiac impedance curve to the data processing module 1303. It should be noted that the start instruction received by the data monitoring module 1302 may be sent by the interaction module 1301, or may be sent by the contact detection module 1305.

The data processing module 1303 may determine a plurality of cardiac function indicators of the user based on the cardiac impedance curve. The cardiac function indicators may include a cardiac output and a stroke volume, and optionally, may further include a heart rate of the user. The data processing module 1303 may further send the cardiac function indicators to the output module 1304. In some embodiments, the data processing module 1303 may store value ranges of the plurality of cardiac function indicators. The data processing module 1303 may further determine a determining result based on actual values of the cardiac function indicators and the preset value ranges. The determining result may indicate whether a single cardiac function indicator is normal, indicate whether a cardiac function of the user is normal, and/or the like. Optionally, the data processing module 1303 may further store value ranges of the plurality of cardiac function indicators in different states. In this case, the data processing module 1303 may further determine a determining result based on the user status sent by the status detection module 1306, actual values of the cardiac function indicators, and preset value ranges in the current user status. After determining the determining result, the data processing module 1303 may also send the determining result to the output module 1304.

The output module 1304 may output the cardiac function indicators sent by the data processing module 1303, and optionally, may further output the determining result sent by the data processing module, and the like. In some embodiments, the output module 1304 may also receive a contact detection result sent by the contact detection module 1305, and output a prompt 2 in response to the contact detection result. The prompt 2 is used to inform the user that an electrode is in poor contact with the skin. In some embodiments, the output module 1304 may further determine an output manner, output volume, or the like. In some embodiments, the output module 1304 outputs the cardiac function indicators, or may send an output instruction 1 to the electronic device 200. The output instruction 1 includes the cardiac function indicators, and the output instruction 1 indicates the electronic device 200 to output the cardiac function indicators.

The contact detection module 1305 may receive a contact detection instruction sent by the interaction module 1301, and in response to the contact detection instruction, determine whether the electrode in the monitoring circuit is in good contact with the skin. When determining that all electrodes are in good contact with the skin, the contact detection module 1305 may send a start instruction to the data monitoring module 1302. The start instruction indicates the data monitoring module 1302 to start to monitor the current flowing through the body of the user and the voltage between the two ends of the thoracic tissue of the user. When determining that an electrode is in poor contact with the skin, the contact detection module 1305 may send a contact detection result to the output module 1304. The contact detection result indicates that an electrode in poor contact with the skin exists in the monitoring circuit, or indicates an electrode in poor contact with the skin in the monitoring circuit.

The status detecting module 1306 may determine the current status of the user. The status of the user may include a resting state and an exercise state, and optionally, may further include a sleep state. In some embodiments, the status detection module 1306 may monitor the status of the user in real time. In some other embodiments, the status detection module 1306 may also receive a detection instruction sent by the interaction module 1301, and determine the current status of the user in response to the detection instruction. After determining the status of the user, the status detection module 1306 may send the status of the user to the data processing module 1303.

It may be understood that the embodiment shown in FIG. 13 is merely an example. In embodiments of this application, the electronic device 100 may further include more or fewer functional modules than or functional modules different from those in the embodiment shown in FIG. 13. In addition, a plurality of functional modules in the foregoing embodiment may be combined into one functional module, or one functional module in the foregoing embodiment may be split into a plurality of functional modules. This is not limited in this application.

The following describes functional modules of a health monitoring system 1000 according to an embodiment of this application.

FIG. 14 is a diagram of functional modules of a health monitoring system 1000 according to an embodiment of this application.

As shown in FIG. 14, the health monitoring system 1000 may include an electronic device 100 and an electronic device 200. The electronic device 100 may include a data monitoring module 1302 and a communication module 1307, and the electronic device 200 may include a communication module 1401, an interaction module 1402, a data processing module 1403, an output module 1404, and the like.

The data monitoring module 1302 may receive a start instruction sent by the communication module 1307, and in response to the start instruction, start to monitor a current flowing through a body of a user and a voltage between two ends of thoracic tissue of the user. In some embodiments, the data monitoring module 1302 may further determine a cardiac impedance curve of the user based on the current flowing through the body of the user and the voltage between the two ends of the thoracic tissue of the user. After determining the cardiac impedance curve, the data monitoring module 1302 may send the cardiac impedance curve to the communication module 1307.

The communication module 1307 may receive a start instruction sent by the communication module 1401 in the electronic device 200, and send the start instruction to the data monitoring module 1302. The communication module 1307 may further receive a cardiac impedance curve sent by the data monitoring module 1302, and send the cardiac impedance curve to the communication module 1401.

The communication module 1401 may receive a start instruction sent by the interaction module 1402, and send the start instruction to the communication module 1307. The communication module 1401 may further receive a cardiac impedance curve sent by the communication module 1307, and send the cardiac impedance curve to the data processing module 1403.

The interaction module 1402 may receive an operation of starting cardiac function monitoring by the user and send a start instruction to the communication module 1401 in response to the operation.

The data processing module 1403 may determine a plurality of cardiac function indicators, for example, a cardiac output and a stroke volume, based on the cardiac impedance curve sent by the communication module 1401. The data processing module 1403 may send the cardiac function indicators to the output module 1404. Optionally, the data processing module 1403 may further determine a determining result. For specific content of the determining result, refer to the description in the foregoing embodiment. Details are not described herein again. The data processing module 1403 may also send the determining result to the output module 1404.

The output module 1404 may output the cardiac function indicators sent by the data processing module 1403, and optionally, may further output the determining result sent by the data processing module 1403.

It may be understood that the embodiment shown in FIG. 14 is merely an example. In embodiments of this application, the health monitoring system 1000 may further include more or fewer functional modules than those in the embodiment shown in FIG. 14 or functional modules different from those in the embodiment shown in FIG. 14. Some functional modules (for example, any one or more of the interaction module, the data processing module, and the output module) in the electronic device 200 may be alternatively disposed in the electronic device 100. In addition, some functional modules may form one functional module, and one functional module may be split into a plurality of functional modules. This is not limited in this application. For specific function descriptions of the contact detection module and the status detection module, refer to related descriptions in the embodiment shown in FIG. 13.

In some other embodiments, the electronic device 100 may further include any one or more of the following modules: an interaction module 1301, a data processing module 1303, an output module 1304, a contact detection module 1305, a status detection module 1306, and the like. For specific functions of the foregoing modules, refer to related descriptions in the embodiment shown in FIG. 13. In addition, it should be noted that the interaction module, the data processing module, and the output module may be disposed in a same electronic device (for example, the electronic device 100 or the electronic device 200), or may be separately disposed in the electronic device 100 and the electronic device 200, or a same functional module may be disposed in the electronic device 100 and the electronic device 200. For example, the output module 1304 is disposed in the electronic device 100, and the output module 1404 is disposed in the electronic device 200. This is not limited in this application.

For ease of subsequent description, the electronic device 100 and the electronic device 200 may be collectively referred to as apparatuses. It should be understood that division into the units in the apparatus is merely logical function division. During actual implementation, all or some of the units may be integrated into one physical entity, or may be physically separated. In addition, the unit in the apparatus may be implemented in a form of invoking software by a processor. For example, the apparatus includes a processor, the processor is connected to a memory, the memory stores instructions, and the processor invokes the instructions stored in the memory, to implement any one of the foregoing methods or functions of the units in the apparatus. The processor is, for example, a general-purpose processor like a central processing unit (central processing unit, CPU) or a microprocessor, and the memory is a memory inside the apparatus or a memory outside the apparatus. Alternatively, the unit in the apparatus may be implemented in a form of a hardware circuit, and functions of some or all units may be implemented by designing the hardware circuit. The hardware circuit may be understood as one or more processors. For example, in an implementation, the hardware circuit is an application-specific integrated circuit (application-specific integrated circuit, ASIC), and functions of some or all of the foregoing units are implemented by designing a logical relationship between elements in the circuit. For another example, in another implementation, the hardware circuit may be implemented by using a programmable logic device (programmable logic device, PLD). Using a field programmable gate array (field programmable gate array, FPGA) as an example, the field programmable gate array may include a large quantity of logic gate circuits, and a connection relationship between the logic gate circuits is configured by using a configuration file, to implement functions of some or all of the foregoing units. All the units of the foregoing apparatus may be implemented in a form of invoking software by a processor, or may be implemented in a form of a hardware circuit, or some of the units are implemented in a form of invoking software by a processor, and the remaining units are implemented in a form of a hardware circuit.

In embodiments of this application, the processor is a circuit with a signal processing capability. In an implementation, the processor may be a circuit with an instruction reading and running capability, for example, a CPU, a microprocessor, a graphics processing unit (graphics processing unit, GPU) (which may be understood as a microprocessor), or a digital signal processor (digital signal processor, DSP). In another implementation, the processor may implement a specific function based on a logical relationship of a hardware circuit. The logical relationship of the hardware circuit is fixed or reconfigurable. For example, the processor is a hardware circuit implemented by an ASIC or a PLD, for example, an FPGA. In the reconfigurable hardware circuit, a process in which the processor loads a configuration file to implement hardware circuit configuration may be understood as a process in which the processor loads instructions to implement functions of some or all of the foregoing units. In addition, the processor may alternatively be a hardware circuit designed for artificial intelligence, and may be understood as an ASIC, for example, a neural network processing unit (Neural Network Processing Unit, NPU), a tensor processing unit (tensor processing unit, TPU), or a deep learning processing unit (deep learning processing unit, DPU).

It can be learned that the units in the foregoing apparatus may be configured as one or more processors (or processing circuits) for implementing the foregoing methods, for example, a CPU, a GPU, an NPU, a TPU, a DPU, a microprocessor, a DSP, an ASIC, an FPGA, or a combination of at least two of these processor forms.

In addition, all or some of the units in the foregoing apparatus may be integrated, or may be implemented independently. In an implementation, these units are integrated together and implemented in a form of a system-on-a-chip (system-on-a-chip, SOC). The SOC may include at least one processor, configured to implement any one of the foregoing methods or implement functions of the units of the apparatus. Types of the at least one processor may be different, for example, include a CPU and an FPGA, a CPU and an artificial intelligence processor, a CPU and a GPU, or the like.

The following describes a possible physical entity structure of an electronic device 100 according to an embodiment of this application.

For example, FIG. 15 is a diagram of a physical entity structure of an electronic device 100 according to an embodiment of this application.

As shown in FIG. 15, the electronic device 100 may be the electronic device 100 in the foregoing embodiment. The electronic device 100 may include a processor 1501 and a memory 1502. Optionally, the electronic device 100 may further include a transmitter 1503 and a receiver 1504. The processor 1501, the memory 1502, the transmitter 1503, and the receiver 1504 may be connected to each other or connected to each other by using a bus 1505.

For example, the memory 1502 is configured to store a computer program and data of the electronic device 100, and the memory 1502 may include but is not limited to a random access memory (random access memory, RAM), a read-only memory (read-only memory, ROM), an erasable programmable read-only memory (erasable programmable read-only memory, EPROM), a compact disc read-only memory (compact disc read-only memory, CD-ROM), or the like.

Software or program code required for all or some of the functions of the electronic device 100 in the foregoing method embodiments is stored in the memory 1502.

In a possible implementation, if software or program code required for some of the functions is stored in the memory 1502, the processor 1501, in addition to invoking the program code in the memory 1502 to implement some of the functions, may cooperate with another component (for example, the transmitter 1503 and the receiver 1504) to jointly complete another function (for example, a data receiving or sending function) described in the method embodiments.

The transmitter 1503 and the receiver 1504 are configured to support the electronic device 100 in performing communication, for example, receiving or sending data or a signal.

In some embodiments, the transmitter 1503 may send, to the electronic device 200, a cardiac impedance curve monitored by the electronic device 100. In some other embodiments, the transmitter 1503 may also send, to the electronic device 200, a current curve and a voltage curve that are collected by the electronic device 100.

In some embodiments, the transmitter 1503 may further send an output instruction 1 to the electronic device 200. The output instruction 1 may include a cardiac function indicator, and the output instruction 1 indicates the electronic device 200 to output the cardiac function indicator. Optionally, the output instruction 1 may further include a determining result.

In some embodiments, the receiver 1504 may receive a start instruction sent by the electronic device 200 to the electronic device 100. The start instruction may be used to trigger the electronic device 100 to start cardiac function monitoring.

In some embodiments, the receiver 1504 may receive user information sent by the electronic device 200 to the electronic device 100.

In some embodiments, the receiver 1504 may receive the cardiac function indicator and/or the determining result sent by the electronic device 200 to the electronic device 100.

For example, the processor 1501 may be the CPU, the GPU, the NPU, the TPU, the DPU, the microprocessor, the DSP, the ASIC, the FPGA, or a combination of at least two of these processor forms that are described above. The processor 1501 may be configured to read a program stored in the memory 1502, to perform an operation performed by the electronic device 100 in any one of the foregoing embodiments.

For specific operations and beneficial effects of the units in the electronic device 100 shown in FIG. 15, refer to corresponding descriptions in the foregoing method embodiments. Details are not described herein again.

It may be understood that the embodiment shown in FIG. 15 is merely an example. In embodiments of this application, the electronic device 100 may further include more or fewer components than those in the embodiment shown in FIG. 15, or components different from those in the embodiment shown in FIG. 15. This is not limited in this application.

The following describes a chip system according to an embodiment of this application.

This application further provides a chip system. The chip system includes at least one processor, configured to implement functions related to the electronic device 100 in any one of the foregoing embodiments.

In a possible design, the chip system further includes a memory, the memory is configured to store program instructions and data, and the memory is located inside or outside the processor.

The chip system may include a chip, or may include a chip and another discrete component.

Optionally, there may be one or more processors in the chip system. The processor may be implemented by using hardware, or may be implemented by using software. When the processor is implemented by using the hardware, the processor may be a logic circuit, an integrated circuit, or the like. When the processor is implemented by using the software, the processor may be a general-purpose processor, and is implemented by reading software code stored in the memory.

Optionally, there may be one or more memories in the chip system. The memory may be integrated with the processor, or may be disposed separately from the processor. This is not limited in embodiments of this application. For example, the memory may be a non-transitory processor, for example, a read-only memory ROM. The memory and the processor may be integrated into a same chip, or may be respectively disposed on different chips. A type of the memory and a manner of disposing the memory and the processor are not specifically limited in embodiments of this application.

For example, the chip system may be a field programmable gate array (field programmable gate array, FPGA), an application-specific integrated circuit (application-specific integrated circuit, ASIC), a system on a chip (system on a chip, SoC), a central processing unit (central processing unit, CPU), a network processor (network processor, NP), a digital signal processor (digital signal processor, DSP), a micro controller unit (micro controller unit, MCU), a programmable logic device (programmable logic device, PLD), or another integrated chip.

It may be understood that the foregoing chip system is merely an example. In embodiments of this application, the chip system may alternatively include more or fewer components than those in the foregoing embodiment, or components different from those in the foregoing embodiment. This is not limited in this application.

The following describes a specific procedure of a monitoring method according to an embodiment of this application.

As shown in FIG. 16, a specific procedure of performing a monitoring method by a first electronic device may include the following steps.

S1601: The first electronic device receives a first instruction, where the first instruction instructs the first electronic device to start cardiac function monitoring.

The first electronic device may be the electronic device 100 in the foregoing embodiments, for example, the earphones 10, the watch 20, or the mobile phone 30.

The first electronic device is provided with a first circuit, and the first circuit includes a first electrode, a second electrode, a third electrode, a fourth electrode, an excitation current generation unit, and a voltage measurement unit. In this embodiment of this application, the first circuit may be the monitoring circuit in the foregoing embodiments, for example, the monitoring circuit in the embodiment shown in FIG. 5D. The first electrode may be the electrode 1, the second electrode may be the electrode 2, the third electrode may be the electrode 3, and the fourth electrode may be the electrode 4.

In a possible implementation, receiving the first instruction specifically includes: receiving the first instruction sent by a second electronic device; or receiving a first operation of the user on the first electronic device, and generating the first instruction. The second electronic device may be the electronic device 200 in the foregoing embodiments.

In another possible implementation, receiving the first instruction specifically includes: detecting that a monitoring condition is met, and generating the first instruction. For specific content of the monitoring condition, refer to related descriptions in step S701 shown in FIG. 7. Details are not described herein again.

S1602: In response to the first instruction, the first electronic device determines, through a first loop, that the first electrode is in good contact with skin, where the first loop includes the first electrode, the excitation current generation unit, and the third electrode.

After receiving the first instruction, the first electronic device may perform step S1602 and step S1603. It should be noted that, the first electronic device may first perform step S1602, and then perform step S1603; or the first electronic device may first perform step S1603, and then perform step S1602.

In addition, in a process in which the first electronic device performs step S1602 to step S1604, the first electrode is in contact with a first position on the skin of the user, the second electrode is in contact with a second position on the skin of the user, and the first position and the second position are respectively at two ends of thoracic tissue. In addition, the third electrode is in contact with a third position on the skin of the user, the fourth electrode is in contact with a fourth position on the skin of the user, a distance between the third position and the first position has a fixed value (that is, is equal to a distance between the first electrode and the third electrode), and a distance between the fourth position and the second position has a fixed value (that is, is equal to a distance between the second electrode and the fourth electrode). The third position and the first position are located on a same side of the thoracic tissue, and the fourth position and the second position are located on a same side of the thoracic tissue. In this embodiment of this application, the first position may be the contact point 1 in the foregoing embodiments, the second position may be the contact point 2 in the foregoing embodiments, the third position may be the contact point 3 in the foregoing embodiments, and the fourth position may be the contact point 4 in the foregoing embodiments.

The first loop may be the loop 1 shown in FIG. 5F.

For a specific manner in which the first electronic device determines that the first electrode is in good contact with the skin, refer to related descriptions in step S703 shown in FIG. 7. Details are not described herein again.

S1603: The first electronic device determines, through a second loop, that the second electrode is in good contact with the skin, where the second loop includes the second electrode, the excitation current generation unit, and the fourth electrode.

The second loop may be the loop 2 shown in FIG. 5G.

For a specific manner in which the first electronic device determines that the second electrode is in good contact with the skin, refer to related descriptions in step S703 shown in FIG. 7. Details are not described herein again.

S1604: The first electronic device determines first information through a third loop, where the third loop includes the first electrode, the excitation current generation unit, the voltage measurement unit, and the second electrode. The first electrode is in contact with the first position on the skin of the user, the second electrode is in contact with the second position on the skin of the user, and the first position and the second position are respectively at the two ends of the thoracic tissue.

In some embodiments, the third loop may include the loop 3 and the loop 4 shown in FIG. 5H.

In some other embodiments, when both the first electrode and the second electrode are in good contact with the skin of the user, the first electrode, the excitation current generation unit, and the second electrode may form a loop, the first electrode, the voltage measurement unit, and the second electrode may form another loop, and the third loop may include the two loops.

S1605: The first electronic device outputs the first information, where the first information includes one or more of the following: a cardiac output, a stroke volume, a heart rate, an ejection fraction, a comprehensive result, a first result, a second result, a third result, and a fourth result, the comprehensive result indicates whether a cardiac function of the user is normal, the first result indicates whether the cardiac output is normal, the second result indicates whether the stroke volume is normal, the third result indicates whether the heart rate is normal, and the fourth result indicates whether the ejection fraction is normal.

The cardiac output, the stroke volume, the heart rate, the ejection fraction, and the like belong to the cardiac function indicators in step S707 shown in FIG. 7. For determining manners and function descriptions of the first result, the second result, the third result, and the fourth result, refer to related descriptions of the determining results of the cardiac function indicators in step S707 shown in FIG. 7. The comprehensive result may be the comprehensive determining result in step S707 shown in FIG. 7.

For a specific manner of outputting the first information by the first electronic device, refer to related descriptions in step S707 shown in FIG. 7, or refer to related descriptions in the embodiments shown in FIG. 9A to FIG. 9C and FIG. 10A to FIG. 10C, or refer to related content in the embodiment shown in FIG. 11. Details are not described herein again.

In this way, the first electronic device can obtain a cardiac function monitoring result of the user in real time, so that the user can learn of a health condition of the user in a timely manner. In addition, the first electronic device may further determine whether an electrode is in good contact with the skin of the user.

In a possible implementation, the method further includes: outputting a first prompt in response to the first instruction, where the first prompt is used to inform the user that cardiac function monitoring is started.

The first prompt may be the prompt 1 in step S702 shown in FIG. 7.

In this way, the first electronic device may remind, by using the first prompt, the user whether cardiac function monitoring is started.

In a possible implementation, before determining the first information through the third loop, the method further includes: determining that a user status is a first state; and determining the first information through the third loop specifically includes: determining a cardiac function indicator through the third loop, where the cardiac function indicator includes any one or more of the following: the cardiac output, the stroke volume, the heart rate, and the ejection fraction; and determining the first information based on the first state and the cardiac function indicator.

In this way, the first electronic device can obtain the user status in real time, and determine, based on the user status, whether the cardiac function indicator of the user is normal.

In a possible implementation, the first state is an exercise state, a resting state, or a sleep state.

In another possible implementation, the first state may further include but is not limited to any one or more of the following: a scared state, an oxygen-deficient state, a diving state, a meditative state, and the like.

In a possible implementation, determining that the user status is the first state specifically includes: detecting that the user status is the first state; or receiving a status setting operation of the user, and in response to the operation, determining that the user status is the first state; or receiving second information sent by the second electronic device, and determining, based on the second information, that the user status is the first state.

The second information may be the user information in step S701 and step S707 shown in FIG. 7.

In this way, the first electronic device may determine the user status based on the status setting operation of the user, or may determine the user status based on information sent by another electronic device. The first electronic device may alternatively detect the user status by using a component such as a sensor.

In a possible implementation, when the first state is the exercise state, the first information further includes a first graphic, and the first graphic indicates a relationship between the stroke volume and an exercise heart rate.

For example, the first graphic may be the diagram 921 of the cardiac function indicator in the output interface 920 shown in FIG. 9C.

In this way, when the user is in the exercise state, the first electronic device may output the first graphic to inform the user of the relationship between the stroke volume and the exercise heart rate.

In a possible implementation, outputting the first information specifically includes: sending a second instruction to the second electronic device, where the second instruction indicates that the first electronic device outputs the first information.

In some embodiments, the second instruction may be the output instruction 1 in step S707 shown in FIG. 7.

In this way, the first electronic device may alternatively output the first information through the second electronic device.

In a possible implementation, the first electronic device is earphones, and the method further includes: playing, by the earphones, first audio before the first instruction is received; and pausing play of the first audio when the first instruction is received.

In a possible implementation, the first electronic device is the earphones; playing the first audio specifically includes: playing the first audio at first volume; and outputting the first information specifically includes: playing the first information at the first volume if the user wears a left earphone or a right earphone.

In a possible implementation, the first electronic device is the earphones, and the method further includes: playing the first information at second volume if the user wears neither the left earphone nor the right earphone, where the second volume is greater than the first volume.

Alternatively, the first volume may be the volume 1 in the embodiment shown in FIG. 11, and the second volume may be the volume 2 in the embodiment shown in FIG. 11.

In this way, when the first electronic device is the earphones, the first electronic device may determine output volume of the first information based on whether the user wears the earphones.

In a possible implementation, the first electronic device is the earphones, and outputting the first information specifically includes: sending a second instruction to the second electronic device if the user wears neither the left earphone nor the right earphone, where the second instruction indicates that the first electronic device outputs the first information.

In some embodiments, the second instruction may be the output instruction 1 in step S1104 shown in FIG. 11.

In this way, when the first electronic device is the earphones, the first electronic device may determine, based on whether the user wears the earphones, whether to output the first information through the second electronic device.

In a possible implementation, the first electronic device is earphones, the earphones include a left earphone and a right earphone, there is a wired connection between the left earphone and the right earphone, the first electrode and the third electrode are disposed on the left earphone, and the second electrode and the fourth electrode are disposed on the right earphone.

When the first electronic device is earphones, for a specific form description and electrode distribution of the earphones, refer to related descriptions of the earphones 10 in the embodiments shown in FIG. 2A to FIG. 2C.

In a possible implementation, the first electronic device is a wearable device, the wearable device includes a movement and a watchband, the first electrode and the third electrode are located at one end of the watchband, the second electrode and the fourth electrode are located at the other end of the watchband, and a length of the watchband is greater than a first length. The first length may be a shortest length capable of crossing two ends (for example, upper and lower ends or left and right ends) of the thoracic tissue.

In a possible implementation, the first electronic device is a wearable device, the wearable device includes a movement and a watchband, the movement includes a first button and a second button, the first electrode and the third electrode are located on a rear surface of the movement, the second electrode is located on the first button, and the fourth electrode is located on the second button.

In a possible implementation, the first electronic device is a wearable device, the wearable device includes a movement and a watchband, the movement includes a first button and a second button, the first electrode and the third electrode are located on the watchband, the second electrode is located on the first button, and the fourth electrode is located on the second button.

When the first electronic device is a wearable device, for a specific form description and electrode distribution of the wearable device, refer to related descriptions of the watch 20 in the embodiments shown in FIG. 3A to FIG. 3E.

In a possible implementation, the first electronic device is a mobile phone, the mobile phone includes one or more buttons, one or more of the first electrode, the second electrode, the third electrode, and the fourth electrode are disposed on the one or more buttons, and the first electrode, the second electrode, the third electrode, and the fourth electrode are not in contact with each other.

In a possible implementation, the one or more buttons of the mobile phone include a volume button and a fingerprint button, and that the one or more of the first electrode, the second electrode, the third electrode, and the fourth electrode are disposed on the one or more buttons specifically includes: The first electrode and the third electrode are disposed on the volume button, and the second electrode and the fourth electrode are disposed on the fingerprint button.

In a possible implementation, the one or more buttons of the mobile phone include a first volume button, a second volume button, and a fingerprint button, and that the one or more of the first electrode, the second electrode, the third electrode, and the fourth electrode are disposed on the one or more buttons specifically includes: The first electrode is disposed on the first volume button, the third electrode is disposed on the second volume button, and the second electrode and the fourth electrode are disposed on the fingerprint button.

When the first electronic device is a mobile phone, for a specific form description and electrode distribution of the mobile phone, refer to related descriptions of the mobile phone 30 in the embodiments shown in FIG. 4A and FIG. 4B.

In this way, when the first electronic device is in different device forms, each electrode may be disposed at different positions.

Implementations of this application may be randomly combined to achieve different technical effects.

All or some of the foregoing embodiments may be implemented by software, hardware, firmware, or any combination thereof. When software is used to implement the embodiments, all or some of the embodiments may be implemented in a form of a computer program product. The computer program product includes one or more computer instructions. When the computer program instructions are loaded and executed on a computer, the procedures or functions according to this application are all or partially generated. The computer may be a general-purpose computer, a dedicated computer, a computer network, or other programmable apparatuses. The computer instructions may be stored in a computer-readable storage medium or may be transmitted from a computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line) or wireless (for example, infrared, radio, or microwave) manner. The computer-readable storage medium may be any usable medium accessible by the computer, or a data storage device, such as a server or a data center, integrating one or more usable media. The usable medium may be a magnetic medium (for example, a floppy disk, a hard disk, or a magnetic tape), an optical medium (for example, a DVD), a semiconductor medium (for example, a solid state disk (solid state disk, SSD)), or the like.

A person of ordinary skill in the art may understand that all or some of the procedures in the methods in the foregoing embodiments may be completed by a computer program instructing related hardware. The program may be stored in a computer-readable storage medium. When the program is executed, the procedures in the foregoing method embodiments may be included. The foregoing storage medium includes any medium that can store program code, such as a ROM or a random access memory RAM, a magnetic disk, or an optical disc.

In conclusion, the foregoing descriptions are merely embodiments of the technical solutions of the present invention, but are not intended to limit the protection scope of the present invention. Any modification, equivalent replacement, or improvement made according to the disclosure of the present invention shall fall within the protection scope of the present invention.

## Claims

1. A monitoring circuit, comprising: a first electrode, a second electrode, a third electrode, a fourth electrode, a first analog switch, a second analog switch, an excitation current generation unit, and a voltage measurement unit, wherein the first analog switch comprises a first port and a second port, and the second analog switch comprises a third port and a fourth port;
the first electrode is connected to the second electrode through the first port of the first analog switch, the excitation current generation unit, and the third port of the second analog switch;
the third electrode is connected to the fourth electrode through the fourth port of the second analog switch, the excitation current generation unit, and the second port of the first analog switch;
the voltage measurement unit is connected to the third electrode and the fourth electrode, or the voltage measurement unit is connected to the first electrode and the second electrode; and
when the first electrode is in contact with a first position on skin of a user, the second electrode is in contact with a second position on the skin of the user, and the first position and the second position are respectively at two ends of thoracic tissue, the excitation current generation unit is configured to generate a current flowing through a heart of the user, and the voltage measurement unit is configured to measure a voltage between two ends of the heart of the user.

2. The circuit according to claim 1, wherein that the first electrode is connected to the second electrode through the first port of the first analog switch, the excitation current generation unit, and the third port of the second analog switch specifically comprises:
the first electrode, the first analog switch, the excitation current generation unit, the second analog switch, and the second electrode are sequentially connected, the first electrode is connected to the first port of the first analog switch, and the second electrode is connected to the third port of the second analog switch; and
that the third electrode is connected to the second electrode through the fourth port of the second analog switch, the excitation current generation unit, and the second port of the first analog switch specifically comprises:
the third electrode, the second analog switch, the excitation current generation unit, the first analog switch, and the fourth electrode are sequentially connected, the third electrode is connected to the fourth port of the second analog switch, and the fourth electrode is connected to the second port of the first analog switch.

3. The circuit according to claim 1 or 2, wherein the excitation current generation unit is connected to the first analog switch and the second analog switch.

4. The circuit according to any one of claims 1 to 3, wherein the first analog switch is configured to electrically connect the first port or the second port, and the second analog switch is configured to electrically connect the third port or the fourth port.

5. The circuit according to claim 4, wherein that the excitation current generation unit is configured to generate the current flowing through the heart of the user, and the voltage measurement unit is configured to measure the voltage between the two ends of the heart of the user specifically comprises:
when the first analog switch is electrically connected to the first port, and the second analog switch is electrically connected to the third port, the excitation current generation unit is configured to generate the current flowing through the heart of the user, and the voltage measurement unit is configured to measure the voltage between the two ends of the heart of the user; or
when the first analog switch is electrically connected to the second port, and the second analog switch is electrically connected to the fourth port, the excitation current generation unit is configured to generate the current flowing through the heart of the user, and the voltage measurement unit is configured to measure the voltage between the two ends of the heart of the user.

6. The circuit according to claim 4 or 5, wherein when the first analog switch is electrically connected to the first port, and the second analog switch is electrically connected to the fourth port, the monitoring circuit is configured to determine whether the first electrode and the third electrode are in good contact with the skin.

7. The circuit according to any one of claims 4 to 6, wherein when the first analog switch is electrically connected to the second port, and the second analog switch is electrically connected to the third port, the monitoring circuit is configured to determine whether the second electrode and the fourth electrode are in good contact with the skin.

8. The circuit according to claim 6 or 7, wherein when the first analog switch is electrically connected to the first port, and the second analog switch is electrically connected to the fourth port, or when the first analog switch is electrically connected to the second port, and the second analog switch is electrically connected to the third port, a frequency of the current generated by the excitation current generation unit is less than a first frequency.

9. The circuit according to any one of claims 1 to 7, wherein a frequency of the current generated by the excitation current generation unit is greater than a first frequency.

10. An electronic device, which is a first electronic device, wherein the first electronic device comprises the circuit according to any one of claims 1 to 9.

11. The electronic device according to claim 10, wherein the first electronic device is earphones, the earphones comprise a left earphone and a right earphone, there is a wired connection between the left earphone and the right earphone, the first electrode and the third electrode are disposed on the left earphone, and the second electrode and the fourth electrode are disposed on the right earphone.

12. The electronic device according to claim 10, wherein the first electronic device is a wearable device, the wearable device comprises a movement and a watchband, the first electrode and the third electrode are located at one end of the watchband, the second electrode and the fourth electrode are located at the other end of the watchband, and a length of the watchband is greater than a first length.

13. The electronic device according to claim 10, wherein the first electronic device is a wearable device, the wearable device comprises a movement and a watchband, the movement comprises a first button and a second button, the first electrode and the third electrode are located on a rear surface of the movement, the second electrode is located on the first button, and the fourth electrode is located on the second button.

14. The electronic device according to claim 10, wherein the first electronic device is a wearable device, the wearable device comprises a movement and a watchband, the movement comprises a first button and a second button, the first electrode and the third electrode are located on the watchband, the second electrode is located on the first button, and the fourth electrode is located on the second button.

15. The electronic device according to claim 10, wherein the first electronic device is a mobile phone, the mobile phone comprises one or more buttons, one or more of the first electrode, the second electrode, the third electrode, and the fourth electrode are disposed on the one or more buttons, and the first electrode, the second electrode, the third electrode, and the fourth electrode are not in contact with each other.

16. A monitoring system, comprising a first electronic device and a second electronic device, wherein there is a wired connection between the first electronic device and the second electronic device, and the monitoring system comprises the circuit according to any one of claims 1 to 9.

17. The system according to claim 16, wherein the first electrode and the third electrode are located on the first electronic device, and the second electrode and the fourth electrode are located on the second electronic device.

18. The system according to claim 16 or 17, wherein the first electronic device is earphones, and the second electronic device is a mobile phone.

19. A monitoring method, applied to a first electronic device, wherein the first electronic device is provided with a first circuit, the first circuit comprises a first electrode, a second electrode, a third electrode, a fourth electrode, an excitation current generation unit, and a voltage measurement unit, and the method comprises:
receiving a first instruction, wherein the first instruction instructs the first electronic device to start cardiac function monitoring;
in response to the first instruction, determining, through a first loop, that the first electrode is in good contact with skin, wherein the first loop comprises the first electrode, the excitation current generation unit, and the third electrode;
determining, through a second loop, that the second electrode is in good contact with the skin, wherein the second loop comprises the second electrode, the excitation current generation unit, and the fourth electrode;
determining first information through a third loop, wherein the third loop comprises the first electrode, the excitation current generation unit, the voltage measurement unit, and the second electrode, the first electrode is in contact with a first position on the skin of a user, the second electrode is in contact with a second position on the skin of the user, and the first position and the second position are respectively at two ends of thoracic tissue; and
outputting the first information, wherein the first information comprises one or more of the following: a cardiac output, a stroke volume, a heart rate, an ejection fraction, a comprehensive result, a first result, a second result, a third result, and a fourth result, the comprehensive result indicates whether a cardiac function of the user is normal, the first result indicates whether the cardiac output is normal, the second result indicates whether the stroke volume is normal, the third result indicates whether the heart rate is normal, and the fourth result indicates whether the ejection fraction is normal.

20. The method according to claim 19, wherein the receiving the first instruction specifically comprises:
receiving the first instruction sent by a second electronic device; or
receiving a first operation of the user on the first electronic device, and generating the first instruction.

21. The method according to claim 19 or 20, wherein the method further comprises:
outputting a first prompt in response to the first instruction, wherein the first prompt is used to inform the user that cardiac function monitoring is started.

22. The method according to any one of claims 19 to 21, wherein before the determining the first information through the third loop, the method further comprises:
determining that a user status is a first state; and
the determining the first information through the third loop specifically comprises:
determining a cardiac function indicator through the third loop, wherein the cardiac function indicator comprises any one or more of the following: the cardiac output, the stroke volume, the heart rate, and the ejection fraction; and
determining the first information based on the first state and the cardiac function indicator.

23. The method according to claim 22, wherein the first state is an exercise state, a resting state, or a sleep state.

24. The method according to claim 22 or 23, wherein the determining that the user status is the first state specifically comprises:
detecting that the user status is the first state; or
receiving a status setting operation of the user, and in response to the operation, determining that the user status is the first state; or
receiving second information sent by the second electronic device, and determining, based on the second information, that the user status is the first state.

25. The method according to any one of claims 22 to 24, wherein when the first state is the exercise state, the first information further comprises a first graphic, and the first graphic indicates a relationship between the stroke volume and an exercise heart rate.

26. The method according to any one of claims 19 to 25, wherein the outputting the first information specifically comprises:
sending a second instruction to the second electronic device, wherein the second instruction indicates that the first electronic device outputs the first information.

27. The method according to any one of claims 19 to 26, wherein the first electronic device is earphones, and the method further comprises:
playing, by the earphones, first audio before the first instruction is received; and
pausing play of the first audio when the first instruction is received.

28. The method according to claim 27, wherein the playing the first audio specifically comprises:
playing the first audio at first volume; and
the outputting the first information specifically comprises:
playing the first information at the first volume if the user wears the left earphone or the right earphone.

29. The method according to claim 28, wherein the method further comprises:
playing the first information at the second volume if the user wears neither the left earphone nor the right earphone, wherein the second volume is greater than the first volume.

30. An electronic device, which is a first electronic device, and comprises one or more memories, one or more processors, and a first circuit, wherein the first circuit comprises a first electrode, a second electrode, a third electrode, a fourth electrode, a first analog switch, a second analog switch, an excitation current generation unit, and a voltage measurement unit, the one or more memories are coupled to the one or more processors, the one or more memories are configured to store computer program code, the computer program code comprises computer instructions, and when the one or more processors execute the computer instructions, the electronic device is enabled to perform the method according to any one of claims 19 to 29.

31. A chip system, used in a first electronic device, wherein the chip system comprises a processing circuit and an interface circuit, the interface circuit is configured to receive code instructions and transmit the code instructions to the processing circuit, and the processing circuit is configured to run the code instructions, to enable the chip system to perform the method according to any one of claims 19 to 29.

32. A readable storage medium, comprising instructions, wherein when the instructions are run on a first electronic device, the first electronic device is enabled to perform the method according to any one of claims 19 to 29.
